(19) Europäisches Patentamt
European Patent Office
Office européen des brevets

(11) EP 4 786 454 A1

(12) EUROPEAN PATENT APPLICATION
published in accordance with Art. 153(4) EPC

(43) Date of publication:
**05.08.2026 Bulletin 2026/32**

(21) Application number: **24870997.4**

(22) Date of filing: **27.09.2024**

(51) International Patent Classification (IPC):
***C07D 235/02*** $^{(2006.01)}$ ***C07D 471/04*** $^{(2006.01)}$
***A61K 31/5025*** $^{(2006.01)}$ ***A61K 31/437*** $^{(2006.01)}$
***A61P 25/28*** $^{(2006.01)}$

(52) Cooperative Patent Classification (CPC):
**A61K 31/437; A61K 31/5025; A61P 25/28; C07D 235/02; C07D 471/04**

(86) International application number:
**PCT/CN2024/121859**

(87) International publication number:
**WO 2025/067461 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311284906**
**26.10.2023 CN 202311410637**
**28.12.2023 CN 202311849240**
**29.04.2024 CN 202410539834**
**20.09.2024 CN 202411320022**

(71) Applicant: **CMS Research & Development Pte. Ltd.**
**Singapore 179803 (SG)**

(72) Inventors:
- **CHEN, Shuhui**
**Shanghai 200131 (CN)**
- **HE, Haiying**
**Shanghai 200131 (CN)**
- **LI, Peng**
**Shanghai 200131 (CN)**
- **ZHANG, Hongbo**
**Shanghai 200131 (CN)**
- **GAO, Na**
**Shanghai 200131 (CN)**

(74) Representative: **Maiwald GmbH**
**Elisenhof**
**Elisenstraße 3**
**80335 München (DE)**

# (54) HETEROARYL DERIVATIVE AND USE THEREOF

(57) Disclosed are a heteroaryl derivative and the use thereof, and specifically disclosed are compounds represented by formula (I) and (V) or pharmaceutically acceptable salts thereof.

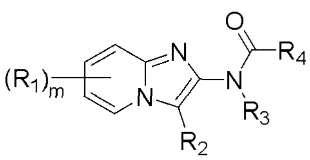

(I)

,

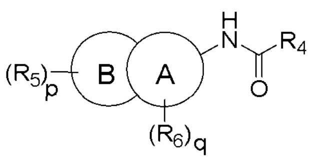

(V)

EP 4 786 454 A1

## Description

The present disclosure claims the priority of:
1) Chinese Patent Application No. 202311284906.6, filed with the China National Intellectual Property Administration (CNIPA) on September 28, 2023; 2) Chinese Patent Application No. 202311410637.3, filed with the CNIPA on October 26, 2023; 3) Chinese Patent Application No. 202311849240.4, filed with the CNIPA on December 28, 2023; 4) Chinese Patent Application No. 202410539834.3, filed with the CNIPA on April 29, 2024; and 5) Chinese Patent Application No. 202411320022.6, filed with the CNIPA on September 20, 2024, the disclosure of which is incorporated herein by reference in its entirety.

## TECHNICAL FIELD

The present disclosure relates to a class of heteroaryl derivatives and use thereof, specifically relates to compounds of formula (I) and formula (V), or pharmaceutically acceptable salts thereof.

## BACKGROUND

Voltage-gated Kv7/KCNQ potassium ion channels are widely distributed in the nervous system, and play an important role in regulating nerve excitability. Research on Kv7 potassium ion channel openers offers significant potential for the development of new drugs to treat nerve hyperexcitability-related diseases, such as epilepsy and pain.

The Kv7 family has five subtypes, among which Kv7.2 and Kv7.3 (encoded by KCNQ2 and KCNQ3) are homologous subunits forming a widely expressed neuronal voltage-gated potassium ion channel, and they are key subunits involved in the neuronal signaling and regulating the hyperexcitability of epilepsy. KCNQ2 and KCNQ3 are mainly distributed in the brain, highly expressed in the cerebellar cortex, amygdala, caudate nucleus, and hippocampus, forming homotetramers/heterotetramers (M-type potassium ion channels), which are the molecular basis for the formation of M-current. Increased M-current can cause hyperpolarization of the cell membrane, thereby reducing neuronal excitability and preventing the occurrence and propagation of action potential bursts and the resulting seizures. Enhancing the open state of Kv7.2/Kv7.3 channels in neurons favors a hyperpolarized resting state, which reduces rapid action potential spiking (i.e., burst firing), and stabilizes the neuronal resting membrane potential, thereby limiting the neuronal excitability. This is also the reason for the antiepileptic drug efficacy.

Retigabine has demonstrated superior efficacy in treating convulsions and epilepsy as a KCNQ2/3 potassium ion channel opener in clinical trials. Therefore, the development of KCNQ2/3 openers has become a hot topic in the clinical treatment of neurological diseases such as epilepsy. XEN1101 from the biopharmaceutical company Xenon has entered Phase 3 clinical trials for the treatment of focal seizures. BHV-7000 from Biohaven has also completed Phase 1 clinical trials, demonstrating a significant reduction in the related side effects, and is a very promising drug for the treatment of nervous system diseases such as epilepsy and pain.

## SUMMARY

The present disclosure provides a compound of formula (I) and a compound of formula (V), or a pharmaceutically acceptable salt thereof,

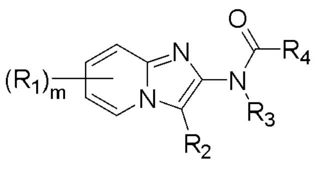

(I)

or

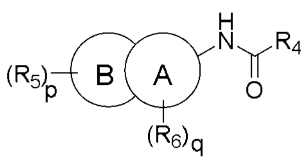

(V)

,

wherein,

- ring A is 9- to 10-membered bicyclic heteroaryl;
- ring B is fused with ring A, and ring B is selected from $C_{4-7}$ cycloalkyl, $C_{4-7}$ cycloalkenyl, 4- to 7-membered heterocycloalkyl and 4- to 7-membered heterocycloalkenyl;
- each $R_1$ is independently selected from H, F, Cl, Br, I, OH, $NH_2$, CN, $CF_3$, $C_{2-4}$ alkyl, $C_{1-4}$ alkoxy, $C_{3-7}$ cycloalkyl and 4- to 7-membered heterocycloalkyl, wherein the $C_{2-4}$ alkyl, $C_{1-4}$ alkoxy, $C_{3-7}$ cycloalkyl and 4-to 7-membered heterocycloalkyl are each independently and optionally substituted with 1, 2 or 3 $R_a$;

$R_2$ is selected from cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl, wherein the cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl are each independently and optionally substituted with 1, 2 or 3 $R_b$;

$R_3$ is selected from H and -C(O)$C_{1-4}$ alkyl;

$R_4$ is selected from $C_{1-6}$ alkyl, $C_{1-6}$ alkoxy, -$CH_2$-$C_{3-7}$ cycloalkyl and -$CH_2$-4- to 7-membered heterocycloalkyl, wherein the $C_{1-6}$ alkyl, $C_{1-6}$ alkoxy, -$CH_2$-$C_{3-7}$ cycloalkyl and -$CH_2$-4- to 7-membered heterocycloalkyl are each independently and optionally substituted with 1, 2, 3, 4 or 5 $R_c$;

each $R_5$ is independently selected from H, F, Cl, Br, I, =O, $C_{1-4}$ alkyl and $C_{1-4}$ alkoxy, wherein the $C_{1-4}$ alkyl and $C_{1-4}$ alkoxy are each independently and optionally substituted with 1, 2 or 3 halogen;

each $R_6$ is independently selected from H, F, Cl, Br, I, CN, $C_{1-4}$ alkyl, $C_{1-4}$ alkoxy, $C_{3-7}$ cycloalkyl, 3-to 7-membered heterocycloalkyl and phenyl, wherein the $C_{1-4}$ alkyl, $C_{1-4}$ alkoxy, $C_{3-7}$ cycloalkyl, 4- to 7-membered heterocycloalkyl and phenyl are each independently and optionally substituted with 1, 2 or 3 $R_{6a}$; each of $R_a$ and $R_c$ is independently selected from H, F, Cl, Br, I, OH, $NH_2$, CN, $CH_3$ and $CF_3$;

each $R_b$ is independently selected from H, F, Cl, Br, I, =O, OH, $NH_2$, CN, $C_{1-4}$ alkyl and $C_{1-4}$ alkoxy, wherein the $C_{1-4}$ alkyl and $C_{1-3}$ alkoxy are each independently and optionally substituted with 1, 2 or 3 F;

each $R_{6a}$ is independently selected from H, F, Cl, Br, I, OH, $NH_2$, CN, $C_{1-4}$ alkyl and $C_{1-4}$ alkoxy, wherein the $C_{1-4}$ alkyl and $C_{1-4}$ alkoxy are each independently and optionally substituted with 1, 2, 3, 4 or 5 F;

m is selected from 1, 2, 3 and 4;

p and q are each independently selected from 1, 2 and 3;

with the proviso that,

1) when $R_2$ is selected from phenyl and 5- to 6-membered heteroaryl, and the phenyl and 5- to 6-membered heteroaryl are each independently and optionally substituted with 1, 2 or 3 $R_b$, the structural fragment

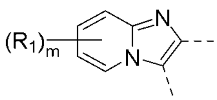

is

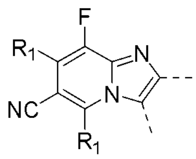

;

or,

2) when $R_2$ is cyclobutyl, and the cyclobutyl is optionally substituted with 1, 2 or 3 $R_b$, m is selected from 2, 3 and 4.

In some technical solutions of the present disclosure, each of the above $R_a$ is independently selected from H, F and OH, and the other variables are as defined in the present disclosure.

In some technical solutions of the present disclosure, each of the above $R_b$ is independently selected from H, F, =O, $CH_3$ and $OCF_3$, and the other variables are as defined in the present disclosure.

In some technical solutions of the present disclosure, each of the above $R_c$ is independently selected from H, F and $CH_3$, and the other variables are as defined in the present disclosure.

In some technical solutions of the present disclosure, each of the above $R_1$ is independently selected from H, F, Cl, OH, $NH_2$, CN, $CF_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl, wherein the $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl are each independently and optionally substituted with 1, 2 or 3 $R_a$, and the other variables are as defined in the present disclosure.

In some technical solutions of the present disclosure, each of the above $R_1$ is independently selected from H, F, CN, $CF_3$,

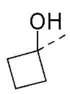

,

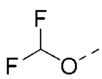

and

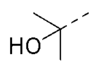

,

and the other variables are as defined in the present disclosure.

In some technical solutions of the present disclosure, the above $R_2$ is selected from $C_{5-8}$ bridged cycloalkyl, $C_{5-8}$ spirocycloalkyl and 4- to 7-membered heterocycloalkyl, wherein the $C_{5-8}$ bridged cycloalkyl, $C_{5-8}$ spirocycloalkyl and 4- to 7-membered heterocycloalkyl are each independently and optionally substituted with 1, 2 or 3 $R_b$, and the other variables are as defined in the present disclosure.

In some technical solutions of the present disclosure, the above $R_2$ is selected from cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl, wherein the cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl are each independently and optionally substituted with 1, 2 or 3 $R_b$, and the other variables are as defined in the present disclosure.

In some technical solutions of the present disclosure, the above $R_2$ is selected from

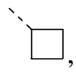, 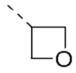,

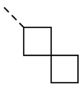, 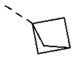, 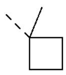, 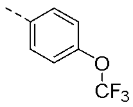, 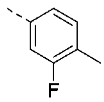, 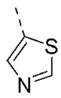, 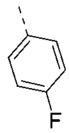, 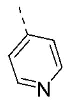, 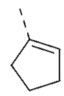, 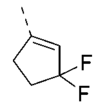 and 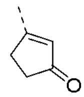,

and the other variables are as defined in the present disclosure.

In some technical solutions of the present disclosure, the above $R_2$ is

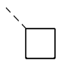, 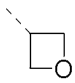, 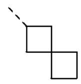, 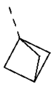,

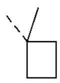 or 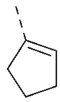,

and the other variables are as defined in the present disclosure.

In some technical solutions of the present disclosure, the above $R_2$ is , and the other variables are as defined in the present disclosure.

In some technical solutions of the present disclosure, the above $R_2$ is

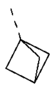,

and the other variables are as defined in the present disclosure.

In some technical solutions of the present disclosure, the above $R_2$ is as defined in the present disclosure.

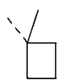,

and the other variables are

In some technical solutions of the present disclosure, the above $R_2$ is

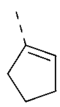,

and the other variables are as defined in the present disclosure.

In some technical solutions of the present disclosure, the above $R_4$ is selected from $CH_2CH_3$, $CH_2CH_2CH_3$, $CH(CH_3)_2$, $C(CH_3)_3$, $CH_2C(CH_3)_3$, $OCH_3$, $OCH_2CH_3$, $OCH_2CH_2CH_3$, $OCH(CH_3)_2$,

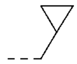,

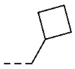, 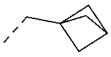, 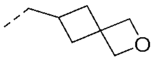, 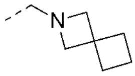, 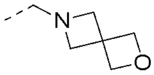, 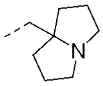 and 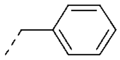,

wherein the $CH_2CH_3$, $CH_2CH_2CH_3$, $CH(CH_3)_2$, $C(CH_3)_3$, $CH_2C(CH_3)_3$, $OCH_3$, $OCH_2CH_3$, $OCH_2CH_2CH_3$, $OCH(CH_3)_2$,

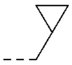, 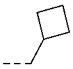, 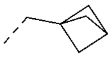, 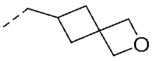, 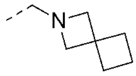, 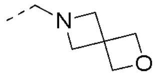, 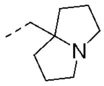 and 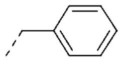

are each independently and optionally substituted with 1, 2, 3, 4 or 5 $R_c$, and the other variables are as defined in the present disclosure.

In some technical solutions of the present disclosure, the above $R_4$ is selected from

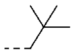, 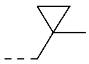,

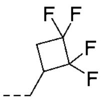, 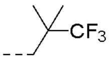, 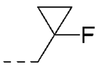, 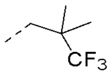, 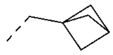 and 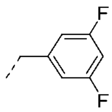,

and the other variables are as defined in the present disclosure.

In some technical solutions of the present disclosure, the above $R_4$ is

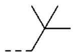,

and the other variables are as defined in the present disclosure.

In some technical solutions of the present disclosure, the above $R_4$ is

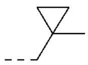,

and the other variables are as defined in the present disclosure.

In some technical solutions of the present disclosure, the above $R_4$ is

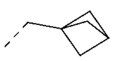,

and the other variables are as defined in the present disclosure.

In some technical solutions of the present disclosure, the above $R_4$ is

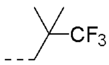,

and the other variables are as defined in the present disclosure.

In some technical solutions of the present disclosure, the above structural unit

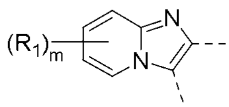

is selected from

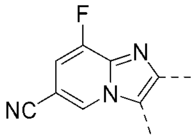, 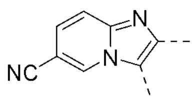, 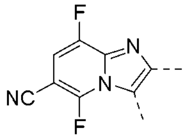, 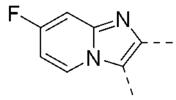, 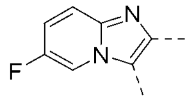,

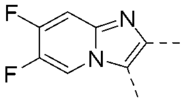, 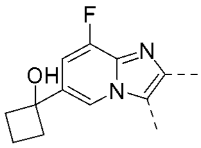, 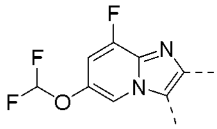, 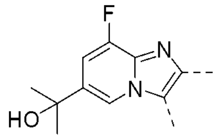 and 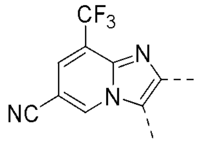,

and the other variables are as defined in the present disclosure.

In some technical solutions of the present disclosure, the above structural unit

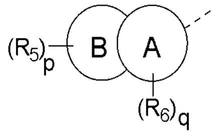 is

selected from

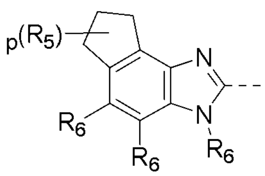, 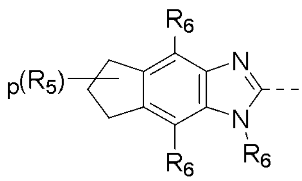, 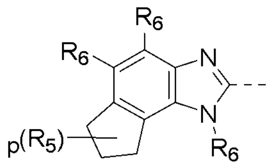,

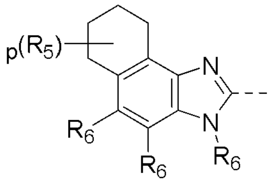, 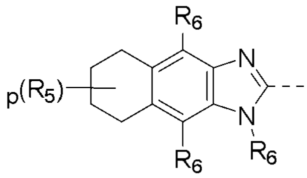, 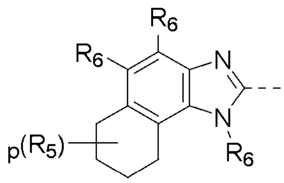, 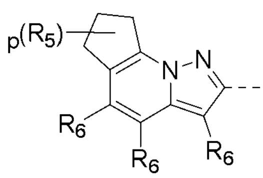,

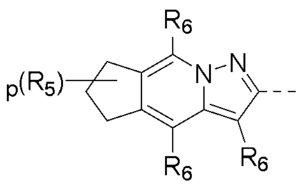, 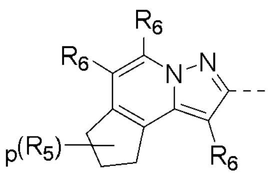, 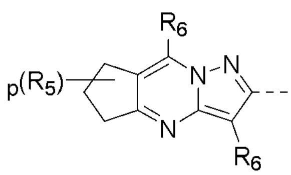, 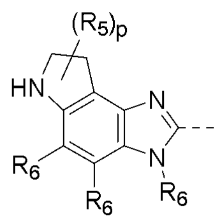,

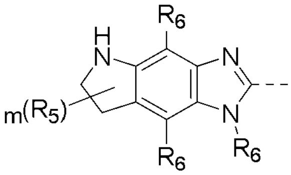, 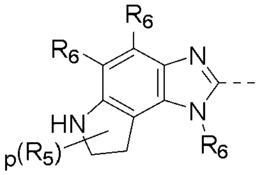, 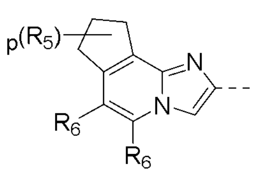, 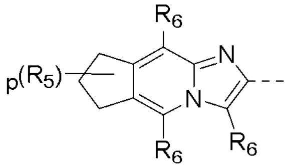,

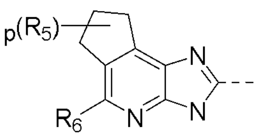, 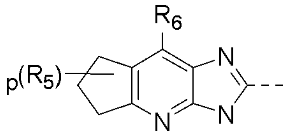, 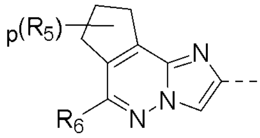, 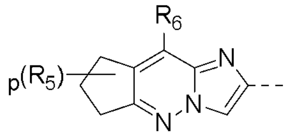,

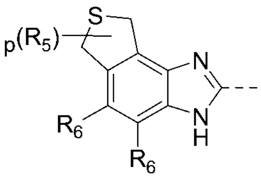, 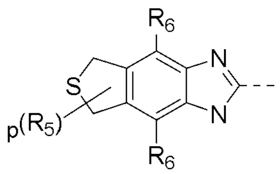, 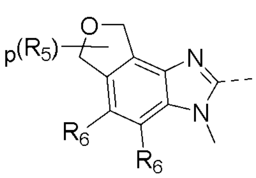, 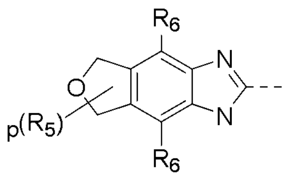,

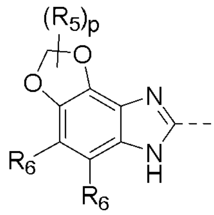 and 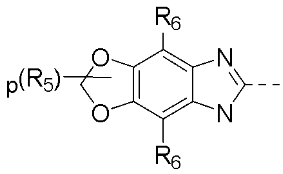,

and the other variables are as defined in the present disclosure.

In some technical solutions of the present disclosure, the above structural unit

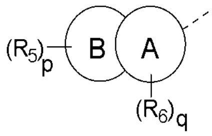 is

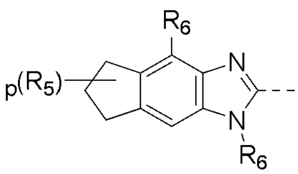,

and the other variables are as defined in the present disclosure.

The present disclosure provides a compound of formula (I), or a pharmaceutically acceptable salt thereof,

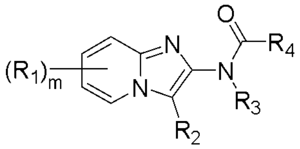

**(I)** ,

wherein,

each $R_1$ is independently selected from H, F, Cl, Br, I, OH, $NH_2$, CN, $CF_3$, $C_{2-4}$ alkyl, $C_{1-4}$ alkoxy, $C_{3-7}$ cycloalkyl and 4-

to 7-membered heterocycloalkyl, wherein the $C_{2-4}$ alkyl, $C_{1-4}$ alkoxy, $C_{3-7}$ cycloalkyl and 4-to 7-membered heterocycloalkyl are each independently and optionally substituted with 1, 2 or 3 $R_a$;
$R_2$ is selected from cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl, wherein the cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl are each independently and optionally substituted with 1, 2 or 3 $R_b$;
$R_3$ is selected from H and -C(O)$C_{1-4}$ alkyl;
$R_4$ is selected from $C_{1-6}$ alkyl, $C_{1-6}$ alkoxy, -$CH_2$-$C_{3-7}$ cycloalkyl and -$CH_2$-4- to 7-membered heterocycloalkyl, wherein the $C_{1-6}$ alkyl, $C_{1-6}$ alkoxy, -$CH_2$-$C_{3-7}$ cycloalkyl and -$CH_2$-4- to 7-membered heterocycloalkyl are each independently and optionally substituted with 1, 2, 3, 4 or 5 $R_c$;
each of $R_a$ and $R_c$ is independently selected from H, F, Cl, Br, I, OH, $NH_2$, CN, $CH_3$ and $CF_3$;
each $R_b$ is independently selected from H, F, Cl, Br, I, =O, OH, $NH_2$, CN, $C_{1-4}$ alkyl and $C_{1-4}$ alkoxy, wherein the $C_{1-4}$ alkyl and $C_{1-3}$ alkoxy are each independently and optionally substituted with 1, 2 or 3 F;
m is selected from 1, 2, 3 and 4;
with the proviso that,

1) when $R_2$ is selected from phenyl and 5- to 6-membered heteroaryl, and the phenyl and 5- to 6-membered heteroaryl are each independently and optionally substituted with 1, 2 or 3 $R_b$, the structural fragment

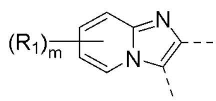

is

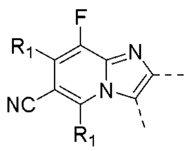

;

or,
2) when $R_2$ is cyclobutyl, and the cyclobutyl is optionally substituted with 1, 2 or 3 $R_b$, m is selected from 2, 3 and 4.

The present disclosure also provides a compound of formula (I), or a pharmaceutically acceptable salt thereof,

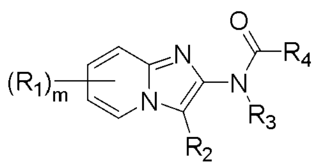

**(I)**

,

wherein,

each $R_1$ is independently selected from H, F, Cl, Br, I, OH, $NH_2$, CN, $C_{2-4}$ alkyl, $C_{1-4}$ alkoxy, $C_{3-7}$ cycloalkyl and 4- to 7-membered heterocycloalkyl, wherein the $C_{2-4}$ alkyl, $C_{1-4}$ alkoxy, $C_{3-7}$ cycloalkyl and 4- to 7-membered heterocycloalkyl are each independently and optionally substituted with 1, 2 or 3 $R_a$;
$R_2$ is selected from cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl, wherein the cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl are each independently and optionally substituted with 1, 2 or 3 $R_b$;
$R_3$ is selected from H and -C(O)$C_{1-4}$ alkyl;
$R_4$ is selected from $C_{1-6}$ alkyl, $C_{1-6}$ alkoxy, -$CH_2$-$C_{3-7}$ cycloalkyl and -$CH_2$-4- to 7-membered heterocycloalkyl, wherein the $C_{1-6}$ alkyl, $C_{1-6}$ alkoxy, -$CH_2$-$C_{3-7}$ cycloalkyl and -$CH_2$-4- to 7-membered heterocycloalkyl are each independently and optionally substituted with 1, 2, 3, 4 or 5 $R_c$;
each of $R_a$ and $R_c$ is independently selected from H, F, Cl, Br, I, OH, $NH_2$, CN, $CH_3$ and $CF_3$;

each $R_b$ is independently selected from H, F, Cl, Br, I, =O, OH, $NH_2$, CN, $C_{1-4}$ alkyl and $C_{1-4}$ alkoxy, wherein the $C_{1-4}$ alkyl and $C_{1-3}$ alkoxy are each independently and optionally substituted with 1, 2 or 3 F;
m is selected from 1, 2, 3 and 4;
with the proviso that,

1) when $R_2$ is selected from phenyl and 5- to 6-membered heteroaryl, and the phenyl and 5- to 6-membered heteroaryl are each independently and optionally substituted with 1, 2 or 3 $R_b$, the structural fragment

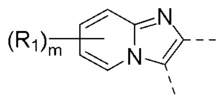

is

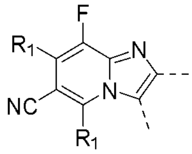

; or,
2) when $R_2$ is cyclobutyl, and the cyclobutyl is optionally substituted with 1, 2 or 3 $R_b$, m is selected from 2, 3 and 4.

The present disclosure also provides a compound of formula (I), or a pharmaceutically acceptable salt thereof,

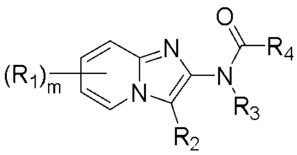

**(I)**

,

wherein,

each $R_1$ is independently selected from F, Cl, Br, I, OH, $NH_2$, CN, $C_{2-4}$ alkyl, $C_{1-4}$ alkoxy, $C_{3-7}$ cycloalkyl and 4- to 7-membered heterocycloalkyl, wherein the $C_{2-4}$ alkyl, $C_{1-4}$ alkoxy, $C_{3-7}$ cycloalkyl and 4- to 7-membered heterocycloalkyl are each independently and optionally substituted with 1, 2 or 3 $R_a$;
$R_2$ is selected from cyclobutyl, $C_{5-8}$ bridged cycloalkyl, $C_{5-8}$ spirocycloalkyl, phenyl, 5- to 6-membered heteroaryl and 4- to 7-membered heterocycloalkyl, wherein the cyclobutyl, $C_{5-8}$ bridged cycloalkyl, $C_{5-8}$ spirocycloalkyl, phenyl, 5- to 6-membered heteroaryl and 4- to 7-membered heterocycloalkyl are each independently and optionally substituted with 1, 2 or 3 $R_b$;
$R_3$ is selected from H and $-C(O)C_{1-4}$ alkyl;
$R_4$ is selected from $C_{1-6}$ alkyl, $C_{1-6}$ alkoxy, $-CH_2-C_{3-7}$ cycloalkyl and $-CH_2$-4- to 7-membered heterocycloalkyl, wherein the $C_{1-6}$ alkyl, $C_{1-6}$ alkoxy, $-CH_2-C_{3-7}$ cycloalkyl and $-CH_2$-4- to 7-membered heterocycloalkyl are each independently and optionally substituted with 1, 2, 3, 4 or 5 $R_c$;
each of $R_a$ and $R_c$ is independently selected from F, Cl, Br, I, OH, $NH_2$, CN, $CH_3$ and $CF_3$;
each $R_b$ is independently selected from F, Cl, Br, I, OH, $NH_2$, CN, $C_{1-4}$ alkyl and $C_{1-4}$ alkoxy, wherein the $C_{1-4}$ alkyl and $C_{1-3}$ alkoxy are each independently and optionally substituted with 1, 2 or 3 F;
m is selected from 1, 2, 3 and 4;
with the proviso that,

1) when $R_2$ is selected from phenyl and 5- to 6-membered heteroaryl, and the phenyl and 5- to 6-membered heteroaryl are each independently and optionally substituted with 1, 2 or 3 $R_b$, the structural fragment

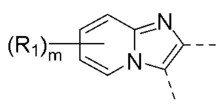

is

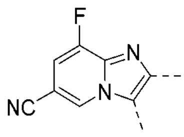;

or,

2) when $R_2$ is cyclobutyl, and the cyclobutyl is optionally substituted with 1, 2 or 3 $R_b$, m is selected from 2, 3 and 4.

In some technical solutions of the present disclosure, each of the above $R_a$ is independently selected from H, F and OH, and the other variables are as defined in the present disclosure.

In some technical solutions of the present disclosure, each of the above $R_a$ is independently selected from F and OH, and the other variables are as defined in the present disclosure.

In some technical solutions of the present disclosure, each of the above $R_b$ is independently selected from H, F, =O, $CH_3$ and $OCF_3$, and the other variables are as defined in the present disclosure.

In some technical solutions of the present disclosure, each of the above $R_b$ is independently selected from F, $CH_3$ and $OCF_3$, and the other variables are as defined in the present disclosure.

In some technical solutions of the present disclosure, each of the above $R_c$ is independently selected from H, F and $CH_3$, and the other variables are as defined in the present disclosure.

In some technical solutions of the present disclosure, each of the above $R_c$ is independently selected from F and $CH_3$, and the other variables are as defined in the present disclosure.

In some technical solutions of the present disclosure, each of the above $R_1$ is independently selected from H, F, Cl, OH, $NH_2$, CN, $CF_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl, wherein the $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl are each independently and optionally substituted with 1, 2 or 3 $R_a$, and the other variables are as defined in the present disclosure.

In some technical solutions of the present disclosure, each of the above $R_1$ is independently selected from H, F, CN, $CF_3$,

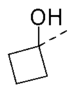, 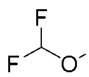 and 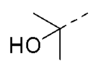,

and the other variables are as defined in the present disclosure.

In some technical solutions of the present disclosure, each of the above $R_1$ is independently selected from H, F, CN and $CF_3$, and the other variables are as defined in the present disclosure.

In some technical solutions of the present disclosure, each of the above $R_1$ is independently selected from H, F, Cl, OH, $NH_2$, CN, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl, wherein the $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl are each independently and optionally substituted with 1, 2 or 3 $R_a$, and the other variables are as defined in the present disclosure.

In some technical solutions of the present disclosure, each of the above $R_1$ is selected from H, F, CN,

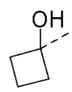, 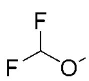 and 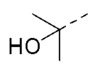,

and the other variables are as defined in the present disclosure.

In some technical solutions of the present disclosure, each of the above $R_1$ is independently selected from F, Cl, OH, $NH_2$, CN, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl, wherein the $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl are each independently and optionally substituted with 1, 2 or 3 $R_a$, and the other variables are as defined in the present disclosure.

In some technical solutions of the present disclosure, each of the above $R_1$ is selected from F, CN,

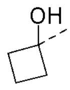, 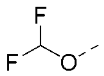 and 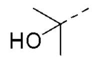,

and the other variables are as defined in the present disclosure.

In some technical solutions of the present disclosure, the above $R_2$ is selected from $C_{5-8}$ bridged cycloalkyl, $C_{5-8}$ spirocycloalkyl, 4- to 7-membered heterocycloalkyl and $C_{5-8}$ cycloalkenyl, wherein the $C_{5-8}$ bridged cycloalkyl, $C_{5-8}$ spirocycloalkyl, 4- to 7-membered heterocycloalkyl and $C_{5-8}$ cycloalkenyl are each independently and optionally substituted with 1, 2 or 3 $R_b$, and the other variables are as defined in the present disclosure.

In some technical solutions of the present disclosure, the above $R_2$ is selected from $C_{5-8}$ bridged cycloalkyl, $C_{5-8}$ spirocycloalkyl and 4- to 7-membered heterocycloalkyl, wherein the $C_{5-8}$ bridged cycloalkyl, $C_{5-8}$ spirocycloalkyl and 4- to 7-membered heterocycloalkyl are each independently and optionally substituted with 1, 2 or 3 $R_b$, and the other variables are as defined in the present disclosure.

In some technical solutions of the present disclosure, the above $R_2$ is selected from $C_{5-8}$ bridged cycloalkyl and $C_{5-8}$ cycloalkenyl, wherein the $C_{5-8}$ bridged cycloalkyl and $C_{5-8}$ cycloalkenyl are each independently and optionally substituted with 1, 2 or 3 $R_b$, and the other variables are as defined in the present disclosure.

In some technical solutions of the present disclosure, the above $R_2$ is $C_{5-8}$ bridged cycloalkyl, wherein the $C_{5-8}$ bridged cycloalkyl is optionally substituted with 1, 2 or 3 $R_b$, and the other variables are as defined in the present disclosure.

In some technical solutions of the present disclosure, the above $R_2$ is

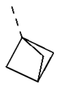,

and the other variables are as defined in the present disclosure.

In some technical solutions of the present disclosure, the above $R_2$ is selected from cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl, wherein the cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl are each independently and optionally substituted with 1, 2 or 3 $R_b$, and the other variables are as defined in the present disclosure.

In some technical solutions of the present disclosure, the above $R_2$ is selected from cyclobutyl, spiro[3.3]heptyl, bicyclo[1.1.1]pentyl, phenyl, pyrazolyl, thiazolyl, pyridyl, oxetanyl, azetidinyl, piperidyl and piperazinyl, wherein the cyclobutyl, spiro[3.3]heptyl, bicyclo[1.1.1]pentyl, phenyl, pyrazolyl, thiazolyl, pyridyl, oxetanyl, azetidinyl, piperidyl and piperazinyl are each independently and optionally substituted with 1, 2 or 3 $R_b$, and the other variables are as defined in the present disclosure.

In some technical solutions of the present disclosure, the above $R_2$ is selected from

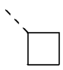, 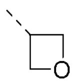,

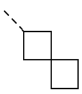, 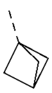, 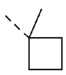, 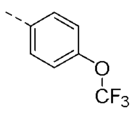, 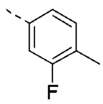, 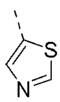, 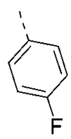, 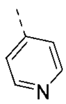, 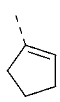, 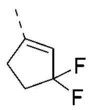 and 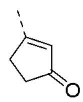,

and the other variables are as defined in the present disclosure.

In some technical solutions of the present disclosure, the above $R_2$ is selected from

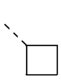, 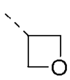,

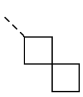, 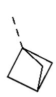, 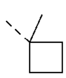, 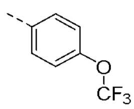, 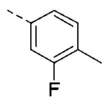 and 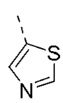,

and the other variables are as defined in the present disclosure.

In some technical solutions of the present disclosure, the above $R_2$ is selected from $C_{5-8}$ bridged cycloalkyl, $C_{5-8}$ spirocycloalkyl and 4- to 7-membered heterocycloalkyl, wherein the $C_{5-8}$ bridged cycloalkyl, $C_{5-8}$ spirocycloalkyl and 4- to 7-membered heterocycloalkyl are each independently and optionally substituted with 1, 2 or 3 $R_b$, and the other variables are as defined in the present disclosure.

In some technical solutions of the present disclosure, the above $R_2$ is selected from

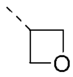, 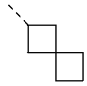

and

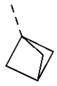,

and the other variables are as defined in the present disclosure.

In some technical solutions of the present disclosure, the above $R_4$ is selected from $CH_2CH_3$, $CH_2CH_2CH_3$, $CH(CH_3)_2$, $C(CH_3)_3$, $C(CH_3)_3$, $OCH_3$, $OCH_2CH_3$, $OCH_2CH_2CH_3$, $OCH(CH_3)_2$,

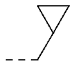

and

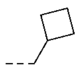,

wherein the $CH_2CH_3$, $CH_2CH_2CH_3$, $CH(CH_3)_2$, $C(CH_3)_3$, $C(CH_3)_3$, $OCH_3$, $OCH_2CH_3$, $OCH_2CH_2CH_3$, $OCH(CH_3)_2$,

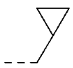 and 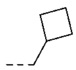

are each independently and optionally substituted with 1, 2, 3, 4 or 5 $R_c$, and the other variables are as defined in the present disclosure.

In some technical solutions of the present disclosure, the above $R_4$ is selected from

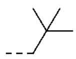, 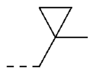,

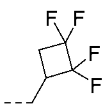, 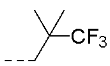 and 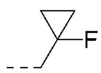,

and the other variables are as defined in the present disclosure.

In some technical solutions of the present disclosure, the above $R_4$ is selected from

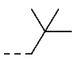, 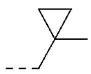,

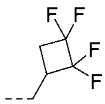 and 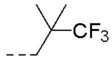,

and the other variables are as defined in the present disclosure.

In some technical solutions of the present disclosure, the above $R_4$ is selected from

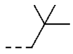

and

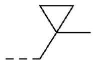,

and the other variables are as defined in the present disclosure.

In some technical solutions of the present disclosure, the above $R_4$ is

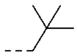,

and the other variables are as defined in the present disclosure.

In some technical solutions of the present disclosure, the above $R_4$ is

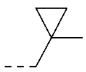,

and the other variables are as defined in the present disclosure. In some technical solutions of the present disclosure, the above structural unit

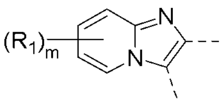 is 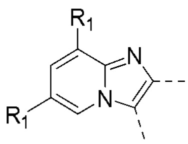,

and the other variables are as defined in the present disclosure.

In some technical solutions of the present disclosure, the above structural unit

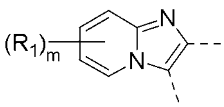

is

selected from 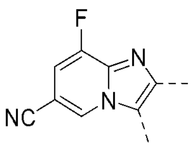, 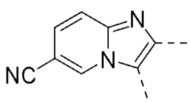, 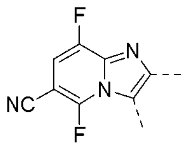, 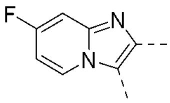, 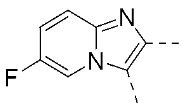,

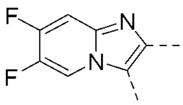, 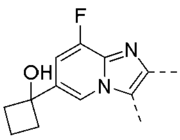, 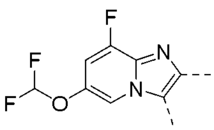, 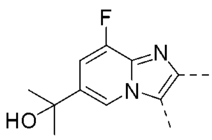 and 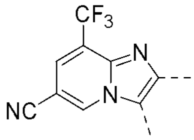,

and the other variables are as defined in the present disclosure.

In some technical solutions of the present disclosure, the above structural unit

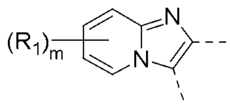

is

selected from 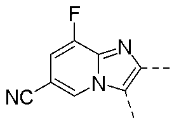, 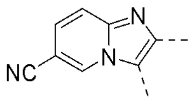, 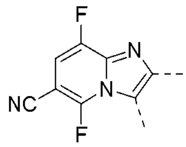, 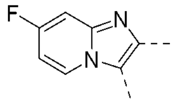, 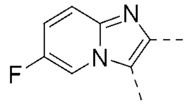,

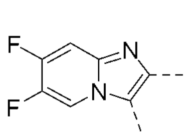, 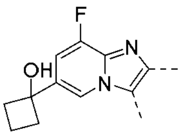, 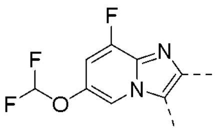 and 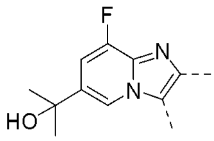,

and the other variables are as defined in the present disclosure.

In some technical solutions of the present disclosure, the above structural unit

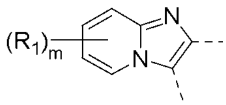

is selected from

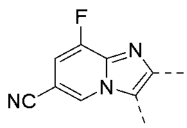 and 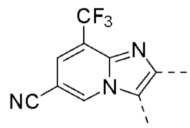,

and the other variables are as defined in the present disclosure.

In some technical solutions of the present disclosure, disclosed herein is the above compound or a pharmaceutically acceptable salt thereof, and the compound is

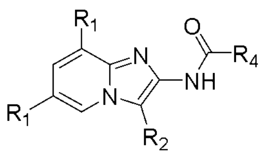

(III) ,

wherein,

each $R_1$ is independently selected from H, F, CN, $CF_3$,

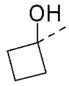, 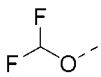 and 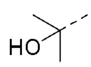;

$R_2$ is selected from $C_{5-8}$ bridged cycloalkyl, $C_{5-8}$ spirocycloalkyl, 4- to 7-membered heterocycloalkyl and $C_{5-8}$

cycloalkenyl, wherein the $C_{5-8}$ bridged cycloalkyl, $C_{5-8}$ spirocycloalkyl, 4- to 7-membered heterocycloalkyl and $C_{5-8}$ cycloalkenyl are each independently and optionally substituted with 1, 2 or 3 $R_b$;

$R_4$ is selected from

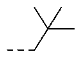, 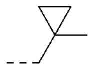, 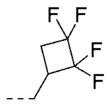, 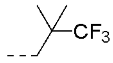 and 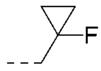.

In some technical solutions of the present disclosure, disclosed herein is the above compound or a pharmaceutically acceptable salt thereof, and the compound is

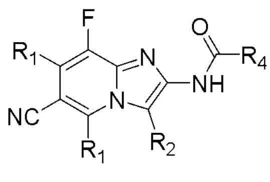

(II) ,

wherein,

$R_2$ is selected from phenyl, 5- to 6-membered heteroaryl and $C_{5-8}$ cycloalkenyl, wherein the phenyl, 5-to 6-membered heteroaryl and $C_{5-8}$ cycloalkenyl are each independently and optionally substituted with 1, 2 or 3 $R_b$;
each $R_b$, each $R_1$ and $R_4$ are as defined in the present disclosure.

In some technical solutions of the present disclosure, disclosed herein is the above compound or a pharmaceutically acceptable salt thereof, and the compound is

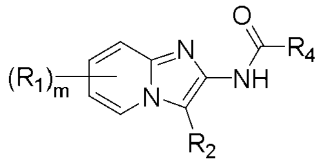

(I)

wherein,

$R_2$ is selected from $C_{5-8}$ bridged cycloalkyl, $C_{5-8}$ spirocycloalkyl and 4- to 7-membered heterocycloalkyl, wherein the $C_{5-8}$ bridged cycloalkyl, $C_{5-8}$ spirocycloalkyl and 4- to 7-membered heterocycloalkyl are each independently and optionally substituted with 1, 2 or 3 $R_b$;
each of $R_1$, $R_4$, $R_b$ and m is as defined in the present disclosure.

In some technical solutions of the present disclosure, disclosed herein is the above compound or a pharmaceutically acceptable salt thereof, and the compound is

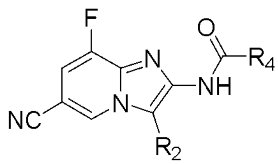

(I-1) ,

wherein, $R_2$ and $R_4$ are as defined in the present disclosure.

The present disclosure also provides a compound of formula (I'), or a pharmaceutically acceptable salt thereof,

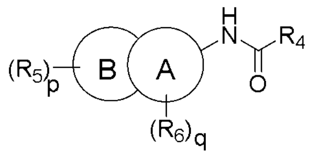

(I')

,

wherein,

ring A is 9- to 10-membered bicyclic heteroaryl;
ring B is fused with ring A, and ring B is selected from $C_{4-7}$ cycloalkyl, $C_{4-7}$ cycloalkenyl, 4- to 7-membered heterocycloalkyl and 4- to 7-membered heterocycloalkenyl;
each $R_5$ is independently selected from H, F, Cl, Br, I, =O, $C_{1-4}$ alkyl and $C_{1-4}$ alkoxy, wherein the $C_{1-4}$ alkyl and $C_{1-4}$ alkoxy are each independently and optionally substituted with 1, 2 or 3 halogen;
each $R_6$ is independently selected from H, F, Cl, Br, I, CN, $C_{1-4}$ alkyl, $C_{1-4}$ alkoxy, $C_{3-7}$ cycloalkyl, 3-to 7-membered heterocycloalkyl and phenyl, wherein the $C_{1-4}$ alkyl, $C_{1-4}$ alkoxy, $C_{3-7}$ cycloalkyl, 4- to 7-membered heterocycloalkyl and phenyl are each independently and optionally substituted with 1, 2 or 3 $R_{6a}$; $R_4$ is selected from $C_{1-6}$ alkyl, -$CH_2$-$C_{3-7}$ cycloalkyl, -$CH_2$-4- to 7-membered heterocycloalkyl and -$CH_2$-phenyl, wherein the $C_{1-6}$ alkyl, -$CH_2$-$C_{3-7}$ cycloalkyl, -$CH_2$-4- to 7-membered heterocycloalkyl and -$CH_2$-phenyl are each independently and optionally substituted with 1, 2, 3, 4 or 5 $R_c$;
each $R_{6a}$ is independently selected from H, F, Cl, Br, I, OH, $NH_2$, CN, $C_{1-4}$ alkyl and $C_{1-4}$ alkoxy, wherein the $C_{1-4}$ alkyl and $C_{1-4}$ alkoxy are each independently and optionally substituted with 1, 2, 3, 4 or 5 F;
each $R_c$ is independently selected from H, F, Cl, Br, I, $CH_3$ and $CF_3$;
m and n are each independently selected from 1, 2 and 3.

In some technical solutions of the present disclosure, each of the above $R_5$ is independently selected from H, F and $CH_3$, and the other variables are as defined in the present disclosure.

In some technical solutions of the present disclosure, each of the above $R_6$ is independently selected from H, F, Cl, CN, $CH_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, azetidinyl and phenyl, wherein the $CH_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, azetidinyl and phenyl are each independently and optionally substituted with 1, 2 or 3 $R_{6a}$, and the other variables are as defined in the present disclosure.

In some technical solutions of the present disclosure, each of the above $R_6$ is independently selected from H, F, Cl, $CH_3$, $CF_3$, $CH_2CH_3$, $C(CH_3)_3$, CN,

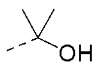, 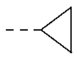, 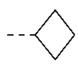, 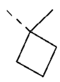 and 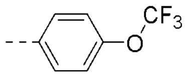,

and the other variables are as defined in the present disclosure.

In some technical solutions of the present disclosure, each of the above $R_6$ is independently selected from H, F, Cl, CN, $CH_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl and azetidinyl, wherein the $CH_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl and azetidinyl are each independently and optionally substituted with 1, 2 or 3 $R_{6a}$, and the other variables are as defined in the present disclosure.

In some technical solutions of the present disclosure, each of the above $R_6$ is independently selected from H, F, Cl, $CH_3$, $CF_3$, $CH_2CH_3$, $C(CH_3)_3$, CN,

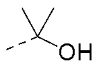, 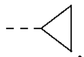, 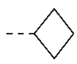 and 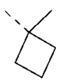,

and the other variables are as defined in the present disclosure.

In some technical solutions of the present disclosure, the above $R_4$ is selected from $CH_2C(CH_3)_3$,

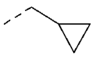, 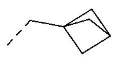, 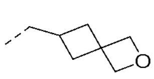, 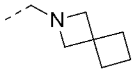, 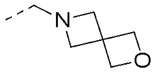, 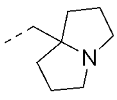 and 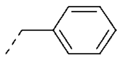,

wherein the $CH_2C(CH_3)_3$,

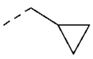, 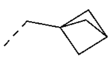, 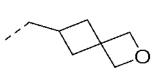, 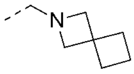, 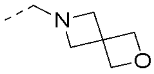, 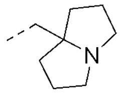 and 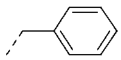

are each independently and optionally substituted with 1, 2, 3, 4 or 5 $R_c$, and the other variables are as defined in the present disclosure.

In some technical solutions of the present disclosure, the above $R_4$ is selected from $CH_2C(CH_3)_3$,

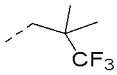, 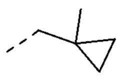, 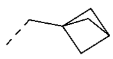 and 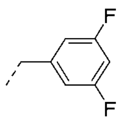,

and the other variables are as defined in the present disclosure.

In some technical solutions of the present disclosure, the above ring A is selected from benzimidazolyl, benzopyrazolyl, pyridoimidazolyl, pyridopyrazolyl, pyrimidoimidazolyl, pyrimidopyrazolyl, indolyl and pyridazinoimidazolyl, and the other variables are as defined in the present disclosure.

In some technical solutions of the present disclosure, the above ring A is selected from benzimidazolyl, benzopyrazolyl, pyridoimidazolyl, pyridopyrazolyl, pyrimidoimidazolyl and pyrimidopyrazolyl, and the other variables are as defined in the present disclosure.

In some technical solutions of the present disclosure, the above ring B is selected from cyclopentyl, cyclohexyl, cyclopentenyl, cyclohexenyl, tetrahydropyrrolyl, dihydropyrrolyl, tetrahydrofuranyl, dihydrofuranyl, tetrahydrothiophenyl, dihydrothienyl, 1,3-dioxolyl, and 1,4-dioxenyl, and the other variables are as defined in the present disclosure.

In some technical solutions of the present disclosure, the above ring B is selected from cyclopentyl, cyclohexyl, cyclopentenyl, cyclohexenyl, pyrrolidinyl and pyrrolinyl, and the other variables are as defined in the present disclosure.

In some technical solutions of the present disclosure, the above structural unit

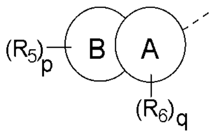

is selected from

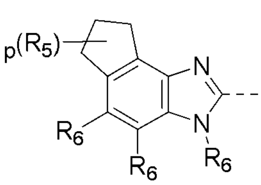, 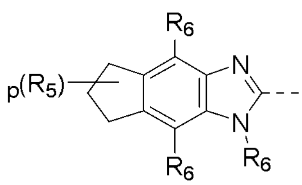, 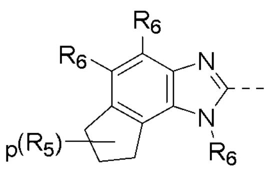,

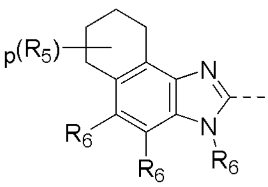, 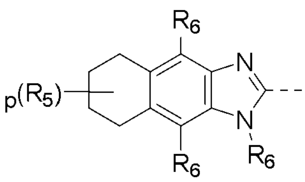, 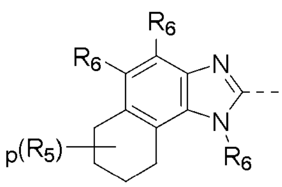, 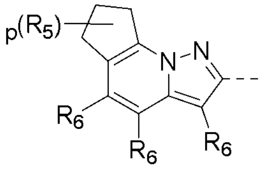,

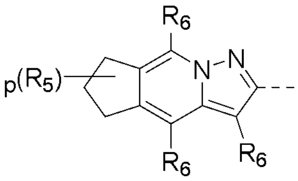 , 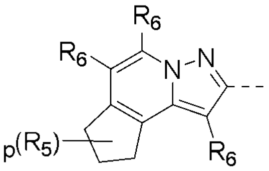 , 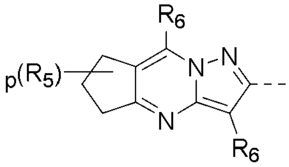 , 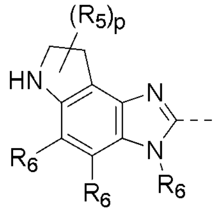 ,

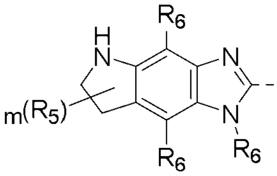 , 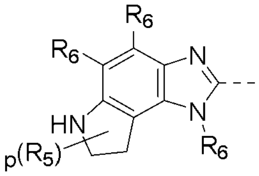 , 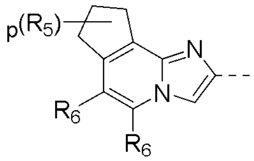 , 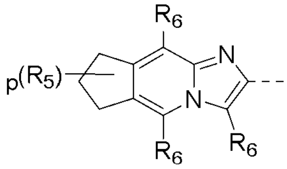 ,

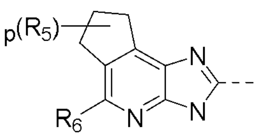 , 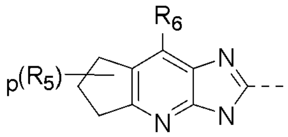 , 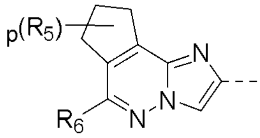 , 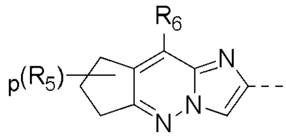 ,

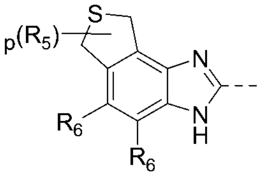 , 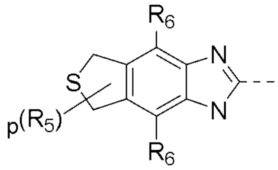 , 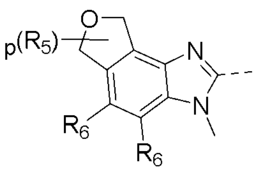 , 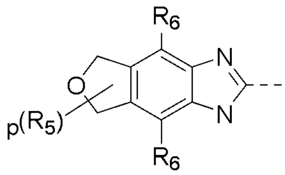 ,

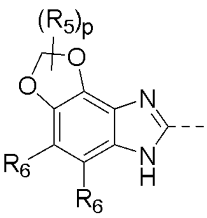 and 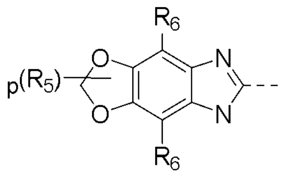 ;

wherein $R_5$, $R_6$ and p are as defined in the present disclosure.

Some other embodiments of the present disclosure are derived from the arbitrary combinations of each of the above variables.

The present disclosure also provides a compound of the following, or a pharmaceutically acceptable salt thereof,

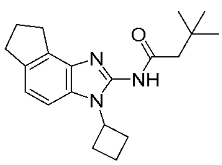 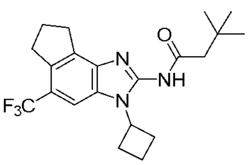 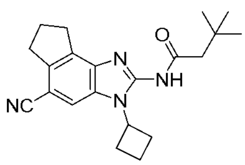 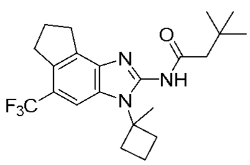 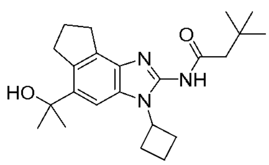

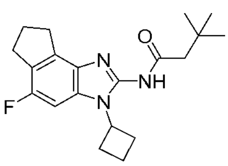 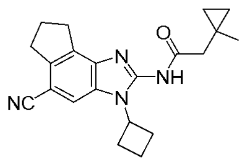 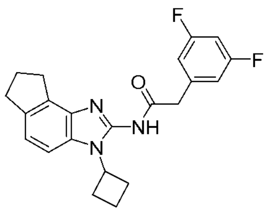 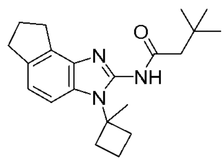 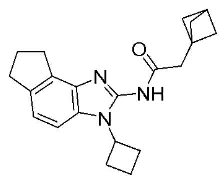

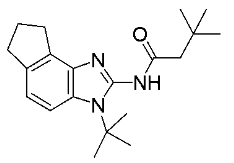 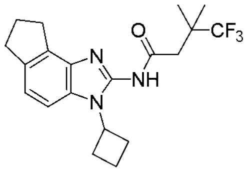 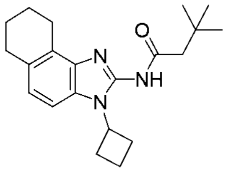 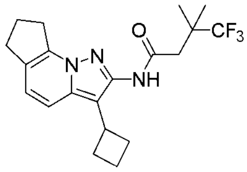 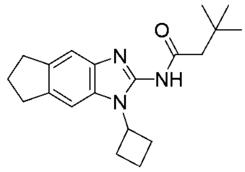

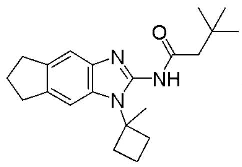 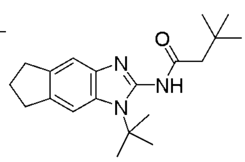 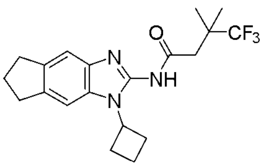 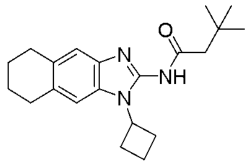 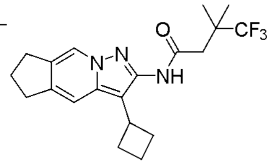

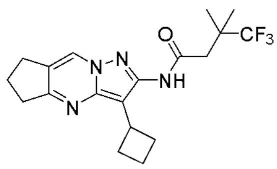 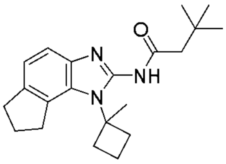 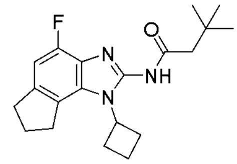 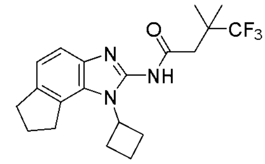 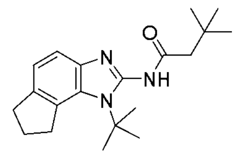

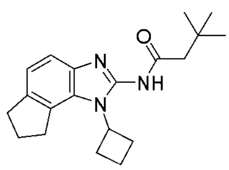 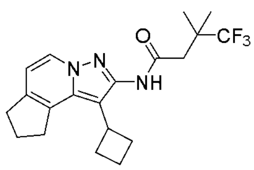 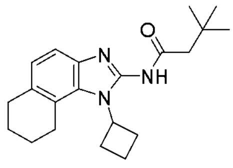 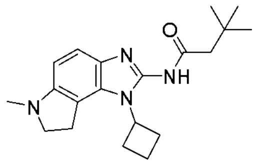 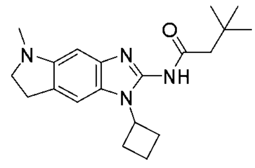

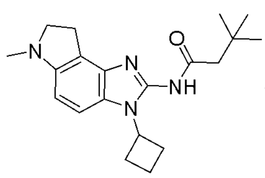 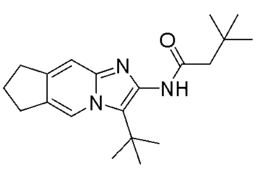 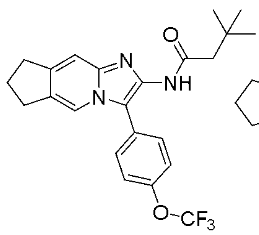 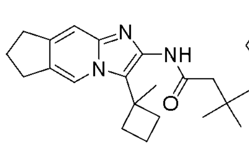 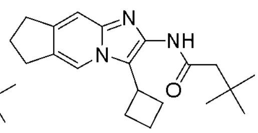

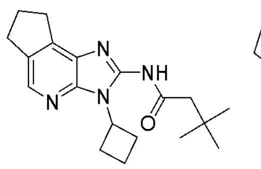 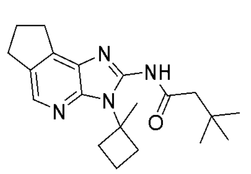 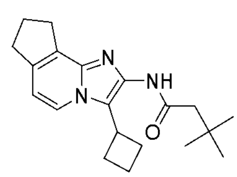 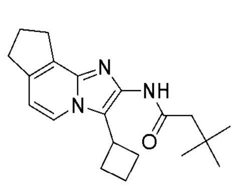 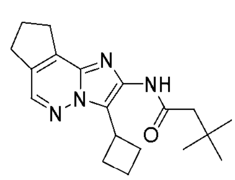

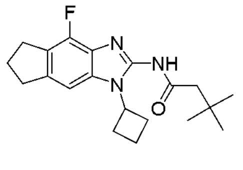 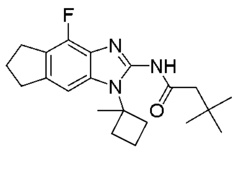 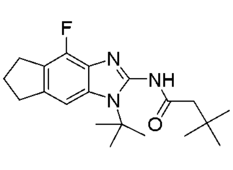 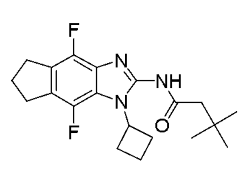 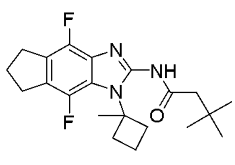

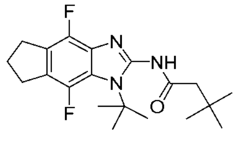 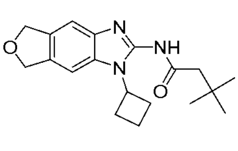 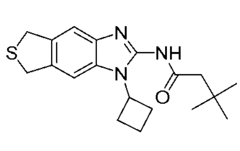 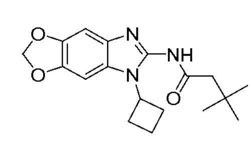 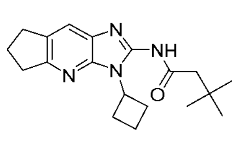

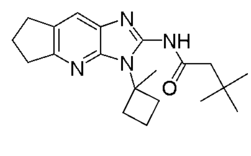
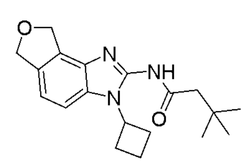
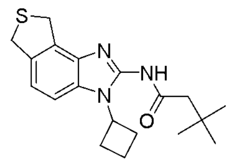
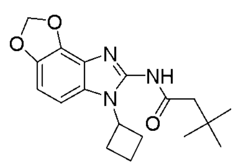
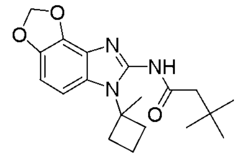

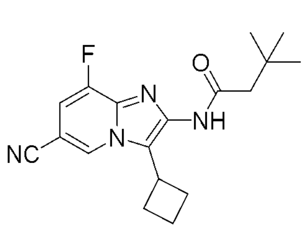
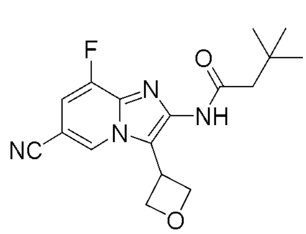
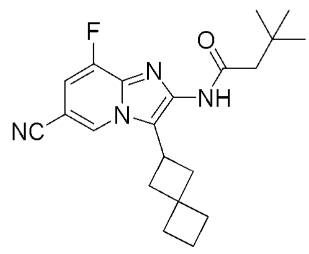
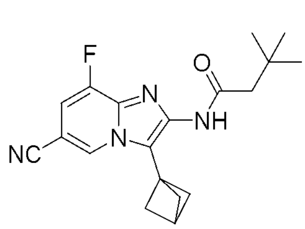

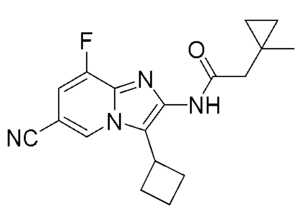
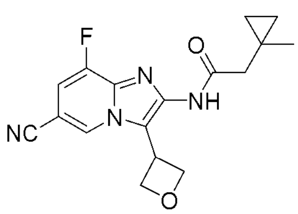
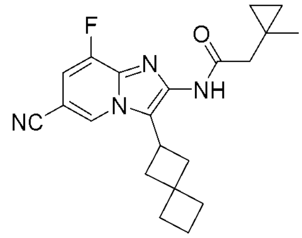
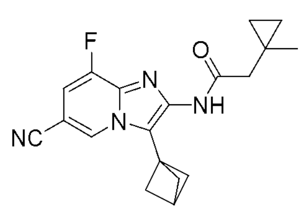

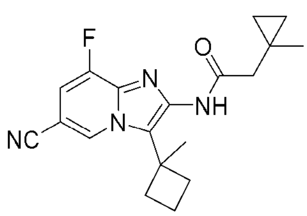
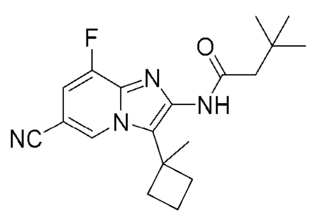
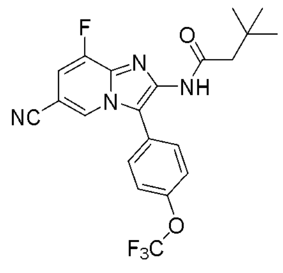
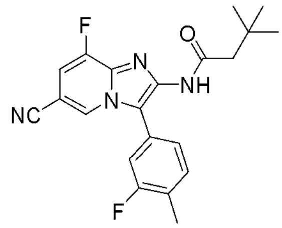

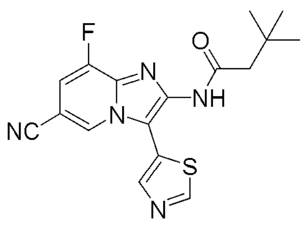
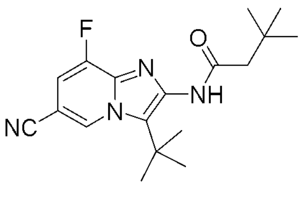
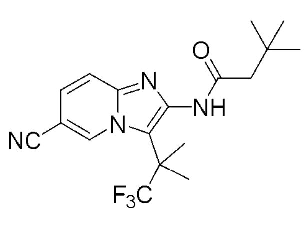
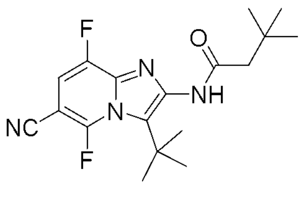

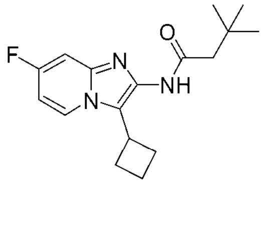
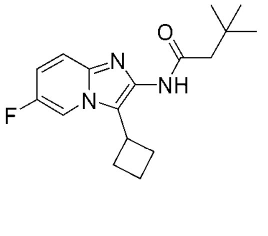
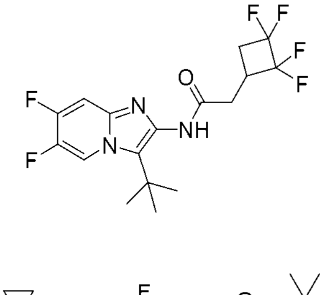
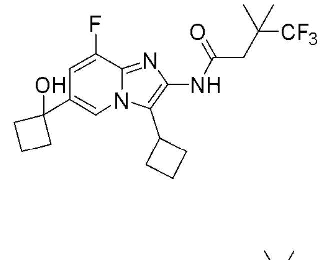

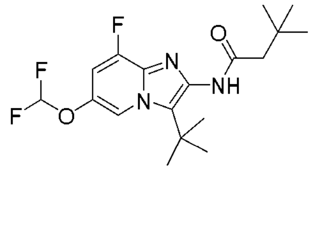
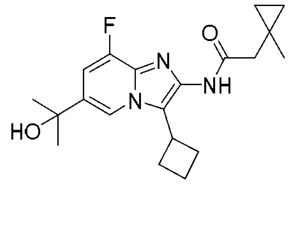
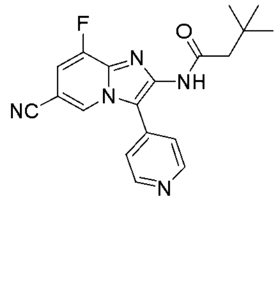
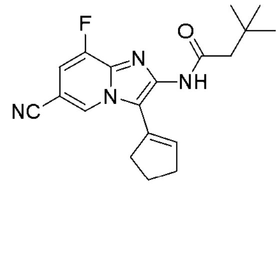

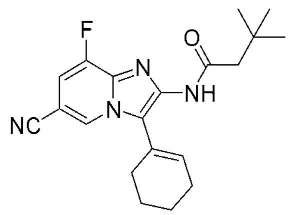 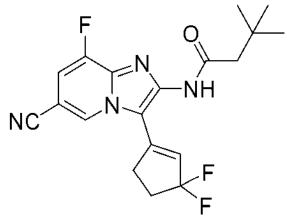 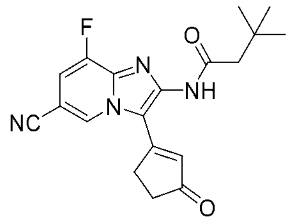 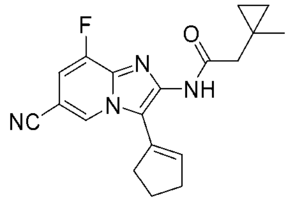

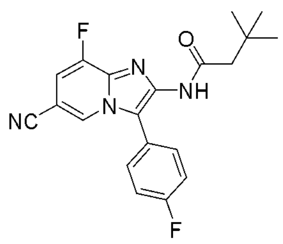 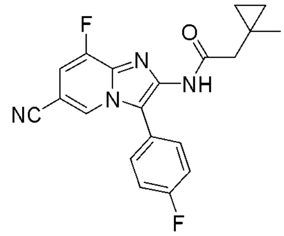 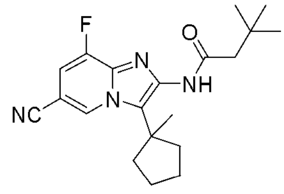 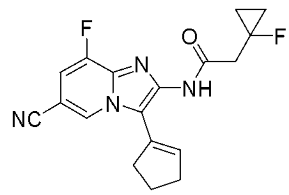

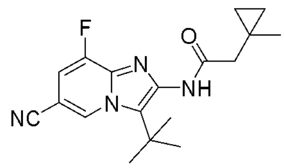 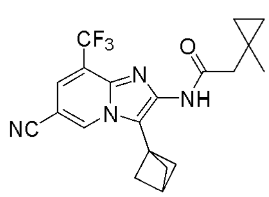 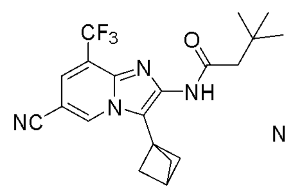 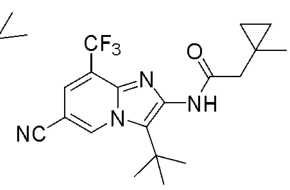

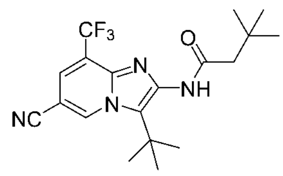 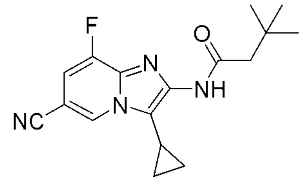 .

The present disclosure also provides a pharmaceutical composition, comprising a therapeutically or prophylactically effective amount of the compound of the present disclosure, or a stereoisomer or a pharmaceutically acceptable salt thereof; further, the pharmaceutical composition also comprises a pharmaceutically acceptable excipient.

The present disclosure also provides use of the above compound or a pharmaceutically acceptable salt thereof in the manufacture of a medicament for the treatment of a Kv7 potassium ion channel opener-related disease.

In some embodiments of the present disclosure, the above Kv7 potassium ion channel opener-related disease includes, but is not limited to, epilepsy, inflammatory pain, neuropathic pain, migraine, depression, anxiety disorder, stroke, Alzheimer's disease, neurodegenerative disease, neuronal hyperexcitability, complication caused by cocaine abuse, nicotine withdrawal syndrome, alcohol withdrawal syndrome, and tinnitus.

In some embodiments of the present disclosure, the above Kv7 potassium ion channel includes Kv7.2, Kv7.3, Kv7.4 and Kv7.5; preferably Kv7.2 and Kv7.3.

The present disclosure also provides the following synthetic routes:

route 1:

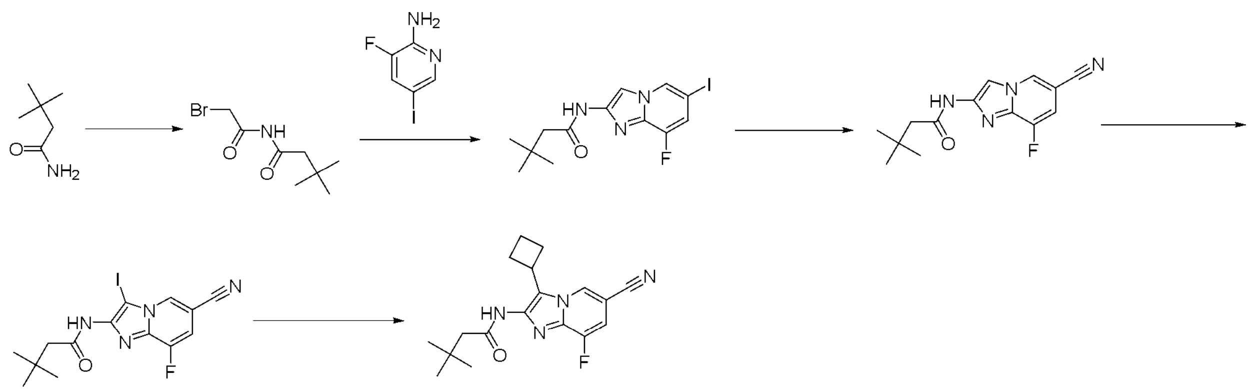

route 2:

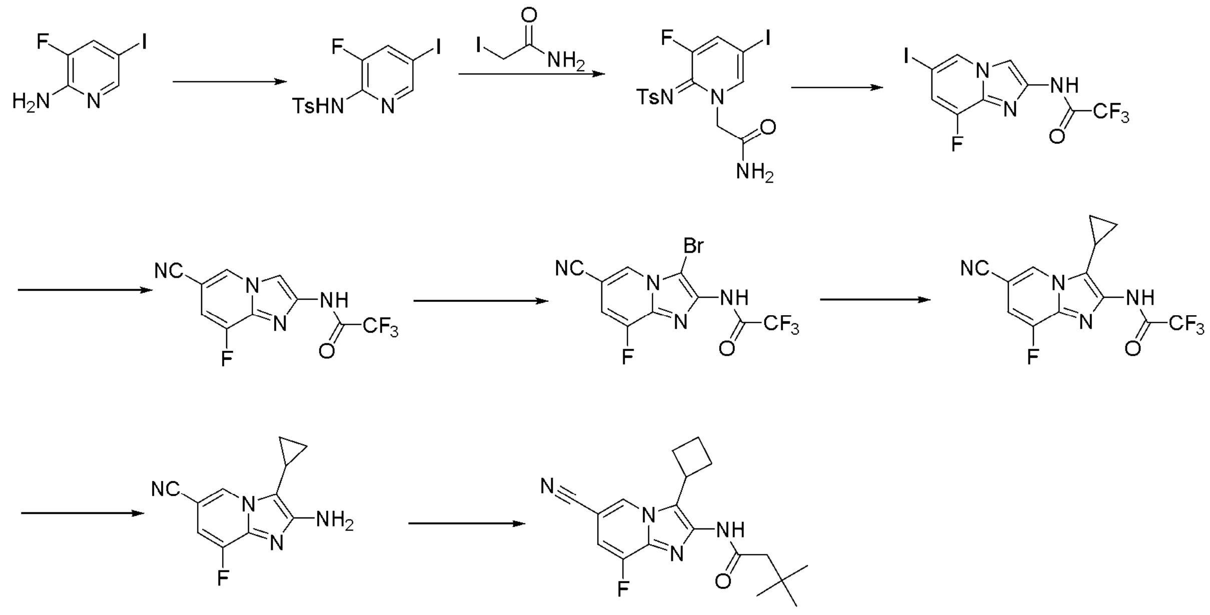

route 3:

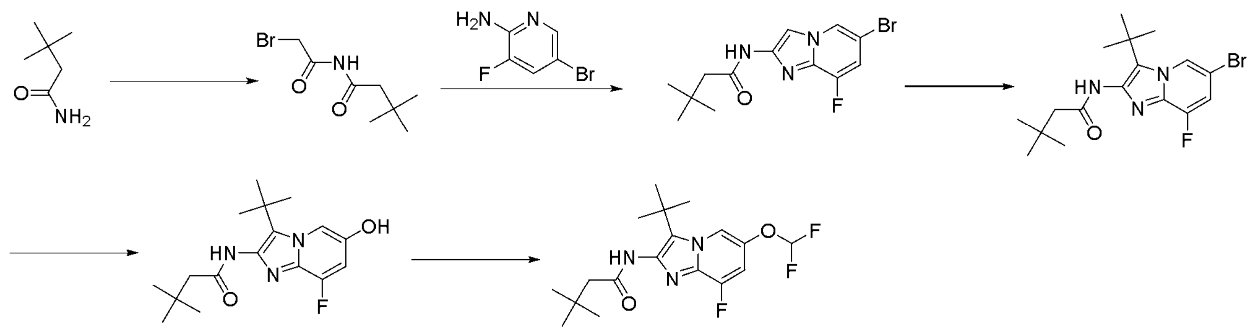

route 4:

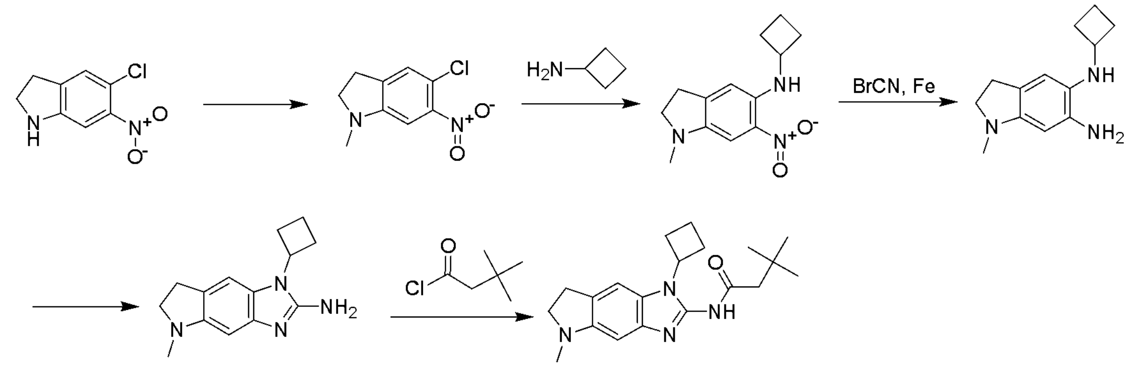


route 5:

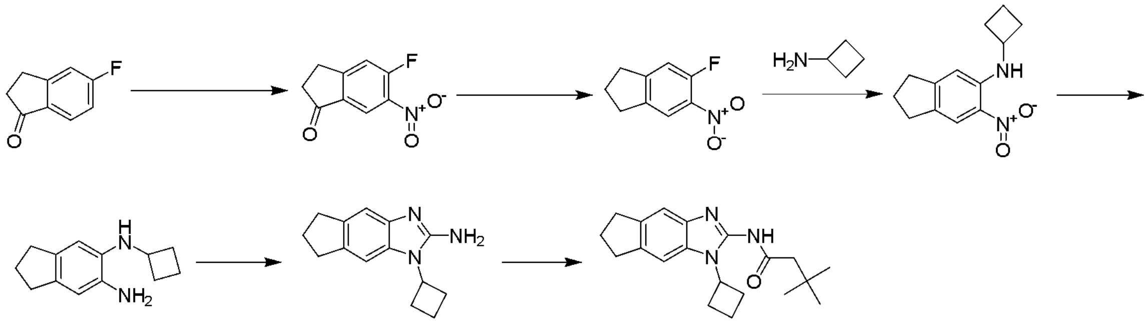

route 6:

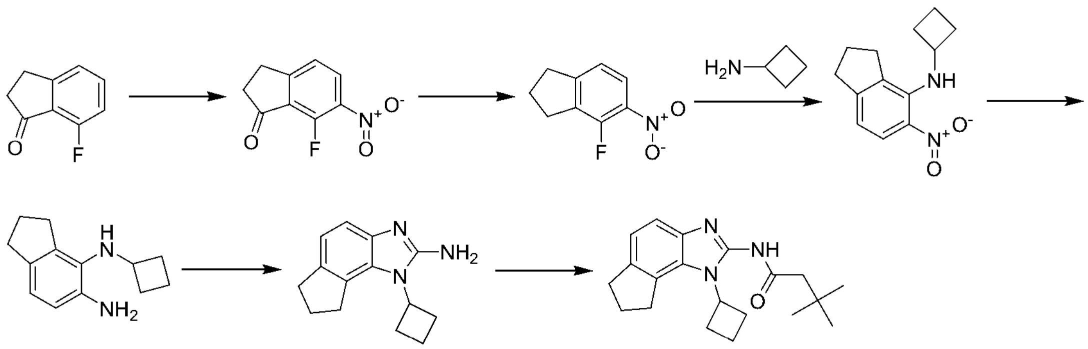

route 7:

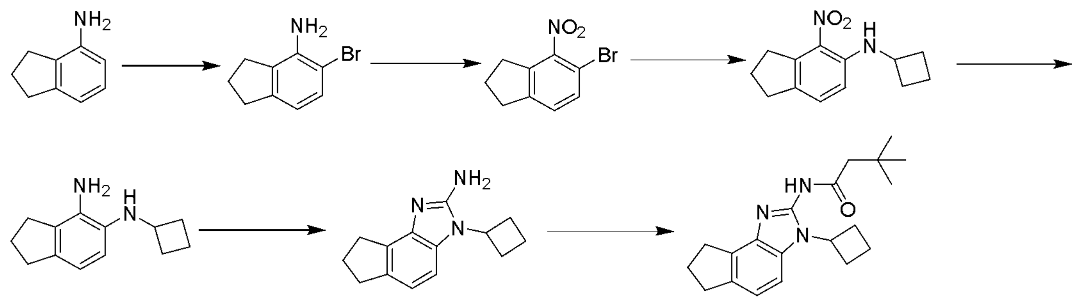

.

The present disclosure also provides the following assay methods:

Assay method 1: hKCNQ2/3 thallium flux assay

Purpose of the assay: To determine the in vitro concentration-response relationship of the compounds in a CHO cell line stably expressing hKCNQ2/3 using FLIPR thallium flux assay.

Cells and reagents of the assay:

(1) Cell line:

CHO cells stably transfected with hKCNQ2/3.

(2) Reagents of cell culture medium:

The information of the reagents of cell culture medium is shown in Table 1.

Table 1: Information of the reagents of cell culture medium

| Name | Supplier | Catalog number | Final concentration |
|---|---|---|---|
| F12 (culture medium) | / | / | / |
| FBS (fetal bovine serum) | / | / | 10% |
| Pen/Strep (penicillin, streptomycin) | Invitrogen | 15140-122 | 1% |
| G418 (antibiotic) | Invitrogen | 10131027 | 400 μg/mL |

(3) Cell culture:

The cell line was passaged three times a week at a split ratio of 1:2 to 1:3. When the cells grew to a confluence degree of greater than 80% in a T-75 cell flask, they were digested with 0.25% trypsin-EDTA solution for about 1 minute, and then the cells were transferred from the cell flask for passage. According to the split ratio, the cells were transferred to another T-75 cell flask containing complete cell growth culture medium. Note: In order to maintain the logarithmic growth of cells, the cell growth should be maintained in a subconfluent monolayer state. The cells were passaged every 2 to 3 days according to the cell doubling time.

Assay procedure:

(1) Day 1, cell preparation: The cells were digested according to the above method, and the cell density and viability were determined using Cell Countess. The volume of the single-cell suspension was adjusted with complete cell growth culture medium. Then, the hKCNQ2/3_CHO cells were seeded at a density of approximately 20,000 cells/well (30 μL/well) into a PDL-precoated 384-well plate. Then, the plate was incubated at 37 °C with 5% $CO_2$ humidified air in an incubator overnight.

(2) Day 2, compound preparation: A test compound was diluted to a storage concentration with dimethyl sulfoxide (DMSO), and stored in a freezer at -20 °C. A compound addition program was set up on the ECHO liquid workstation, and the compound was added to the plate using the ECHO liquid workstation. A positive control was prepared using Retigabine (RTG) with a maximum concentration of 100 μM, which was subjected to a 3-fold dilution, resulting in a total of 9 doses constituting the plate map required for plating. Prepare the plate using a stimulation buffer containing 5 mM $K^+$ and 1 mM $Tl^+$.

Note: 1) $K_2SO_4$ and $Tl_2SO_4$ were prepared in a 1× chloride-free buffer. When different voltage-gated potassium channels were involved, the concentrations of $Tl^+$ and $K^+$ were needed to be adjusted. The concentration of $Tl^+$ was approximately 0.5 mM to 5 mM, and the concentration of $K^+$ was approximately 5 mM to 30 mM. 2) Each 1 mM solution of $K_2SO_4$ and $Tl_2SO_4$ contained 2 mM $K^+$ and $Tl^+$. The final concentration should be calculated based on the ion concentrations. 3) TlCl was easily precipitated and a chloride-free buffer was required.

(3) Day 2, assay testing: When the cells reached the confluence degree, the cell analysis plate was taken out from the incubator. The assay buffer was prepared and the $Tl^+$ dye (Molecular Devices # R8222) was diluted. The culture medium was discarded using the liquid workstation Bravo and 25 μL of $Tl^+$ dye was added to each well. The plate was incubated for 1 hour, and then the cell analysis plate, composite plate, and FLIPR tip were placed in FLIPR$^{TETRA}$ (Molecular Devices, USA) for FLIPR assay. The tip was set up, and then the baseline was read for 60 seconds. Then, 12.5 μL from the composite plate (3×) was added to the cell analysis plate, and the data were recorded for at least 5 minutes at the same time.

(4) Data processing: The maximum signal was generated with FLIPR software. Excel (2013) and Prism 6.01 were used for data analysis. The data quality control was performed with S/B > 2.00 and Z-factor > 0.50. $IC_{50}$ or $EC_{50}$ was

defined as the midline between the lowest and the highest plateaus, and it was calculated using a four-parameter logistic model with Prism 6.01.

Assay method 2: Pharmacokinetic evaluation in mice

Assay method: Mix a test compound with a mixture of 5% DMSO, 60% polyethylene glycol (PEG400) and 35% water, vortex and sonicate to prepare a clear solution of 0.2 mg/mL. Filter the solution through a microporous filter membrane for later use. Select the male CD-1 mice weighing 25 to 35 g, and administer a candidate compound solution to them via intravenous injection at a dosage of 1 mg/kg. After fasting, administer the candidate compound solution to them orally at a dosage of 5 mg/kg. After the administration to the animals, collect 25 μL of blood at 0.083, 0.25, 0.5, 1, 2, 4, 8, 12, and 24 hours, and place the blood in commercially available anticoagulant tubes pre-filled with EDTA-K2. Centrifuge the tubes for 10 minutes to separate the plasma, and store them at -60 °C. Determine the content of the target compound in the plasma samples by LC/MS/MS, and calculate the pharmacokinetic parameters using Phoenix WinNonlin software (Pharsight, USA).

Definition of each parameter: IV: intravenous injection; PO: oral administration; $C_0$: instantaneous required concentration after intravenous injection; Cmax: peak plasma concentration after administration; $T_{max}$: time required to reach the peak drug concentration after administration; $T_{1/2}$: time required for plasma concentration to decrease by half; Vdss: apparent volume of distribution, which refers to the ratio constant between the amount of drug in the body and the plasma concentration when the drug reaches dynamic equilibrium in the body; Cl: clearance, which refers to the apparent volume of distribution of a drug cleared from the body per unit time; $T_{last}$: time of the last test point; $AUC_{0-last}$: area under the plasma concentration-time curve, which refers to the area enclosed by the plasma concentration curve on the time axis; F: a measure of the speed and extent to which a drug is absorbed into the blood circulation, which is an important indicator for evaluating the degree of drug absorption.

Technical effect

The compounds of the present disclosure bind well to both Kv7.2 protein and human Kv7.3 protein which has a highly homologous binding site. They have a significant agonistic effect on hKCNQ2/3 potassium ion channels and exhibit good pharmacokinetic properties, thus exerting a good pharmacodynamic effect.

Definition and description

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having specific substituents disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Some specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

"Pharmaceutical composition" means containing one or more of the compounds described herein, an isomer or a pharmaceutically acceptable salt thereof, and other component(s) such as physiologically/pharmaceuticalally acceptable carrier(s) and excipient(s). The purpose of a pharmaceutical composition is to facilitate administration to a living organism, promote the absorption of the active ingredient and thereby exerting its biological activity.

The term "pharmaceuticalally acceptable excipient" refers to those excipients that do not cause significant irritation to the organism and do not impair the biological activity and properties of the active compound. Suitable excipients are well known to those skilled in the art, such as carbohydrates, waxes, watersoluble and/or water-swellable polymers,

hydrophilic or hydrophobic materials, gelatin, oils, solvents, water, etc.

The pharmaceutical composition of the present disclosure can be prepared by combining the compound of the present disclosure with a suitable pharmaceutically acceptable excipient.

The pharmaceutical composition disclosed herein can be produced by a method well known in the art, such as a conventional mixing method, a dissolution method, a granulation method, a sugarcoating pill method, a grinding method, an emulsification method, a freeze drying method, and the like.

The term "treatment" refers to administering the compound of the present disclosure or a formulation thereof to improve or eliminate a disease or one or more symptoms related to the disease, and includes:

(i) suppressing a disease or disease state, that is, curbing its development;
(ii) alleviating a disease or disease state, that is, causing the disease or disease state to subside.

The term "prevention" refers to administering the compound of the present disclosure or a formulation thereof to prevent a disease or one or more symptoms related to the disease, including preventing the occurrence of a disease or disease state in mammals, particularly when such mammals are susceptible to the disease state but have not yet been diagnosed with the disease state.

The term "therapeutically effective amount" refers to the amount of the compound of the present disclosure used (i) to treat or prevent a particular disease, condition, or disorder; (ii) to alleviate, improve, or eliminate one or more symptoms of a particular disease, condition, or disorder; or (iii) to prevent or delay the onset of one or more symptoms of a particular disease, condition, or disorder described herein. The amount of the compound of the present disclosure constituting the "therapeutically effective amount" varies depending on the compound, the disease state and the severity thereof, the route of administration, and the age of the mammal to be treated, but may routinely be determined by those skilled in the art based on their own knowledge and the content of the present disclosure.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomers, (D)-isomers, (L)-isomers, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers are contained in a molecule and the relationship between the molecules is non-mirror image.

Unless otherwise specified, "(+)" means dextrorotation, "(-)" means levorotation, and "(±)" means racemization.

Unless otherwise specified, a wedged solid bond (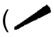) and a wedged dashed bond (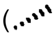) indicate the absolute configuration of a stereocenter; a straight solid bond (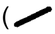) and a straight dashed bond (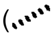) indicate the relative configuration of a stereocenter; a wavy line (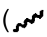) indicates a wedged solid bond (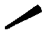) or a wedged dashed bond (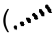); or a wavy line (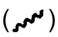) indicates a straight solid bond (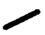) or a straight dashed bond (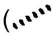).

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomeric excess" or "enantiomeric excess" means the difference between the relative percentages of two isomers or two enantiomers. For example, if one isomer or enantiomer is present in an amount of 90% and the other isomer or enantiomer is present in an amount of 10%, the isomeric or enantiomeric excess (ee value) is 80%.

Optically active (R)- and (S)-isomer, or D and L isomer can be prepared using chiral synthesis or chiral reagents or other conventional techniques. If one kind of enantiomer of certain compound disclosed herein is to be obtained, the pure desired enantiomer can be obtained by asymmetric synthesis or derivatization with chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomer which is then subjected to diastereomeric resolution through

the conventional method in the art to afford the pure enantiomer after recovery. In addition, the enantiomer and the diastereoisomer are generally isolated through chromatography which uses a chiral stationary phase and optionally combines with a chemical derivatization method (for example, carbamate generated from amine).

Some compounds of the present disclosure can exist as atropisomers, which are conformational isomers that occur when the rotation around a single bond in the molecule is prevented or significantly slowed down due to the steric interactions with other parts of the molecule. The compounds disclosed herein include all the atropisomers, which can be pure, single atropisomers, or mixtures enriched in one of the atropisomers, or nonspecific mixtures of each. Separation of the isomers can be permitted if the rotational potential around the single bond is sufficiently high and the interconversion between the conformations is sufficiently slow.

Compounds disclosed herein may contain an unnatural proportion of atomic isotopes at one or more of the atoms that constitutes the compounds. For example, a compound may be labeled with a radioisotope such as tritium ($^3H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "substituted" means that any one or more hydrogen atoms on a particular atom are substituted with a substituent, which can include deuterium and hydrogen variants, provided that the valence state of the particular atom is normal and the substituted compound is stable. When the substituent is oxygen (i.e., =O), it means that two hydrogen atoms are substituted. The term "optionally substituted" means that it may or may not be substituted, and unless otherwise specified, the type and number of substituents can be arbitrary on a chemically feasible basis.

When any variable (e.g., R) appears once or more than once in the composition or structure of a compound, its definition is independent in each case. Thus, for example, if a group is substituted with 0 to 2 R groups, the group can optionally be substituted with at most two R groups, and the R in each case has independent options. Furthermore, a combination of substituents and/or the variants thereof is allowed only in the case that such combination can result in a stable compound.

When the number of a linking group is 0, such as $-(CRR)_0-$, it indicates that the linking group is a single bond.

When the number of a substituent is 0, it means that the substituent is absent. For example, $-A-(R)_0$ indicates that the structure is actually -A.

When a substituent is vacant, it means that the substituent is absent. For example, when X is vacant in A-X, it means that the structure is actually A.

When one of the variables is a single bond, it means that the two groups it connects are directly connected. For example, when L in A-L-Z represents a single bond, it means that the structure is actually A-Z.

When an enumerated linking group does not indicate its linking direction, its linking direction is arbitrary. For example, when the linking group L in

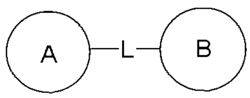

is -M-W-, the -M-W- can be linked to the ring A and the ring B in the same direction as the reading order from left to right to constitute

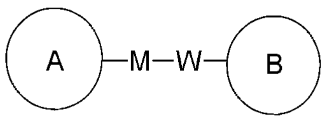

,

or can be linked to the ring A and the ring B in the reverse direction as the reading order from left to right to constitute

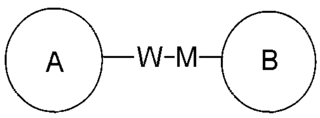

.

A combination of the linking groups, substituents and/or variants thereof is allowed only when such combination can result in a stable compound.

Unless otherwise specified, when a group has one or more connectable sites, any one or more sites of the group can be connected to other groups through chemical bonds. When the connection mode of the chemical bond is unlocated, and there is H atom(s) at a connectable site(s), when connecting the chemical bond, the number of hydrogen atoms at said site decreases correspondingly with the number of the connected chemical bond, and the group will become a group of corresponding valence. The chemical bond between the site and other groups can be represented by a straight solid bond

(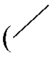),

a straight dashed bond

(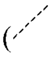),

or a wavy line

(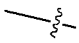).

For example, the straight solid bond in $-OCH_3$ indicates that the group is connected to other groups through the oxygen atom in the group; the straight dashed bond in

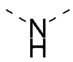

indicates that the group is connected to other groups through two ends of the nitrogen atom in the group; the wavy line in

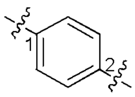

indicates that the group is connected to other groups through the carbon atoms at the 1- and 2-positions in the phenyl group;

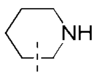

indicates that any connectable site on the piperidyl can be connected to other groups via one chemical bond, including at least four connection modes:

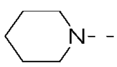, 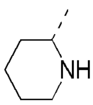, 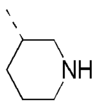 and 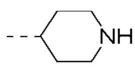.

Even if an H atom is drawn on -N-,

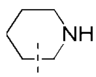

still includes the groups with the connection mode of

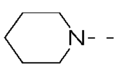.

It is just that, when one chemical bond is connected, the H at that site will be reduced by one, and it becomes the corresponding monovalent piperidyl group.

Unless otherwise specified, the number of atoms in a ring is usually defined as the number of the members of the ring. For example, "5- to 7-membered ring" refers to a "ring" in which 5 to 7 atoms are arranged in a circular arrangement.

Unless otherwise specified, the term "halo" or "halogen" on its own or as a part of another substituent refers to a fluorine, chlorine, bromine or iodine atom.

Unless otherwise specified, the term "$C_{1-6}$ alkyl" on its own or in combination with other terms is respectively used to refer to a radical of a straight or branched, saturated hydrocarbon group consisting of 1 to 6 carbon atoms. The $C_{1-6}$ alkyl includes $C_{1-2}$, $C_{1-3}$, $C_{1-4}$, $C_{1-5}$, $C_{2-3}$, $C_{2-4}$, $C_3$, $C_4$, $C_5$, $C_6$ alkyl, etc.; it can be monovalent (e.g., methyl), divalent (e.g., methylene), or polyvalent (e.g., methyne). Examples of $C_{1-6}$ alkyl include, but are not limited to, methyl (Me), ethyl (Et), propyl (including n-propyl and isopropyl), butyl (including n-butyl, isobutyl, s-butyl and t-butyl), etc.

Unless otherwise specified, the term "$C_{1-4}$ alkyl" is used to refer to a radical of a straight or branched, saturated hydrocarbon group consisting of 1 to 4 carbon atoms. The $C_{1-4}$ alkyl includes $C_{1-2}$, $C_{1-3}$, and $C_{2-3}$ alkyl, etc.; it can be monovalent (e.g., methyl), divalent (e.g., methylene), or polyvalent (e.g., methyne). Examples of $C_{1-4}$ alkyl include, but are not limited to, methyl (Me), ethyl (Et), propyl (including n-propyl and isopropyl), butyl (including n-butyl, isobutyl, s-butyl and t-butyl), etc.

Unless otherwise specified, the term "$C_{2-4}$ alkyl" on its own or in combination with other terms is respectively used to refer to a radical of a straight or branched, saturated hydrocarbon group consisting of 2 to 4 carbon atoms. The $C_{2-4}$ alkyl includes $C_{2-4}$, $C_{2-3}$ alkyl, etc.; it can be monovalent (e.g., ethyl), divalent (e.g., ethylene), or polyvalent (e.g., ethylidyne). Examples of $C_{2-4}$ alkyl include, but are not limited to, ethyl (Et), propyl (including n-propyl and isopropyl), butyl (including n-butyl, isobutyl, s-butyl, and t-butyl), etc.

Unless otherwise specified, the term "$C_{1-4}$ alkoxy" on its own or in combination with other terms respectively refers to those alkyl groups comprising 1 to 4 carbon atoms that are connected to the remainder of a molecule by an oxygen atom. The $C_{1-4}$ alkoxy includes $C_{1-3}$, $C_{1-2}$, $C_{2-4}$, $C_4$, and $C_3$ alkoxy, etc. It can be monovalent, divalent, or polyvalent. Examples of $C_{1-4}$ alkoxy include, but are not limited to, methoxy, ethoxy, propoxy (including n-propoxy and isopropoxy), butoxy (including n-butoxy, isobutoxy, s-butoxy and t-butoxy), etc.

Unless otherwise specified, the term "$C_{1-6}$ alkoxy" refers to those alkyl groups comprising 1 to 6 carbon atoms that are connected to the remainder of a molecule by an oxygen atom. The $C_{1-6}$ alkoxy includes $C_{1-4}$, $C_{1-3}$, $C_{1-2}$, $C_{2-6}$, $C_{2-4}$, $C_6$, $C_5$, $C_4$, $C_3$ alkoxy, etc. Examples of $C_{1-6}$ alkoxy include, but are not limited to, methoxy, ethoxy, propoxy (including n-propoxy and isopropoxy), butoxy (including n-butoxy, isobutoxy, s-butoxy and t-butoxy), pentoxy (including n-pentoxy, isopentoxy and neopentoxy), hexyloxy, etc.

Unless otherwise specified, "$C_{3-7}$ cycloalkyl" on its own or in combination with other terms respectively refers to a radical of a saturated cyclic hydrocarbon group consisting of 3 to 7 carbon atoms. It comprises monocyclic or polycyclic rings, wherein the polycyclic rings include spiro, fused, bridged rings, etc. The $C_{3-7}$ cycloalkyl includes $C_{3-5}$, $C_{3-6}$, $C_{4-5}$, $C_{4-6}$, $C_{5-7}$, $C_4$, $C_5$, $C_6$, $C_7$ cycloalkyl, etc.; it may be monovalent, divalent, or polyvalent. Examples of $C_{3-7}$ cycloalkyl include, but are not limited to, cyclopropyl, cyclobutyl, cyclopentyl, cyclohexyl,

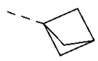, 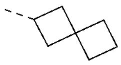,

etc.

Unless otherwise specified, "$C_{4-7}$ cycloalkyl" on its own or in combination with other terms respectively refers to a radical of a saturated cyclic hydrocarbon group consisting of 4 to 7 carbon atoms. It comprises monocyclic or polycyclic rings, wherein the polycyclic rings include spiro, fused, bridged rings, etc. The $C_{4-7}$ cycloalkyl includes $C_{4-5}$, $C_{4-6}$, $C_4$, $C_5$, $C_6$, $C_7$ cycloalkyl, etc.; it may be monovalent, divalent, or polyvalent. Examples of $C_{4-7}$ cycloalkyl include, but are not limited to, cyclobutyl, cyclopentyl, cyclohexyl,

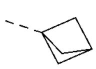, 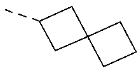,

etc.

Unless otherwise specified, "$C_{5-8}$ bridged cycloalkyl" refers to a radical of a saturated bridged cyclic hydrocarbon group consisting of 5 to 8 carbon atoms. The $C_{5-8}$ bridged cycloalkyl includes $C_{5-6}$, $C_{5-7}$, $C_{5-8}$, $C_{6-7}$, $C_{6-8}$, $C_{7-8}$ bridged cycloalkyl, etc.; it can be monovalent, divalent, or polyvalent. Examples of $C_{5-8}$ bridged cycloalkyl include, but are not limited to,

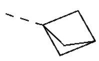,

etc.

Unless otherwise specified, "$C_{5-8}$ spirocycloalkyl" refers to a radical of a saturated spirocyclic hydrocarbon group consisting of 5 to 8 carbon atoms. The $C_{5-8}$ spirocycloalkyl includes $C_{5-6}$, $C_{5-7}$, $C_{5-8}$, $C_{6-7}$, $C_{6-8}$, $C_{7-8}$ spirocycloalkyl, etc.; it can be monovalent, divalent, or polyvalent. Examples of $C_{5-8}$ spirocycloalkyl include, but are not limited to,

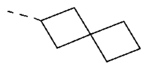, etc.

Unless otherwise specified, the term "4- to 7-membered heterocycloalkyl" on its own or in combination with other terms respectively refers to a radical of a saturated cyclic group consisting of 4 to 7 ring atoms, of which 1, 2, 3 or 4 ring atoms are heteroatoms independently selected from O, S and N, and the remainder are carbon atoms, wherein, the carbon atoms are optionally oxidized (i.e., C(O)), the nitrogen atoms are optionally quaternized, and the nitrogen and sulfur heteroatoms may be optionally oxidized (i.e., NO and $S(O)_p$, wherein p is 1 or 2). It includes monocyclic or polycyclic rings, wherein polycyclic rings include spiro, fused and bridged rings. Furthermore, with respect to the "4- to 7-membered heterocycloalkyl", the heteroatoms may occupy the connection site between the heterocycloalkyl and the remainder of the molecule. The 4- to 7-membered heterocycloalkyl includes 4- to 5-membered, 4- to 6-membered, 5- to 6-membered, 5- to 7-membered, 4-membered, 5-membered, 6-membered, 7-membered heterocycloalkyl, etc. Examples of 4- to 7-membered heterocycloalkyl include, but are not limited to, pyrrolidinyl, pyrazolidinyl, imidazolidinyl, tetrahydrothiophenyl (including tetrahydrothiophen-2-yl and tetrahydrothiophen-3-yl, etc.), tetrahydrofuranyl (including tetrahydrofuran-2-yl, etc.), tetrahydropyranyl, piperidyl (including 1-piperidyl, 2-piperidyl, 3-piperidyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidiny, 1,2-oxazinyl, 1,2-thiazinyl, hexahydropyridazinyl, homopiperazinyl, homopiperidyl,

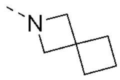,

etc.

Unless otherwise specified, "$C_{5-8}$ cycloalkenyl" refers to a radical of a partially unsaturated cyclic hydrocarbon group consisting of 5 to 8 carbon atoms containing at least one carbon-carbon double bond. It comprises monocyclic and bicyclic systems, wherein the bicyclic system includes spiro, fused and bridged rings, and any ring in this system is non-aromatic. The $C_{5-8}$ cycloalkenyl includes $C_{5-6}$, $C_{5-7}$, $C_{6-7}$, $C_{6-8}$, $C_{7-8}$ cycloalkenyl, etc.; it can be monovalent, divalent, or polyvalent. Examples of $C_{5-8}$ cycloalkenyl include, but are not limited to, cyclopentenyl, cyclopentadienyl, cyclohexenyl, cyclohexadienyl, etc.

Unless otherwise specified, "$C_{4-7}$ cycloalkenyl" refers to a radical of a partially unsaturated cyclic hydrocarbon group consisting of 4 to 7 carbon atoms containing at least one carbon-carbon double bond. It comprises monocyclic and polycyclic systems, wherein the polycyclic system includes spiro, fused and bridged rings, and any ring in this system is non-aromatic. The $C_{4-7}$ cycloalkenyl includes $C_{4-5}$, $C_{5-6}$, $C_4$, $C_5$, $C_6$, $C_7$ cycloalkenyl, etc.; it can be monovalent, divalent, or polyvalent. Examples of $C_{4-7}$ cycloalkenyl include, but are not limited to, cyclobutenyl, cyclopentenyl, cyclopentadienyl, cyclohexenyl, cyclohexadienyl, etc.

Unless otherwise specified, the terms "5- to 6-membered heteroaromatic ring" and "5- to 6-membered heteroaryl" herein can be used interchangeably. The term "5- to 6-membered heteroaryl" refers to a radical of a monocyclic group having a conjugated π-electron system consisting of 5 to 6 ring atoms, of which 1, 2, 3 or 4 ring atoms are heteroatoms independently selected from O, S and N, and the remainder are carbon atoms. The nitrogen atom in it is optionally quaternized, and the nitrogen and sulfur heteroatoms may be optionally oxidized (i.e., NO and $S(O)_p$, wherein p is 1 or 2). The 5- to 6-membered heteroaryl can be connected to the remainder of the molecule via heteroatoms or carbon atoms. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, 3-pyrrolyl, etc.), pyrazolyl (including 2-pyrazolyl, 3-pyrazolyl, etc.), imidazolyl (including N-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1H-1,2,3-triazolyl, 2H-1,2,3-triazolyl, 1H-1,2,4-triazolyl, 4H-1,2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridyl (including 2-pyridyl, 3-pyridyl, 4-pyridyl, etc.), pyrazinyl or pyrimidinyl (including 2-pyrimidinyl, 4-pyrimidinyl, etc.).

Unless otherwise specified, the term "4- to 7-membered heterocycloalkenyl" on its own or in combination with other terms respectively refers to a radical of a partially unsaturated cyclic group consisting of 4 to 7 ring atoms comprising at least one carbon-carbon double bond, of which 1, 2, 3 or 4 ring atoms are heteroatoms independently selected from O, S and N, and the remainder are carbon atoms, wherein, the carbon atoms are optionally oxidized (i.e., C(O)), the nitrogen atom is optionally quaternized, and the nitrogen and sulfur heteroatoms may be optionally oxidized (i.e., NO and $S(O)_p$,

wherein p is 1 or 2). It comprises monocyclic or polycyclic system, wherein the polycyclic system includes spiro, fused and bridged rings, and any ring in this system is non-aromatic. Furthermore, with respect to the "4- to 7-membered heterocycloalkenyl", the heteroatoms may occupy the connection site between the heterocycloalkenyl and the remainder of the molecule. The 4- to 7-membered heterocycloalkenyl includes 4- to 5-membered, 4- to 6-membered, 5- to 6-membered, 5-to 7-membered, 4-membered, 5-membered, 6-membered, 7-membered heterocycloalkenyl, etc. Examples of 4-to 7-membered heterocycloalkenyl include, but are not limited to,

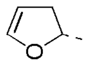 , 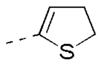 , 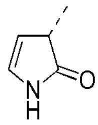 and 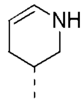 .

Unless otherwise specified, the terms "9- to 10-membered bicyclic heteroaromatic ring" and "9- to 10-membered bicyclic heteroaryl" disclosed herein can be used interchangeably. The term "9- to 10-membered heteroaryl" refers to a radical of a cyclic group having a conjugated $\pi$-electron system consisting of 9 or 10 ring atoms, of which 1, 2, 3 or 4 ring atoms are heteroatoms independently selected from O, S and N, and the remainder are carbon atoms. It is a bicyclic system in which each ring is aromatic. The nitrogen atom in it is optionally quaternized, and the nitrogen and sulfur heteroatoms may be optionally oxidized (i.e., NO and $S(O)_p$, wherein p is 1 or 2). The 9- to 10-membered heteroaryl can be connected to the remainder of the molecule via heteroatoms or carbon atoms. The 9- to 10-membered bicyclic heteroaryl includes 9-membered and 10-membered bicyclic heteroaryl. Examples of the 9- to 10-membered bicyclic heteroaryl include, but are not limited to, benzimidazolyl, benzopyrazolyl, pyridoimidazolyl, pyridopyrazolyl, etc.

Compounds disclosed herein can be prepared by a variety of synthetic methods well known to those skilled in the art, including the following enumerated embodiment, the embodiment formed by the following enumerated embodiment in combination with other chemical synthesis methods, and equivalent alternatives well known to those skilled in the art. Preferred embodiments include, but are not limited to examples disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In a single crystal X-Ray diffraction (SXRD), the diffraction intensity data of a cultivated single crystal are collected using a Bruker D8 venture diffractometer with a light source of CuK$\alpha$ radiation in a scanning mode of $\varphi/\omega$ scan; after collecting the relevant data, the crystal structure is further analyzed by the direct method (Shelxs97) to confirm the absolute configuration.

The abbreviations used in the present disclosure: prep-HPLC represents preparation and separation by high-performance liquid chromatography; DPPA represents diphenylphosphoryl azide.

All solvents used in the present disclosure are commercially available. Compounds are named according to general naming principles in the art or by ChemDraw® software, and commercially available compounds are named with their vendor directory names.

## BRIEF DESCRIPTION OF DRAWINGS

Fig. 1. Diagram of the binding mode of the compound A with KCNQ2;
Fig. 2. Diagram of the binding mode of the compound B with KCNQ2;
Fig. 3. Diagram of the binding mode of the compound C with KCNQ2;
Fig. 4. Diagram of the binding mode of the compound D with KCNQ2;
Fig. 5. Diagram of the binding mode of the compound E with KCNQ2;
Fig. 6. Diagram of the binding mode of the compound F with KCNQ2;
Fig. 7. Diagram of the binding mode of the compound G with KCNQ2.

## DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

Calculation Example 1

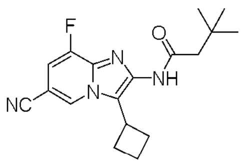

A

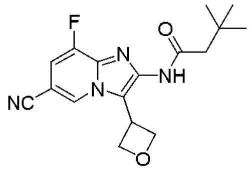

B

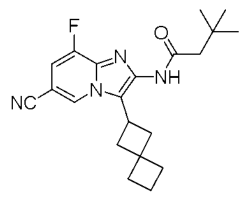

C

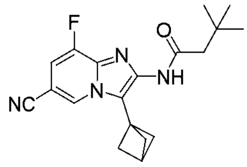

D

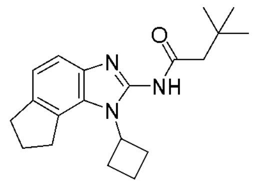

E

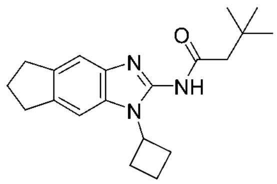

F

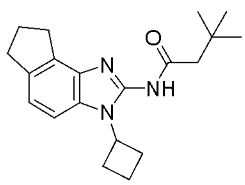

G

The Glide SP[1] and default options in Maestro (Schrödinger version 2022-1) were used. The co-crystal structure PDB: 7CR2 of KCNQ2 and Retigabine in the PDB database was selected and used as a docking template after energy optimization. To prepare the protein, hydrogen atoms were added using the protein preparation wizard module of Maestro[2], and the OPLS4 force field was used. For the preparation of a ligand, a three-dimensional structure of the molecule was generated using LigPrep, and the energy was minimized[3]. The conformation of the small molecule was sampled using the Confgen module. A cubic docking grid with a side length of 25Å*25Å*25Å was generated with Retigabine in 7CR2 as a centroid. During the molecular docking process, the compounds were placed in the protein model respectively, and the interaction type between the protein receptor and a ligand was analyzed, and then the reasonable docking conformation was selected and saved according to the calculated docking score and binding mode. The binding modes of the compounds A to G are shown in Fig. 1 to Fig. 7.

[1] Glide, Schrödinger, LLC, New York, NY, 2022.
[2] Maestro, Schrödinger, LLC, New York, NY, 2022.
[3] LigPrep, Schrödinger, LLC, New York, NY, 2022.

Conclusion: The compounds of the present disclosure have a good binding with KCNQ2.

Example 1

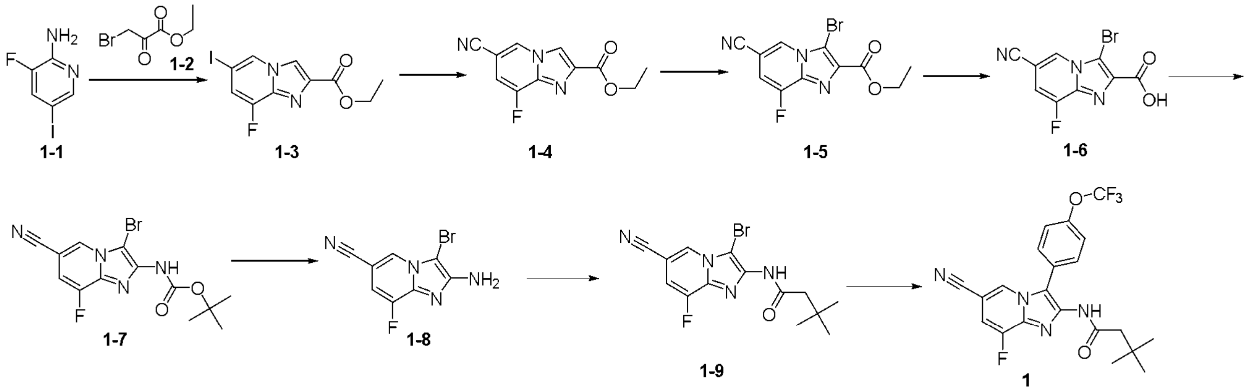

1-1, 1-2, 1-3, 1-4, 1-5, 1-6, 1-7, 1-8, 1-9, 1

Step 1: Compound 1-1 (10 g, 42.02 mmol) was added to ethanol (200 mL), and 1-2 (9.83 g, 50.42 mmol) was added. The mixture was heated to 85 °C and reacted for 12 hours under nitrogen. The reaction solution was cooled to room temperature, concentrated under reduced pressure to give a crude product. The crude product was separated by column

chromatography (petroleum ether/ethyl acetate = 100/0 to 10/90, to dichloromethane/methanol = 100/0 to 5/95, V/V) to give compound 1-3. LCMS(ESI): 335.0 $[M+H]^+$.

Step 2: Compound 1-3 (1.14 g, 3.41 mmol) was added to N,N-dimethylacetamide (20 mL). Zinc cyanide (601.01 mg, 5.12 mmol), tetrakis(triphenylphosphine)palladium (197.16 mg, 170.62 μmol), and [1,1'-bis(diphenylphosphino) ferrocene]palladium(II) chloride (124.84 mg, 170.62 μmol) were successively added. The reaction solution was heated to 130 °C and reacted for 2 hours under nitrogen. The reaction solution was cooled to room temperature, and then filtered under reduced pressure. The filtrate was collected, and concentrated under reduced pressure to give a crude product. The crude product was separated by column chromatography (petroleum ether/ethyl acetate, 100/0 to 50/50, V/V) to give compound 1-4. LCMS(ESI): 234.0 $[M+H]^+$.

Step 3: Compound 1-4 (1.5 g, 6.43 mmol) was added to N,N-dimethylformamide (20 mL). The mixture was cooled to 0 °C in an ice-water bath. Then, N-bromosuccinimide (1.37 g, 7.72 mmol) was added. The reaction solution was warmed to 25 °C, and reacted for 2 hours under nitrogen. Water (40 mL) was added to the reaction solution, and a solid was precipitated out. The mixture was filtered under reduced pressure, and the filter cake was collected to give compound 1-5. LCMS(ESI): 312.0 $[M+H]^+$.

Step 4: Compound 1-5 (1 g, 3.20 mmol) was added to methanol (1.5 mL), tetrahydrofuran (3 mL), and water (3 mL). Then, lithium hydroxide (153.48 mg, 6.41 mmol) was added. The reaction solution was reacted at 25 °C under nitrogen for 1 hour. After the reaction was completed, hydrochloric acid (10 mL, 2 M) was added dropwise to quench the reaction, and then water (100 mL) was added. The resulting mixture was extracted with ethyl acetate (100 mL), washed with saturated brine, dried over anhydrous sodium sulfate, filtered under reduced pressure, and evaporated to dryness to give compound 1-6. LCMS(ESI): 284.1 $[M+H]^+$.

Step 5: Compound 1-6 (270 mg, 950.57 μmol) was added to tert-butanol (5 mL). N,N-Diisopropylethylamine (147.42 mg, 1.14 mmol) and diphenylphosphoryl azide (313.91 mg, 1.14 mmol) were successively added. The reaction solution was heated to 85 °C and reacted for 12 hours under nitrogen. The reaction solution was concentrated under reduced pressure, and ethyl acetate (100 mL) was added to the residue. The resulting mixture was washed with water (100 mL × 3). The organic phase was collected, and then washed with saturated brine (100 mL), dried over anhydrous sodium sulfate, and concentrated under reduced pressure to give a crude compound. The crude product was separated by column chromatography (petroleum ether/ethyl acetate, 100/0 to 20/80, V/V) to give compound 1-7. LCMS(ESI): 356.9 $[M+H]^+$.

Step 6: Compound 1-7 (50 mg, 140.78 μmol) was added to dichloromethane (1 mL), and trifluoroacetic acid (0.5 mL) was added at 0 °C. The reaction solution was reacted at 25 °C under nitrogen for 1 hour. The reaction solution was concentrated under reduced pressure to give a crude product. Water (10 mL) and ethyl acetate (20 mL) were added. The pH was adjusted to alkaline using saturated sodium bicarbonate. The resulting mixture was washed with water (10 mL × 3). The organic phase was collected, and then washed with saturated brine (10 mL), dried over anhydrous sodium sulfate, and concentrated under reduced pressure to give a crude product. The crude product was separated by column chromatography (petroleum ether/ethyl acetate, 100/0 to 50/50, V/V) to give compound 1-8. LCMS(ESI): 254.9 $[M+H]^+$.

Step 7: Compound 1-8 (35 mg, 137.23 μmol) was added to acetone (4 mL). N,N-Diisopropylethylamine (19.51 mg, 150.95 μmol) and tert-butylacetyl chloride (19.40 mg, 144.09 μmol) were successively added at 0 °C. The reaction solution was reacted at 25 °C under nitrogen for 2 hours. Water (25 mL) was slowly added to the reaction solution, and the resulting mixture was extracted with ethyl acetate (25 mL × 2). The organic phases were combined, and then washed with saturated brine (25 mL), dried over anhydrous sodium sulfate, and filtered. The filtrate was concentrated under reduced pressure to give a crude product. The crude product was separated by column chromatography (petroleum ether/ethyl acetate, 100/0 to 50/50, V/V) to give compound 1-9. LCMS(ESI): 353.0 $[M+H]^+$.

Step 8: Compound 1-9 (32 mg, 90.60 μmol) was added to dioxane (5 mL). Then, 4-(trifluoromethoxyl) phenylboronic acid (27.99 mg, 135.90 μmol), [1,1'-bis(diphenylphosphino)ferrocene]dichloropalladium(II) (6.63 mg, 9.06 μmol), potassium carbonate (25.04 mg, 181.21 μmol), and water (0.5 mL) were added. The mixture was stirred at 85 °C under nitrogen for 2 hours. Water (20 mL) was added to the reaction solution, and the resulting mixture was extracted with ethyl acetate (20 mL × 2). The organic phases were combined, and then washed with saturated brine (20 mL), dried over anhydrous sodium sulfate, and filtered. The filtrate was concentrated under reduced pressure to give a crude product. The crude product was separated by Prep-HPLC (chromatographic column: 2-Phenomenex Gemini C18 75*40mm*3μm; mobile phase: [water (ammonia + ammonium bicarbonate)-acetonitrile]; acetonitrile ratio: 50% to 80%) to give compound 1. LCMS(ESI): 435.0 $[M+H]^+$. 1H NMR(400 MHz,$CDCl_3$)δ ppm 8.27(s, 1 H)7.54(d, J=8.8 Hz, 2 H)7.42(br d, J=8.8 Hz, 2 H)7.11(d, J=8.8 Hz, 1 H)2.20(br s, 2 H)0.99(s, 9 H).

Example 2

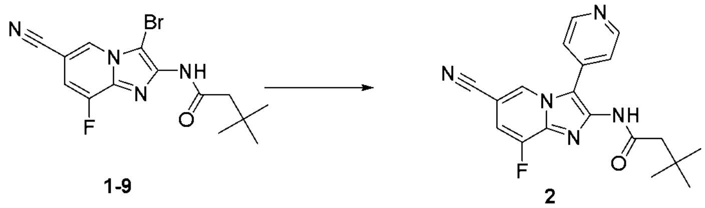

1-9 2

Compound 1-9 (0.11 g, 311.45 μmol) was added to dioxane (5 mL), and then 4-pyridylboronic acid (76.56 mg, 622.90 μmol), [1,1'-bis(diphenylphosphino)ferrocene]dichloropalladium(II) (35.99 mg, 31.14 μmol), potassium carbonate (86.09 mg, 622.90 μmol), and water (0.5 mL) were added. The mixture was stirred at 85 °C under nitrogen for 2 hours. Water (20 mL) was added to the reaction solution, and the resulting mixture was extracted with ethyl acetate (20 mL × 2). The organic phases were combined, and then washed with saturated brine (20 mL), dried over anhydrous sodium sulfate, and filtered. The filtrate was concentrated under reduced pressure to give a crude product. The crude product was separated by Prep-HPLC (chromatographic column: Welch Xtimate C18 150*25mm*5 μm; mobile phase: [water (ammonia + ammonium bicarbonate) - acetonitrile]; acetonitrile ratio: 18% to 48%) to give compound 2. LCMS(ESI): 352.1 $[M+H]^+$. $^1$H NMR(400 MHz, $CDCl_3$)δ ppm 8.84(d, J=4.0 Hz, 2 H), 8.40(s, 1 H), 7.94(s, 1 H), 7.43(d, J=5.6 Hz, 2 H), 7.16(d, J=8 Hz, 1 H), 2.23(s, 2 H), 1.03(s, 9 H).

Example 3

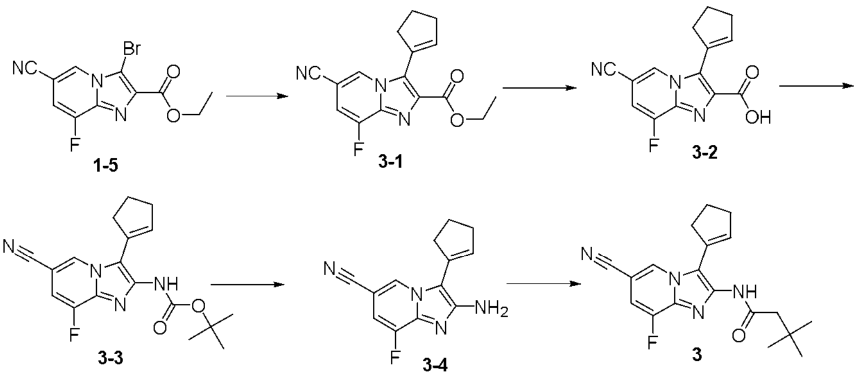

1-5 3-1 3-2 3-3 3-4 3

Step 1: Compound 1-5 (1.33 g, 4.26 mmol) was added to dioxane (20 mL). 2-cyclopentenyl-4,4,5,5-tetramethyl-1,3,2-dioxaborolane (1.65 g, 8.52 mmol), tetrakis(triphenylphosphine)palladium (246.22 mg, 213.08 μmol), potassium carbonate (1.18 g, 8.52 mmol), and water (2 mL) were added successively. The reaction solution was heated to 85 °C and reacted for 2 hours under nitrogen. Ethyl acetate (200 mL) was added to the reaction solution, and the mixture was washed with water (200 mL × 3). The organic phase was collected, and then washed with saturated brine (100 mL), dried over anhydrous sodium sulfate, and concentrated under reduced pressure to give a crude product. The crude product was separated by column chromatography (petroleum ether/ethyl acetate, 100/0 to 20/80, V/V) to give compound 3-1. LCMS(ESI): 300.1 $[M+H]^+$.

Step 2: Compound 3-1 (0.95 g, 3.17 mmol) was added to methanol (1.5 mL), tetrahydrofuran (3 mL), and water (3 mL). Lithium hydroxide (152.03 mg, 6.35 mmol) was added, and the reaction solution was reacted at 25 °C under nitrogen for 1 hour. After the reaction was completed, hydrochloric acid (10 mL, 2 M) was added dropwise to quench the reaction, and then water (100 mL) was added. The resulting mixture was extracted with ethyl acetate (100 mL), washed with saturated brine, dried over anhydrous sodium sulfate, and concentrated under reduced pressure to give crude compound 3-2. LCMS(ESI): 272.1 $[M+H]^+$.

Step 3: Compound 3-2 (0.86 g, 3.17 mmol) was added to tert-butanol (20 mL). N,N-Diisopropylethylamine (491.71 mg, 3.80 mmol) and diphenylphosphoryl azide (1.05 g, 3.80 mmol) were successively added. The reaction solution was heated to 85 °C and reacted for 6 hours under nitrogen. Ethyl acetate (100 mL) was added to the reaction solution. The resulting mixture was washed with water (100 mL × 3). The organic phase was collected, and then washed with saturated brine (100 mL), dried over anhydrous sodium sulfate, and concentrated under reduced pressure to give a crude product. The crude product was separated by column chromatography (petroleum ether/ethyl acetate, 100/0 to 20/80, V/V) to give compound 3-3. LCMS(ESI): 343.3 $[M+H]^+$.

Step 4: Compound 3-3 (0.2 g, 584.17 μmol) was added to dichloromethane (1 mL), and trifluoroacetic acid (0.5

mL) was added at 0 °C. The reaction solution was reacted at 25 °C under nitrogen for 1 hour. The reaction solution was concentrated under reduced pressure to give a crude compound. Water (10 mL) and ethyl acetate (20 mL) were added. The pH was adjusted to alkaline with saturated sodium bicarbonate. The resulting mixture was washed with water (10 mL × 3). The organic phase was collected, and then washed with saturated brine (10 mL), dried over anhydrous sodium sulfate, and concentrated under reduced pressure to give a crude product. The crude product was separated by column chromatography (petroleum ether/ethyl acetate, 100/0 to 50/50, V/V) to give compound 3-4. LCMS(ESI): 243.1 $[M+H]^+$.

Step 5: Compound 3-4 (100 mg, 412.79 μmol) was added to acetone (5 mL). N,N-diisopropylethylamine (58.68 mg, 454.07 μmol) and tert-butylacetyl chloride (58.34 mg, 433.43 μmol) were successively added at 0 °C. The reaction solution was reacted at 25 °C under nitrogen for 2 hours. Water (50 mL) was slowly added to the reaction solution. The resulting mixture was extracted with ethyl acetate (50 mL × 2), washed with saturated brine, dried over anhydrous sodium sulfate, and filtered. The filtrate was concentrated under reduced pressure to give a crude product. The crude product was separated by column chromatography (petroleum ether/ethyl acetate, 100/0 to 50/50, V/V) to give compound 3. LCMS(ESI): 341.2 $[M+H]^+$. $^1H$ NMR (400 MHz, $CDCl_3$)δ ppm 8.61(m, 1 H), 8.45(s, 1 H), 7.12(d, J=8.4 Hz, 1 H), 6.20(s, 1 H), 2.69 - 2.67(m, 4 H), 2.31(s, 2 H), 2.14 - 2.07(m, 2 H), 1.10(s, 9 H).

Example 4

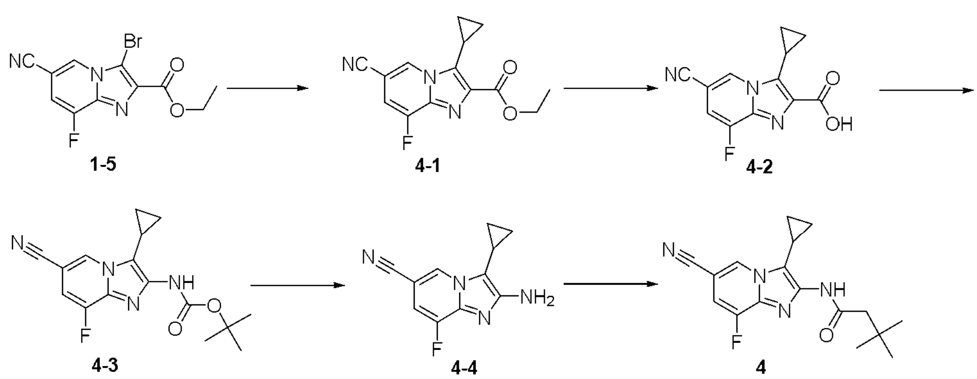

1-5 4-1 4-2

4-3 4-4 4

Step 1: Compound 1-5 (1 g, 3.20 mmol) was added to dioxane (10 mL). Potassium cyclopropyltrifluoroborate (948.28 mg, 6.41 mmol), [1,1'-bis(diphenylphosphino)ferrocene]dichloropalladium(II) (234.45 mg, 320.42 μmol), and potassium carbonate (885.69 mg, 6.41 mmol) were successively added. The reaction solution was heated to 85 °C and reacted for 24 hours under nitrogen. Ethyl acetate (200 mL) was added to the reaction solution, and the resulting mixture was washed with water (200 mL × 3). The organic phase was collected, washed with saturated brine (200 mL), dried over anhydrous sodium sulfate, and concentrated under reduced pressure to give a crude compound. The crude product was separated by column chromatography (petroleum ether/ethyl acetate, 100/0 to 20/80, V/V) to give compound 4-1. LCMS(ESI): 274.1 $[M+H]^+$.

Step 2: Compound 4-1 (0.69 g, 2.53 mmol) was added to methanol (1.5 mL), tetrahydrofuran (3 mL), and water (3 mL). Lithium hydroxide (120.95 mg, 5.05 mmol) was added, and the reaction solution was reacted at 25 °C under nitrogen for 1 hour. After the reaction was completed, hydrochloric acid (10 mL, 2 M) was added dropwise to quench the reaction, and then water (100 mL) was added. The resulting mixture was extracted with ethyl acetate (100 mL), washed with saturated brine, dried over anhydrous sodium sulfate, and concentrated under reduced pressure to give crude compound 4-2. LCMS(ESI): 246.1$[M+H]^+$.

Step 3: Compound 4-2 (0.4 g, 1.63 mmol) was added to tert-butanol (10 mL). N,N-Diisopropylethylamine (252.99 mg, 1.96 mmol) and DPPA (538.71 mg, 1.96 mmol) were successively added. The reaction solution was heated to 85 °C and reacted for 6 hours under nitrogen. Ethyl acetate (100 mL) was added to the reaction solution, and the resulting mixture was washed with water (100 mL × 3). The organic phase was collected, and then washed with saturated brine (100 mL), dried over anhydrous sodium sulfate, and concentrated under reduced pressure to give a crude product. The crude product was separated by column chromatography (petroleum ether/ethyl acetate, 100/0 to 20/80, V/V) to give compound 4-3. LCMS(ESI): 317.1 $[M+H]^+$.

Step 4: Compound 4-3 (106 mg, 335.09 μmol) was added to dichloromethane (1 mL), and trifluoroacetic acid (0.5 mL) was added at 0 °C. The reaction solution was reacted at 25 °C under nitrogen for 1 hour. The reaction solution was concentrated to give a crude compound. Water (10 mL) and ethyl acetate (20 mL) were added. The pH was adjusted to alkaline with saturated sodium bicarbonate. The resulting mixture was washed with water (10 mL × 3). The organic phase was collected, and then washed with saturated brine (100 mL), dried over anhydrous sodium sulfate, and concentrated

under reduced pressure to give a crude product. The crude product was separated by column chromatography (petroleum ether/ethyl acetate, 100/0 to 50/50, V/V) to give compound 4-4. LCMS(ESI):217.1 $[M+H]^+$.

Step 5: Compound 4-4 (64 mg, 296.00 μmol) was added to acetone (5 mL). N,N-Diisopropylethylamine (42.08 mg, 325.60 μmol) and tert-butylacetyl chloride (43.83 mg, 325.60 μmol) were successively added at 0 °C. The reaction solution was reacted at 25 °C under nitrogen for 2 hours. Water (50 mL) was slowly added to the reaction solution, and the resulting mixture was extracted with ethyl acetate (50 mL × 2). The organic phases were combined, and then washed with saturated brine (50 mL), dried over anhydrous sodium sulfate, and filtered. The filtrate was concentrated under reduced pressure to give a crude product. The crude product was separated by column chromatography (petroleum ether/ethyl acetate, 100/0 to 50/50, V/V) to give compound 4. LCMS(ESI): 315.2 $[M+H]^+$. $^1H$ NMR(400 MHz, $CDCl_3$)δ 8.51(s, 1 H),7.80(s, 1 H), 7.06(d, J=8.0 Hz, 1 H), 2.34(s, 2 H), 2.10 - 2.01(m, 1 H), 1.15(s, 9 H), 1.14 - 1.07(m, 2 H), 0.63 - 0.57(m, 2 H).

Example 5

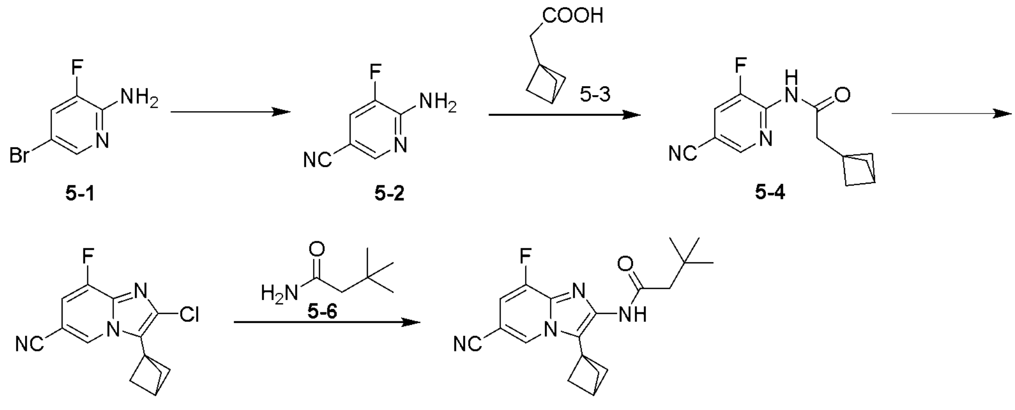


5-1 5-2 5-4 5-5 5

Step 1: Tetrahydrofuran (10 mL), water (40 mL), compound 5-1 (2 g, 10.47 mmol), and zinc cyanide (1.23 g, 10.47 mmol) were added to a 100 mL single-necked flask, and stirring was started. The reaction system was purged with nitrogen, and then (2-di-tert-butylphosphino-2',4',6'-triisopropyl-1,1'-biphenyl)(2'-amino-1,1'-biphenyl-2-yl)palladium(II) methanesulfonate (831.80 mg, 1.05 mmol) was added. The mixture was heated to 45 °C, and reacted for 15 hours. The reaction solution was cooled to room temperature. Water (50 mL) was added, and the resulting mixture was extracted with added ethyl acetate (30 mL × 2). The resulting organic phase was washed with saturated brine (30 mL), dried over anhydrous sodium sulfate, and filtered. The filtrate was concentrated under reduced pressure. The residue was separated and purified by silica gel column chromatography (V/V petroleum ether: ethyl acetate = 1:0 to 2:3) to give a crude product. Methyl tert-butyl ether (5 mL) was added to the crude product. The mixture was stirred for 10 min, and then filtered. The filter cake was dried to give 5-2. LCMS(ESI): 138.0 $[M+H]^+$. $^1H$ NMR(400 MHz, $CDCl_3$)δ ppm 8.19(s, 1H), 7.43 - 7.40(m, 1H), 5.21(brs, 2H).

Step 2: Compound 5-3 (331.22 mg, 2.63 mmol) was added to dichloromethane (12 mL), and stirring was started. Then, pyridine (941.9 μL, 11.67 mmol), n-butylphosphonic anhydride (2,4,6-tributyl-1,3,5,2,4,6-trioxatriphosphinane 2,4,6-trioxide, CAS: 163755-62-2, 6.31 g, 8.75 mmol, 50% ethyl acetate solution), and compound 5-2 (400 mg, 2.92 mmol) were successively added. The mixture was reacted at room temperature (15 °C) for 48 hours. Water (20 mL) and dichloromethane (20 mL) were added to the reaction system, and then the layers were separated. The resulting organic phase was dried over added anhydrous sodium sulfate, and filtered. The filtrate was concentrated under reduced pressure to give a crude product. The crude product was separated and purified by silica gel column chromatography (V/V petroleum ether: ethyl acetate = 1:0 to 1:1) to give compound 5-4. LCMS(ESI): 246.2 $[M+H]^+$. $^1H$ NMR(400 MHz, $CDCl_3$)δ ppm 8.50(d, J= 1.6Hz, 1H), 7.75(brs, 1H), 7.69(dd, J= 9.2 Hz, 1.6Hz, 1H), 2.86(s, 2H), 2.54(s, 1H), 1.87(s, 6H).

Step 3: Compound 5-4 (20 mg, 81.55 μmol) was added to chlorobenzene (4 mL), and stirring was started. Then, thionyl chloride (29.61 μL, 407.74 μmol) and pyridine (23.04 μL, 285.42 μmol) were successively added. The mixture was heated to 90 °C and reacted for 13 hours. The reaction solution was concentrated under reduced pressure at 50 °C. The crude product was separated and purified by silica gel column chromatography (gradient elution: petroleum ether: ethyl acetate = 100:0 to 85:15) to give compound 5-5. LCMS(ESI): 262.1$[M+H]^+$. $^1H$ NMR(400 MHz, $CDCl_3$)δ ppm 8.38(s, 1H), 7.05(dd, J= 9.2 Hz,1.2 Hz, 1H), 2.77(s, 1H), 2.49(s, 6H).

Step 4: Compound 5-5 (50 mg, 191.07 μmol) and compound 5-6 (264.08 mg, 2.29 mmol) were added to dioxane (5 mL), and stirring was started. The reaction system was purged with nitrogen, and then cesium carbonate (186.77 mg,

573.22 μmol), potassium iodide (95.15 mg, 573.22 μmol), and (2-dicyclohexylphosphino-3,6-dimethoxy-2',4',6'-triisopropyl-1,1'-biphenyl)(2'-amino-1,1'-biphenyl-2-yl)palladium(II) methanesulfonate (34.64 mg, 38.21 μmol) were successively added. The mixture was heated to reflux and reacted for 3 hours. The reaction solution was cooled to room temperature, and then saturated ammonium chloride solution (20 mL) was added to the reaction system. Then, the mixture was extracted with added ethyl acetate (20 mL × 2). The resulting organic phase was washed with saturated brine (15 mL), then dried over added anhydrous sodium sulfate, and filtered. The filtrate was concentrated under reduced pressure. The residue was separated and purified by silica gel column chromatography (V/V petroleum ether: ethyl acetate = 1:0 to 1:1) to give a crude product. The crude product was then separated by preparative high-performance liquid chromatography (chromatographic column: WePure Biotech XP tC18 100mm*30mm*10μm; mobile phase: A (water, containing 10 mM ammonium bicarbonate) and B (acetonitrile); gradient: B%: 32% to 62%) to give compound 5. LCMS(ESI): 341.1[M+H]$^+$. $^1$H NMR(400 MHz, $CDCl_3$)δ ppm 8.42(s, 1H), 7.33(brs, 1H),7.02(d, J= 9.2 Hz, 1H), 2.73(s, 1H), 2.42(s, 6H), 2.32(s, 2H), 1.14(s, 9H).

Example 6

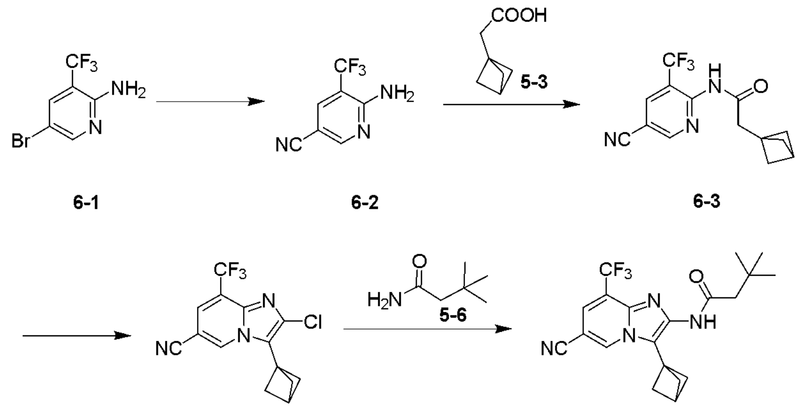


6-1 6-2 6-3

6-4 6

Step 1: Tetrahydrofuran (25 mL), water (100 mL), compound 6-1 (5 g, 20.75 mmol), and zinc cyanide (4.87 g, 41.49 mmol) were added to a 250 mL single-necked flask, and stirring was started. The reaction system was purged with nitrogen, and then (2-di-tert-butylphosphino-2',4',6'-triisopropyl-1,1'-biphenyl)(2'-amino-1,1'-biphenyl-2-yl)palladium(II) methanesulfonate (1.65 g, 2.07 mmol) was added. The mixture was heated to 45 °C, and reacted for 15 hours. Water (50 mL) was added, and the resulting mixture was extracted with added ethyl acetate (80 mL × 2). The resulting organic phase was washed with saturated brine (100 mL), then dried over added anhydrous sodium sulfate, and filtered. The filtrate was concentrated under reduced pressure. The residue was separated and purified by silica gel column chromatography (V/V petroleum ether: ethyl acetate = 1:0 to 2:3) to give a crude product. Then, methyl tert-butyl ether (10 mL) was added. The mixture was stirred for 10 minutes, and filtered. The filter cake was dried to give compound 6-2. LCMS(ESI): 188.2[M+H]$^+$. $^1$H NMR(400 MHz, $CDCl_3$)δppm 8.50(d, J= 1.6Hz, 1H), 7.95(s, 1H), 5.53(s, 2H).

Step 2: Dichloromethane (10 mL), 5-3 (394.39 mg, 3.13 mmol), and N,N-dimethylformamide (129.52 μL, 1.68 mmol) were added to a 100 mL three-necked flask, and stirring was started. The mixture was cooled to 0 to 5 °C, and then oxalyl chloride (357.88 μL, 4.09 mmol) was added dropwise. The mixture was warmed to room temperature (20 °C), and reacted for 1 hour. Then, compound 6-2 (450 mg, 2.40 mmol) was added, and 4-dimethylaminopyridine (293.80 mg, 2.40 mmol) and a solution of triethylamine (2.01 mL, 14.43 mmol) in dichloromethane (3 mL) were immediately added dropwise. The mixture was reacted at room temperature (15 °C) for 18 hours. Saturated ammonium chloride solution (30 mL) and dichloromethane (15 mL) were added to the reaction system, and then the layers were separated. The resulting organic phase was washed with saturated ammonium chloride solution (30 mL), dried over added anhydrous sodium sulfate, and filtered. The filtrate was concentrated under reduced pressure to give a crude product. The crude product was separated and purified by silica gel column chromatography (V/V petroleum ether: ethyl acetate = 1:0 to 3:2) to give compound 6-3. LCMS(ESI): 296.1 [M+H]$^+$.

Step 3: Chlorobenzene (36 mL) and compound 6-3 (360 mg, 1.22 mmol) were added to a 100 mL single-necked flask, and stirring was started. Then, thionyl chloride (442.78 μL, 6.10 mmol) and pyridine (344.44 μL, 4.27 mmol) were successively added. The mixture was heated to 90 °C and reacted for 15 hours. The reaction solution was concentrated under reduced pressure. The crude product was separated and purified by silica gel column chromatography (V/V

petroleum ether: ethyl acetate = 1:0 to 17:3) to give compound 6-4. LCMS(ESI): 312.1 [M+H]$^+$. $^1$H NMR(400 MHz, $CDCl_3$)δ ppm 8.67(s, 1H), 7.66(s, 1H), 2.79(s, 1H), 2.51(s, 6H).

Step 4: Compound 5-6 (480.37 mg, 4.17 mmol) and compound 6-4 (130 mg, 417.08 μmol) were added to dioxane (8 mL), and stirring was started. The reaction system was purged with nitrogen, and then cesium carbonate (407.68 mg, 1.25 mmol), (2-dicyclohexylphosphino-3,6-dimethoxy-2',4',6'-triisopropyl-1,1'-biphenyl)(2'-amino-1,1'-biphenyl-2-yl) palladium(II) methanesulfonate (75.62 mg, 83.42 μmol), and potassium iodide (103.85 mg, 625.62 μmol) were added. The mixture was heated to 120 °C, and reacted for 1.5 hours. The reaction solution was cooled to room temperature, and 20 mL of saturated ammonium chloride solution was added to the reaction system. Then, the mixture was extracted with added ethyl acetate (20 mL × 2). The resulting organic phase was washed with saturated brine (15 mL), then dried over added anhydrous sodium sulfate, and filtered. The filtrate was concentrated under reduced pressure. The crude product was separated and purified by silica gel column chromatography (V/V petroleum ether: ethyl acetate = 1:0 to 1:1), and then separated by preparative high-performance liquid chromatography (chromatographic column: Waters Xbridge BEH C18 100mm*30mm*10 μm; mobile phase: A (water, containing 10 mM ammonium bicarbonate) and B (acetonitrile); gradient: B%: 40% to 70%) to give compound 6. LCMS(ESI): 391.1 [M+H]$^+$. $^1$H NMR(400 MHz, $CDCl_3$)δ ppm 8.73(s, 1H), 7.80(brs, 1H),7.62(s, 1H), 2.74(s, 1H), 2.40(s, 6H), 2.35(s, 2H), 1.13(s, 9H).

Example 7

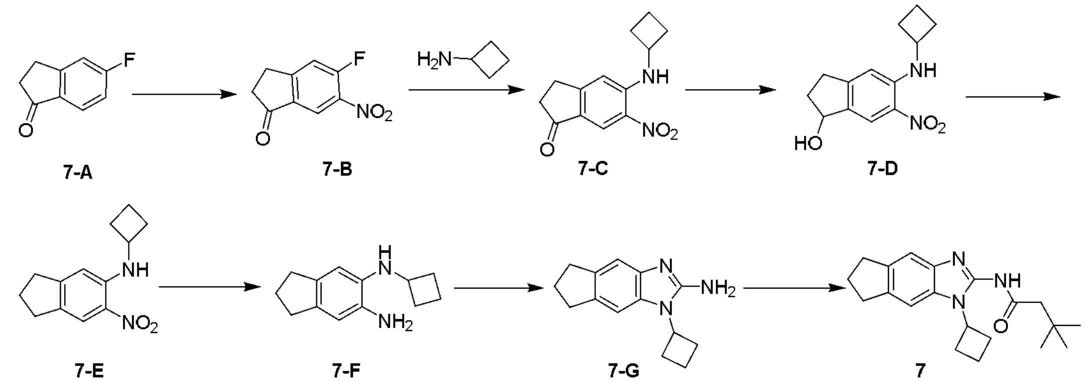

7-A 7-B 7-C 7-D

7-E 7-F 7-G 7

Step 1: Compound 7-A (10 g, 66.60 mmol) was slowly added in batches to fuming nitric acid (100 mL), and the mixture was stirred at 0 °C for 8 hours. The reaction solution was slowly poured into ice water (500 mL), and a solid was precipitated out. The mixture was filtered. The filter cake was collected, and dried under reduced pressure to give compound 7-B. $^1$H NMR($CDCl_3$, 400MHz): δ ppm 8.45(d, J =7.0 Hz, 1H), 7.42(d, J = 10.0 Hz, 1H), 3.32-3.20(m, 2H), 2.97-2.79(m, 2H).

Step 2: Compound 7-B (1 g, 5.12 mmol) was added to tetrahydrofuran (15 mL). Then, cyclobutylamine (400.89 mg, 5.64 mmol) and N,N-diisopropylethylamine (1.99 g, 15.37 mmol) were added. The mixture was stirred at 25 °C for 16 hours. The mixture was diluted with added water (20 mL), and the resulting mixture was extracted with ethyl acetate (20 mL × 2). The combined organic layers were washed with saturated brine (20 mL), then dried over sodium sulfate, and filtered. The filtrate was concentrated to give compound 7-C. MS ESI: 246.9 [M+H]$^+$.

Step 3: Compound 7-C (0.9 g, 3.65 mmol) was added to tetrahydrofuran (15 mL), and then sodium borohydride (207.40 mg, 5.48 mmol) was added. The mixture was stirred at 0 °C for 1 hour. The mixture was diluted with added saturated aqueous solution of ammonium chloride (20 mL). The aqueous layer was extracted with ethyl acetate (20 mL × 2). The combined organic layers were washed with saturated brine (20 mL), then dried over anhydrous sodium sulfate, and filtered. The filtrate was concentrated to give compound 7-D. MS ESI: 249.0 [M+H]$^+$.

Step 4: Compound 7-D (0.85 g, 3.42 mmol) was added to trifluoroacetic acid (10 mL), and then triethylsilane (796.19 mg, 6.85 mmol) was added. The mixture was stirred at 25 °C for 16 hours. The aqueous layer was diluted with added aqueous solution of sodium bicarbonate (20 mL), and the resulting mixture was extracted with ethyl acetate (20 mL × 2). The combined organic layers were washed with saturated brine (20 mL), then dried over anhydrous sodium sulfate, and filtered. The filtrate was concentrated to give compound 7-E. MS ESI: 233.1 [M+H]$^+$.

Step 5: Compound 7-E (0.7 g, 3.01 mmol) was added to ethanol (50 mL) and water (10 mL), and then reduced iron powder (841.56 mg, 15.07 mmol) and ammonium chloride (806 mg, 15.07 mmol) were added. The mixture was stirred at 90 °C for 16 hours. The aqueous layer was diluted with added water (50 mL), and the resulting mixture was extracted with ethyl acetate (50 mL × 2). The combined organic layers were washed with saturated brine (50 mL), then dried over anhydrous sodium sulfate, and filtered. The filtrate was concentrated to give compound 7-F. MS ESI: 202.9 [M+H]$^+$.

Step 6: Compound 7-F (0.6 g, 2.97 mmol) was added to ethanol (25 mL), and then cyanogen bromide (628.31 mg, 5.93 mmol) was added. The mixture was stirred at 25 °C for 16 hours. The mixture was diluted with added aqueous solution of sodium bicarbonate (50 mL), and the aqueous layer was extracted with ethyl acetate (50 mL × 2). The combined organic layers were washed with saturated brine (50 mL), then dried over anhydrous sodium sulfate, and filtered. The filtrate was concentrated to give compound 7-G. MS ESI: 227.9 $[M+H]^+$.

Step 7: Compound 7-G (0.15 g, 0.660 mmol) was added to acetone (20 mL), and then N,N-diisopropylethylamine (252.60 mg, 1.95 mmol) and tert-butylacetyl chloride (131.54 mg, 0.977 mmol) were added. The mixture was stirred at 25 °C for 16 hours. The reaction solution was concentrated under reduced pressure, and acetone (10 mL) and NaOH aqueous solution (2 N, 20 mL) were added. The mixture was stirred at 25 °C for 1 hour, and extracted with ethyl acetate (50 mL × 2). The combined organic layers were washed with saturated brine (50 mL), then dried over anhydrous sodium sulfate, and filtered. The filtrate was concentrated to give a crude product. The crude product was separated by column chromatography (petroleum ether/ethyl acetate, 1/0 to 2/1, V/V) to give compound 7. MS ESI: 326.1 $[M+H]^+$. $^1H$ NMR(DMSO-$d_6$, 400MHz): δppm 10.26(br s, 1H), 7.55(br s, 1H), 7.35(s, 1H), 4.73(br s, 1H), 3.00-2.85(m, 4H), 2.84-2.54(m, 2H), 2.33(br s, 2H), 2.26(s, 2H), 2.09-2.02(m, 2H), 1.94(q, J=10.2 Hz, 1H), 1.86-1.74(m, 1H), 1.05(s, 9H).

Example 8

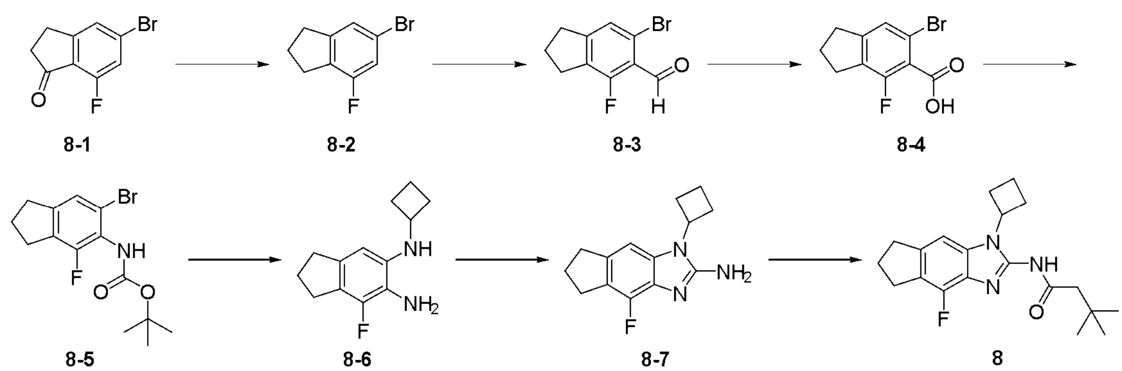

8-1 8-2 8-3 8-4

8-5 8-6 8-7 8

Step 1: Compound 8-1 (6 g, 26.20 mmol) was added to trifluoroacetic acid (30 mL), and triethylsilane (9.14 g, 78.59 mmol) was added. The reaction solution was kept at 25 °C and reacted for 24 hours under nitrogen. Saturated sodium bicarbonate solution was added dropwise to the reaction solution, and the pH was adjusted to 7 to 8. The mixture was extracted with ethyl acetate (150 mL × 3), dried over anhydrous sodium sulfate, filtered, and rotary-evaporated to dryness under reduced pressure to give a crude product. The crude product was separated and purified by column chromatography (petroleum ether/ethyl acetate, 100/0, V/V) to give compound 8-2.

Step 2: A mixed solution of lithium diisopropylamide in tetrahydrofuran and n-heptane (2 M, 22.32 mL) was added to tetrahydrofuran (30 mL). The mixture was cooled to -78 °C, and then compound 8-2 (6.4 g, 29.76 mmol) was added. The mixture was stirred and reacted for 30 minutes, and then N,N-dimethylformamide (89.28 mmol, 6.87 mL) was added. The reaction solution was warmed to 25 °C and reacted for 30 minutes under nitrogen. A saturated ammonium chloride solution (500 mL) was added dropwise to the reaction solution. The resulting mixture was extracted with ethyl acetate (100 mL × 3), dried over anhydrous sodium sulfate, filtered, and rotary-evaporated to dryness under reduced pressure to give a crude product. The crude product was separated and purified by column chromatography (petroleum ether/ethyl acetate, 100/0 to 5/1, V/V) to give compound 8-3.

Step 3: Compound 8-3 (5 g, 20.57 mmol) was added to a mixed solution of water (50 mL) and tert-butanol (50 mL). The mixture was cooled to 0 °C, and then sodium chlorite (7.44 g, 82.26 mmol), sodium dihydrogen phosphate (9.87 g, 82.28 mmol), and 2-methyl-2-butene (205.70 mmol, 21.79 mL) were added. The reaction solution was kept at 0 °C and reacted for 2 hours under nitrogen. The reaction solution was extracted with ethyl acetate (100 mL × 3), dried over anhydrous sodium sulfate, filtered, and rotary-evaporated to dryness under reduced pressure to give compound 8-4. LCMS(ESI):259.0$[M+H]^+$.

Step 4: Compound 8-4 (1.7 g, 6.56 mmol) was added to tert-butanol (17 mL). The mixture was cooled to 0 °C, and then N,N-diisopropylethylamine (1.37 mL) and diphenylphosphoryl azide (2.17 g, 7.87 mmol) were added. The reaction solution was heated to 80 °C and reacted for 2 hours under nitrogen. The reaction solution was rotary-evaporated to dryness under reduced pressure to give a crude product. The crude product was separated and purified by column chromatography (petroleum ether/ethyl acetate, 100/0 to 20/1, V/V) to give compound 8-5. LCMS(ESI):232.0$[M-Boc+H]^+$. 1H NMR(400 MHz, $CDCl_3$)δ ppm 7.24(s, 1 H), 5.95(s, 1 H), 2.89-2.94(m, 4 H), 2.09-2.16(m, 2 H), 1.51(s, 9 H).

Step 5: Compound 8-5 (0.4 g, 1.21 mmol) was added to dioxane (5 mL). Cyclobutylamine (2.42 mmol, 207.61 μL), (2-dicyclohexylphosphino-2,6-diisopropoxy-1,1'-biphenyl)(2'-amino-1,1'-biphenyl-2-yl)palladium(II) methanesulfonate (219.63 mg, 242.28 μmol), 2-dicyclohexylphosphino-2',6'-diisopropoxy-1,1'-biphenyl (260.10 mg, 484.57 μmol), and potassium tert-butoxide (271.87 mg, 2.42 mmol) were added. The reaction solution was heated to 100 °C and reacted for 2 hours under nitrogen. The reaction solution was rotary-evaporated to dryness under reduced pressure to give a crude product. The crude product was separated and purified by column chromatography (petroleum ether/ethyl acetate, 100/0 to 20/1, V/V) to give compound 8-6. LCMS(ESI):221.1[M+H]$^+$.

Step 6: Compound 8-6 (0.266 g, 1.21 mmol) was added to ethanol (5 mL), and cyanogen bromide (2.42 mmol, 177.27 μL) was added. The reaction solution was kept at 25 °C, and reacted for 1 hour under nitrogen. The reaction solution was rotary-evaporated to dryness under reduced pressure to give a crude product. The crude product was separated and purified by column chromatography (dichloromethane/methanol, 100/0 to 20/1, V/V) to give compound 8-7. LCMS(ESI):246.0[M+H]$^+$.

Step 7: Compound 8-7 (55 mg, 179.38 μmol) was added to acetone (5 mL), and N,N-diisopropylethylamine (34.37 μL) was added. The reaction solution was cooled to 0 °C under nitrogen, and then tert-butylacetyl chloride (27.41 μL) was added dropwise. The reaction solution was kept at 25 °C, and reacted for 1 hour under nitrogen. The reaction solution was filtered under reduced pressure. The filtrate was collected, and rotary-evaporated to dryness under reduced pressure. Methanol (5 mL) and 10% sodium hydroxide aqueous solution (2 mL) were added, and the mixture was stirred and reacted for 1 hour. Then, the mixture was rotary-evaporated to dryness under reduced pressure. The resulting product was extracted with ethyl acetate (10 mL × 3), washed with saturated aqueous solution of sodium chloride, dried over anhydrous sodium sulfate, and rotary-evaporated to dryness under reduced pressure to give a crude product. The crude product was separated and purified by column chromatography (petroleum ether/ethyl acetate, 100/0 to 10/1, V/V) to give compound 8. LCMS(ESI):344.3[M+H]$^+$. $^1$H NMR(400 MHz, DMSO-$d_6$)δ ppm 10.36(s, 1H), 7.44(s, 1H), 4.70-4.79 ppm(m, 1H), 3.03(t, J = 7.2 Hz, 2H), 2.96(t, J = 7.2 Hz, 2H), 2.72-2.85 ppm(m, 2H), 2.38-2.40 ppm(m, 2H), 2.29(s, 2H), 2.11-2.13 ppm(m, 2H), 1.92-1.97 ppm(m, 1H), 1.81-1.86 ppm(m, 1H), 1.066(s, 9H).

Example 9

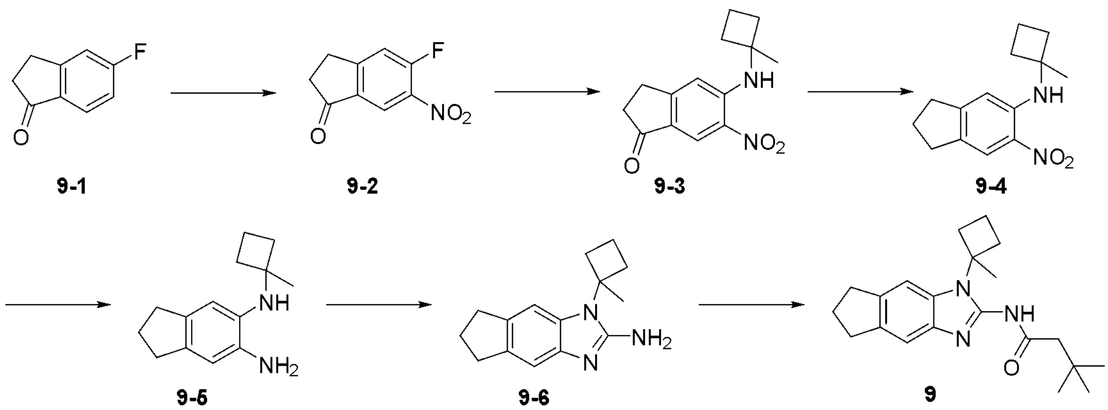

9-1 9-2 9-3 9-4 9-5 9-6 9

Step 1: Compound 9-1 (10 g, 66.60 mmol) was added in batches to fuming nitric acid (100 mL), and the mixture was stirred and reacted at 0 °C for 8 hours. The reaction solution was slowly poured into ice water (500 mL), and the mixture was filtered. The filter cake was collected, and dried under reduced pressure to give compound 9-2. $^1$H NMR(400 MHz, $CDCl_3$)δ ppm 8.44(d, J= 7.2 Hz, 1 H), 7.42(d, J= 10.4 Hz, 1 H), 3.27(t, J= 6.0 Hz, 2 H), 2.81-2.84(m, 2 H).

Step 2: Compound 9-2 (1 g, 5.12 mmol) was added to tetrahydrofuran (15 mL). Then, 1-methyl-cyclobutylamine hydrochloride (972.00 mg, 6.15 mmol) and N,N-diisopropylethylamine (2.68 mL) were added. The reaction solution was kept at 25 °C, stirred and reacted for 16 hours. The mixture was diluted with added water (20 mL), and the resulting mixture was extracted with ethyl acetate (20 mL × 3). The combined organic phases were washed with saturated brine (20 mL × 2), then dried over sodium sulfate, and filtered. The filtrate was concentrated to give compound 9-3. LCMS(ESI):261.1 [M+H]$^+$.

Step 3: Compound 9-3 (0.5 g, 1.92 mmol) was added to trifluoroacetic acid (5 mL), and triethylsilane (613.63 μL) was added. The reaction solution was kept at 25 °C and reacted for 24 hours under nitrogen. The reaction solution was then cooled to 0 °C, and saturated sodium bicarbonate solution was added dropwise to adjust the pH to 7 to 8. The resulting mixture was extracted with ethyl acetate (50 mL × 2), washed with saturated brine (20 mL × 2), dried over anhydrous sodium sulfate, filtered, and rotary-evaporated to dryness under reduced pressure to give compound 9-4.

Step 4: Compound 9-4 (0.8 g, 3.25 mmol) was added to ethanol (40 mL) and water (10 mL). Then, reduced iron powder (907.01 mg) and ammonium chloride (868.68 mg) were added. The reaction solution was heated to 90 °C and stirred for 16 hours. The mixture was diluted with added water (200 mL). The resulting mixture was extracted with ethyl acetate (20 mL × 2). The combined organic phases were washed with saturated brine (20 mL × 2), then dried over anhydrous sodium sulfate, and filtered. The filtrate was concentrated to give compound 9-5.

Step 5: Compound 9-5 (0.7 g, 3.24 mmol) was added to ethanol (10 mL), and then cyanogen bromide (685.51 mg, 6.47 mmol) was added. The reaction solution was kept at 25 °C, stirred and reacted for 12 hours. The mixture was diluted with added ethyl acetate (50 mL). Then, a 10% aqueous solution of sodium bicarbonate (50 mL) was added to quench the reaction. The aqueous phase was extracted with ethyl acetate (50 mL × 2). The combined organic phases were washed with saturated brine (50 mL), then dried over anhydrous sodium sulfate, and filtered. The filtrate was concentrated to give compound 9-6.

Step 6: tert-Butylacetyl chloride (209.16 mg, 1.55 mmol, 215.85 μL) was added to acetone (10 mL), and then N,N-diisopropylethylamine (541.30 μL) and compound 9-6 (0.25 g, 1.04 mmol) were added. The mixture was stirred at 25 °C for 16 hours. Water (20 mL) was added to the reaction solution, and the resulting mixture was extracted with ethyl acetate (20 mL × 3). The organic phases were combined, washed with saturated brine (20 mL × 2), dried over anhydrous sodium sulfate, and filtered. The filtrate was concentrated to give a crude product. The crude product was added to acetonitrile (5 mL), and an aqueous solution of sodium hydroxide (1 M, 4.57 mL) was added. The reaction solution was kept at 25 °C, stirred and reacted for 2 hours. Water (20 mL) was added, and the resulting mixture was extracted with added ethyl acetate (20 mL × 3). The combined organic phases were washed with saturated brine (20 mL × 2), then dried over anhydrous sodium sulfate, and filtered. The filtrate was concentrated to give a crude product. The crude product was separated by pre-HPLC (column type: C18 100 × 40 mm; mobile phase: [$H_2O$(TFA)-ACN]; gradient: 23% to 53% ACN, 8 min) to give compound 9. LCMS(ESI):340.1[M+H]$^+$. $^1$H NMR(400 MHz, $CDCl_3$)δ ppm 6.93-7.13(m, 2H), 2.95(t, J = 6.8 Hz, 4H), 2.76-2.90(m, 2H), 2.52(t, J = 8.0 Hz, 2H), 2.31(s, 2H), 2.06-2.20(m, 2H), 1.86-2.06(m, 3H), 1.67(s, 3H), 1.07 ppm(s, 9H).

Example 10

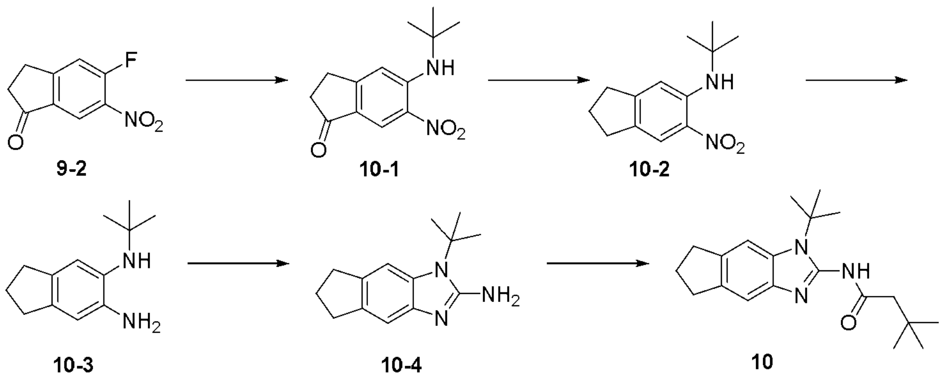

9-2, 10-1, 10-2, 10-3, 10-4, 10

Step 1: Compound 9-2 (1 g, 5.12 mmol) was added to tetrahydrofuran (20 mL). Then, tert-butylamine (592.32 μL) and N,N-diisopropylethylamine (2.68 mL) were added. The reaction solution was kept at 25 °C, stirred and reacted for 16 hours. The mixture was diluted with added water (20 mL), and the resulting mixture was extracted with ethyl acetate (20 mL × 3). The combined organic layers were washed with saturated brine (20 mL × 2), then dried over sodium sulfate, and filtered. The filtrate was concentrated to give compound 10-1.

Step 2: Compound 10-1 (0.5 g, 2.01 mmol) was added to trifluoroacetic acid (5 mL), and triethylsilane (965.00 μL) was added. The reaction solution was kept at 25 °C and reacted for 24 hours under nitrogen. The reaction solution was cooled to 0 °C. Saturated sodium bicarbonate solution was added dropwise, and the pH was adjusted to 7 to 8. The resulting mixture was extracted with ethyl acetate (50 mL × 2), washed with saturated brine (20 mL × 2), dried over anhydrous sodium sulfate, filtered, and rotary-evaporated to dryness under reduced pressure to give compound 10-2. LCMS(ESI):179.1[M+H]$^+$. $^1$H NMR(400 MHz, $CDCl_3$)δ ppm 7.95(s, 1 H), 6.66(s, 1 H), 2.84(q, J= 7.2 Hz, 4 H), 2.06-2.10(m, 2 H), 0.98(s, 1H).

Step 3: Compound 10-2 (0.1 g, 426.82 μmol) was added to ethanol (30 mL) and water (8 mL). Then, iron powder (119.19 mg, 2.13 mmol) and ammonium chloride (114.15 mg, 2.13 mmol) were added. The reaction solution was heated to 90 °C and stirred for 16 hours. The mixture was diluted with added water (200 mL). The aqueous phase was extracted with ethyl acetate (20 mL × 3). The combined organic phases were washed with saturated brine (20 mL × 2), dried over anhydrous sodium sulfate, and filtered. The filtrate was concentrated to give compound 10-3.

Step 4: Compound 10-3 (0.3 g, 1.47 mmol) was added to ethanol (5 mL), and then cyanogen bromide (311.06 mg, 2.94 mmol) was added. The reaction solution was kept at 25 °C, stirred and reacted for 12 hours. The reaction solution was rotary-evaporated to dryness under reduced pressure to give a crude product. The crude product was separated by pre-HPLC (Welch Xtimate C18 150*25mm*5μm; mobile phase: [$H_2O(NH_3.H_2O+NH_4HCO_3)$-ACN]; gradient: 33% to 63% ACN, 8 min) to give compound 10-4.

Step 5: tert-Butylacetyl chloride (16.66 μL) was added to acetone (10 mL). Then, N,N-diisopropylethylamine (20.89 μL) and compound 10-4 (25 mg, 109.02 μmol) were added. The mixture was stirred at 25 °C for 16 hours. Water (20 mL) was added to the reaction solution, and the resulting mixture was extracted with ethyl acetate (20 mL x 3). The combined organic phases were washed with saturated brine (20 mL x 2), then dried over anhydrous sodium sulfate, and filtered. The filtrate was concentrated to give a crude product. The crude product was added to acetonitrile (5 mL), and an aqueous solution of sodium hydroxide (1 M, 4.57 mL) was added. The reaction solution was kept at 25 °C, stirred and reacted for 2 hours. Water (20 mL) was added, and the resulting mixture was extracted with added ethyl acetate (20 mL x 3). The combined organic phases were washed with saturated brine (20 mL x 2), then dried over anhydrous sodium sulfate, and filtered. The filtrate was concentrated to give a crude product. The crude product was separated by pre-HPLC (column type: Welch Xtimate C18 150*25mm*5μm; mobile phase: [$H_2O(NH_3H_2O+NH_4HCO_3)$-ACN]; gradient: 58% to 88% ACN, 8 min)to give compound 10. LCMS(ESI):328.2[M+H]$^+$. $^1$H NMR(400 MHz, $CDCl_3$)δ ppm 7.53(s, 1H), 7.23-7.05(m, 1H), 2.92-3.01(q, J=7.2Hz, 4H), 2.39(s, 2H), 2.13(t, J = 7.6 Hz, 2H), 1.93(s, 9H), 1.07 ppm(s, 9H).

**Biological Assay**

Assay Example 1: hKCNQ2/3 thallium flux assay

Purpose of the assay: To determine the in vitro concentration-response relationship of the compounds in a CHO cell line stably expressing hKCNQ2/3 using FLIPR thallium flux assay.

Cells and reagents of the assay:

(1) Cell line: CHO cells stably transfected with hKCNQ2/3.

(2) Reagents of cell culture medium: F12 culture medium, fetal bovine serum FBS (10%), penicillin-streptomycin (Invitrogen, Catalog number 15140-122), and G418 (Invitrogen, Catalog number 10131027) were included.

(3) Cell culture:

The cell line was passaged three times a week at a split ratio of 1:2 to 1:3. When the cells grew to a confluence degree of greater than 80% in a T-75 cell flask, they were digested with 0.25% trypsin-EDTA solution for about 1 minute, and then the cells were transferred from the cell flask for passage. According to the split ratio, the cells were transferred to another T-75 cell flask containing complete cell growth culture medium. Note: In order to maintain the logarithmic growth of cells, the cell growth should be maintained in a subconfluent monolayer state. The cells were passaged every 2 to 3 days according to the cell doubling time.

Assay procedure:

(1) Day 1, cell preparation: The cells were digested according to the above method, and the cell density and viability were determined using Cell Countess. The volume of the single-cell suspension was adjusted with complete cell growth culture medium. Then, the hKCNQ2/3_CHO cells were seeded at a density of approximately 20,000 cells/well (30 μL/well) into a PDL-precoated 384-well plate. Then, the plate was incubated at 37 °C with 5% $CO_2$ humidified air in an incubator overnight.

(2) Day 2, compound preparation: A test compound was diluted to a storage concentration with dimethyl sulfoxide (DMSO), and stored in a freezer at -20 °C. A compound addition program was set up on the ECHO liquid workstation, and the compound was added to the plate using the ECHO liquid workstation. A positive control was prepared using Retigabine (RTG) with a maximum concentration of 100 μM, which was subjected to a 3-fold dilution, resulting in a total of 9 doses. A buffer containing 5 mM $K^+$ and 1 mM $Tl^+$ was added to the plate.

Note: 1) $K_2SO_4$ and $Tl_2SO_4$ were prepared in a 1× chloride-free buffer. When different voltage-gated potassium channels were involved, the concentrations of $Tl^+$ and $K^+$ were needed to be adjusted. The concentration of $Tl^+$ was

approximately 0.5 mM to 5 mM, and the concentration of $K^+$ was approximately 5 mM to 30 mM. 2) Each 1 mM solution of $K_2SO_4$ and $Tl_2SO_4$ contained 2 mM $K^+$ and $Tl^+$. The final concentration should be calculated based on the ion concentrations. 3) TlCl was easily precipitated and a chloride-free buffer was required.

(3) Day 2, assay testing: When the cells reached the confluence degree, the cell analysis plate was taken out from the incubator. The assay buffer was prepared and the $Tl^+$ dye (Molecular Devices # R8222) was diluted. The culture medium was discarded using the liquid workstation Bravo and 25 μL of $Tl^+$ dye was added to each well. The plate was incubated for 1 hour, and then the cell analysis plate, composite plate, and FLIPR tip were placed in FLIPR$^{TETRA}$ (Molecular Devices, USA) for FLIPR assay. The tip was set up, and then the baseline was read for 60 seconds. Then, 12.5 μL from the composite plate (3×) was added to the cell analysis plate, and the data were recorded for at least 5 minutes at the same time.

(4) Data processing:

The maximum signal was generated with FLIPR software. Excel (2013) and Prism 6.01 were used for data analysis. The data quality control was performed with S/B > 2.00 and Z-factor > 0.50. $IC_{50}$ or $EC_{50}$ was defined as the midline between the lowest and the highest plateaus, and it was calculated using a four-parameter logistic model with Prism 6.01.

The assay results are shown in Table 1.

Table 1. Assay results of the agonistic effect of the compounds of the present disclosure on hKCNQ2/3

| Compound number | $EC_{50}$(μM) |
|---|---|
| Compound **3** | 0.48 |
| Compound **5** | 0.20 |
| Compound **6** | 0.71 |
| Compound **7** | 0.42 |
| Compound **8** | 0.42 |
| Compound **9** | 0.16 |
| Compound **10** | 0.12 |

Conclusion: The compounds of the present disclosure have a significant agonistic effect on KCNQ2/3.

Assay Example 2: Evaluation of the behavioral pharmacodynamics of the test compounds in a maximal electroshock-induced epilepsy model

Purpose of the assay:

This study used behavioral tests in male CD-1 mice to investigate the effects of test compounds on acute epileptic seizures behavior induced by maximal electroshock.

Assay materials:

Male CD-1 mice (25 to 35g) were used as the experimental animals and fed with standard feed (Beijing Vital River Laboratory Animal Technology Co., Ltd.).

Assay procedure:

After arriving at the animal facility, the experimental animals needed to acclimatize for at least one week. The mice were observed daily for vital signs, and randomly divided into groups according to their body weights one day before the drug administration. One hour before the electrical stimulation, different dosages of the test compound were orally administered to each group. On the day of the assay, the male CD-1 mice in each group were anesthetized locally at the cornea with 2% lidocaine. Then, silver bipolar electrodes were used to electrically stimulate the cornea of the mice with 18 mA, 60 Hz, a pulse width of 0.6 ms, and a stimulation time of 1 s to induce seizures. Subsequently, the latency of seizures and the duration of limb rigidity in mice after the electrical stimulation were observed, and the protection rate from seizures for each group was statistically analyzed.

The assay results are shown in the table below:

Table 2. Assay results of the pharmacodynamics of the compounds of the present disclosure

| Compound number | Dosage (mg/kg) | Protection rate from seizures |
|---|---|---|
| Compound **1** | 5 | 66.7% |
| Compound **3** | 5 | 66.7% |
| Compound **7** | 5 | 66.67% |
| Compound **8** | 5 | 50% |
| Compound **9** | 5 | 50% |
| Compound **10** | 5 | 33.33% |

Assay conclusion: The compounds of the present disclosure exhibit good antiseizure activity in the maximal electroshock-induced epilepsy model.

Assay Example 3: Pharmacokinetic evaluation of compounds

Purpose of the assay:

The purpose of this assay was to evaluate the pharmacokinetic behaviors after a single intravenous bolus and gavage administration of a compound, to investigate the bioavailability after the gavage administration, and to provide animal experimental data for clinical research.

Assay materials:

CD-1 mice (male, 7 to 9 weeks old)

Assay procedure:

The pharmacokinetic characteristics of the compounds after intravenous and oral administration were assayed in rodents using a standard protocol. In the assay, a candidate compound was prepared into a clear solution and administered to mice via single intravenous and oral administration. The solvent for both intravenous and oral administration was a mixed solvent prepared with 5% DMSO, 60% PEG400, and 35% water. Four female CD-1 mice were used in this project. Two mice were administered intravenously, and plasma samples were collected at 0.083, 0.25, 0.5, 1, 2, 4, 8 and 24 h after the administration. The other two mice were administered orally via gavage, and plasma samples were collected at 0.25, 0.5, 1, 2, 4, 8 and 24 h after the administration. The plasma samples were stirred at 4 °C and 3,200 × g for 10 min, and the supernatants were separated to give plasma samples. 20 times the volume of methanol solution containing internal standard was added to precipitate the proteins. After centrifugation at 12,000 × g for 15 min at 4 °C, 50 μL of the supernatant was taken and transferred to a 96-well plate, and centrifuged a second time, and the resulting supernatant was injected for analysis. The plasma concentration was quantitatively analyzed by LC-MS/MS method, and the pharmacokinetic parameters such as peak concentration ($C_{max}$), clearance (CL), half-life ($T_{1/2}$), tissue distribution (Vdss), area under the plasma concentration-time curve ($AUC_{0\text{-}last}$), and bioavailability (F) were calculated.

The assay results are shown in Table 3:

Table 3. Pharmacokinetic assay results of the compounds of the present disclosure

| **Compound number** | Dosage of administration mg/kg | Peak concentration Cmax (nM) | Clearance CL (mL/min/kg) | Tissue distribution Vdss (L/kg) | Half-life T1/2 (IV, h) | Area under the plasma concentration-time curve AUC0-last PO (nM.hr) | Bioavailability F (%) |
|---|---|---|---|---|---|---|---|
| **Compound 1** | IV:1 PO:5 | 355 | 67.8 | 4.89 | 0.88 | 1405 | 51.1 |

(continued)

| **Compound number** | Dosage of administration mg/kg | Peak concentration Cmax (nM) | Clearance CL (mL/min/kg) | Tissue distribution Vdss (L/kg) | Half-life T1/2 (IV, h) | Area under the plasma concentration-time curve AUC0-last PO (nM.hr) | Bioavailability F (%) |
|---|---|---|---|---|---|---|---|
| **Compound 3** | IV:0.5 PO:2 | 1154 | 25.6 | 0.94 | 0.5 | 2346 | 57.3 |

Assay conclusion: The compounds of the present disclosure have good pharmacokinetic properties, including good oral bioavailability, oral exposure, half-life, clearance, etc.

Assay Example 4: Evaluation of plasma protein binding rate

Purpose of the assay:

The protein binding rates of the compounds in the plasma of CD-1 mice, Sprague-Dawley rats, beagle dogs, cynomolgus monkeys, and human were determined using equilibrium dialysis method.

Assay materials:

Commercially available plasma, dual-layer dialysis membrane, and equilibrium dialysis device.

Assay procedure:

The dual-layer dialysis membrane was soaked in ultrapure water for about 1 hour, removed and then divided into two parts. It was then soaked in an ethanol:water (20:80, v:v) solution for 20 minutes or placed at 2 to 8 °C, and it was valid for 1 month. Before starting the assay, the dialysis membrane was rinsed twice with ultrapure water and then soaked in ultrapure water for another 20 minutes for later use.

On the day of assay, the frozen plasma of CD-1 mice, Sprague-Dawley rats, beagle dogs, cynomolgus monkeys, and human was thawed in running cold tap water. After the plasma was completely thawed, the plasma was centrifuged at 3220×g for 5 minutes, and the suspended substances and precipitates in it were removed. The pH of the plasma was determined. The plasma with a pH in the range of 7.4 ± 0.1 was directly used, or the plasma with a pH in the range of 7.4 ± 0.1 after an adjustment with 1% phosphoric acid or 1M sodium hydroxide was used.

Preparation process of plasma samples: 597 μL of blank plasma from each of the above five species was taken, respectively. 3 μL of a compound or warfarin working solution was added, and the mixture was mixed thoroughly to give a plasma sample with a compound concentration of 2 μM and a warfarin concentration of 2 μM (n=1). The concentration of the organic phase DMSO was 0.5%. The sample was thoroughly mixed before proceeding to the next step.

Preparation process of T0 samples: 50 μL of a plasma sample containing the compound and warfarin was transferred to a sample receiving plate (n=3), and 50 μL of blank PBS was immediately added. Then, 500 μL of stop solution was added to the T0 sample of the compound and warfarin, and the mixture was stored at 2 to 8 °C, waiting to be processed later together with other dialysis-processed samples.

Dialysis process of plasma samples: 100 μL of a compound (2 μM) and warfarin (2 μM) plasma sample was added to the donor of each dialysis well (n=3), and 100 μL of blank PBS was added to the corresponding receiver of the dialysis well. The dialysis plate was placed in a 5% $CO_2$ incubator, and incubated at 37 °C with shaking at approximately 100 rpm for 4 hours.

After the dialysis was completed, 50 μL of dialysis-processed PBS sample and dialysis-processed plasma sample (n=3) were transferred to a new 96-well plate (sample receiving plate). The corresponding volume of the corresponding blank plasma or PBS was added to the sample to make the final volume of each sample well 100 μL, with a plasma to PBS volume ratio of 1:1. All the samples were analyzed by LC-MS/MS after protein precipitation.

The formulas for calculating the unbound fraction (%Unbound), bound fraction (%Bound) and recovery fraction (%Recovery) of a compound are as follows:

$\%Unbound = 100 \times F / T$

$\%Bound = 100 - \%Unbound$

$\%Recovery = 100 \times (F + T) / T_0$

wherein F is the concentration of the compound at the receiver of dialysis; T is the concentration of the compound at the supply end of dialysis; and T0 is the concentration of the compound in the plasma at time zero.

The assay results are shown in Table 4:

Table 4. Assay results of the plasma protein binding rate of the compounds of the present disclosure

| Compound number | Unbound PPB%(h,r,m,d,c) |
|---|---|
| Compound **1** | 5.1/ 7.1/ 4.7/ 4.6/ 7.2 |
| Compound **3** | 10.9/ 9.7/ 8.7/ 20.3/ 13.4 |
| Compound **5** | 23.1/16.6 /14.1/27.8/15.9 |
| Compound **7** | 0.4 / 0.3 / 0.3 / 0.6 / 1.2 |
| Compound **9** | 0.3/ 0.2/ 0.2/ 0.4/ 0.4 |

Assay conclusion: The compounds of the present disclosure have a suitable plasma protein binding rate.

Assay Example 5: Permeability evaluation

Purpose of the assay:

The purpose of this study was to evaluate and study the P-gp substrate of the compounds in MDR1 MDCK I cells.

Assay materials:

MDR1-MDCK I cell line.

Assay procedure:

Transport assay: The test sample, nadolol, and metoprolol were administered at a concentration of 2.00 μM, and digoxin was administered at a concentration of 10.0 μM. The administration was performed bidirectionally (A-B and B-A directions), with two replicates for each administration concentration. The dosing solution, receiving solution, and transport buffer were pre-incubated in a 37.0 °C water bath for 30 minutes. The cell layer was rinsed twice with transport buffer. The dosing solution and receiving solution were added to the corresponding wells of the cell plate, respectively (75.0 μL to each apical well and 250 μL to each basolateral well, respectively). After the addition of samples, the cell plate was incubated in an incubator at 37.0°C, 5.0% $CO_2$ and saturated humidity for 90 minutes. The initial dosing solution was used as the T0 sample. After 90 minutes of incubation, samples were collected from each well and labeled as A-B receiver, B-A receiver, A-B donor, and B-A donor, respectively. After the sample collection, all samples were mixed with an appropriate volume of transport buffer and stop solution. All samples were vortexed, and then centrifuged at 20.0 °C and 3220 × g for 10 minutes. An appropriate volume of the supernatant was transferred to a sample analysis plate. After sealing the plate, the samples were stored at 2.0 to 8.0 °C if not analyzed immediately. Analysis was performed using LC-MS/MS.

Cell membrane integrity assay: After the transport assay, the integrity of the MDR1-MDCK II cell layer was assayed by the Lucifer Yellow Rejection Assay. The remaining solution in the apical and basolateral wells was removed. 75.0 μL of transport buffer containing 100 μM Lucifer Yellow was added to the apical well, and 250 μL of transport buffer was added to the basolateral well, respectively. The cell plate was incubated in a cell culture incubator at 37 °C, 5% $CO_2$ and saturated humidity for 30 minutes. Then, 20.0 μL of sample was taken from the apical end and mixed with 60.0 μL of transport buffer, and 80.0 μL of sample was taken from the basolateral end. The relative fluorescence unit (RFU) was measured at 425/528 nm (excitation/emission) using a microplate reader.

In this study, the sample analysis of the compounds and control compounds of nadolol, metoprolol, and digoxin was performed using LC-MS/MS. The retention time of the analytes and internal standards, chromatogram acquisition, and chromatogram integration were processed using the software Analyst (Sciex, Framingham, MA, USA). The sample analysis did not include standard curves or quality control samples. Semi-quantitative determination was performed using the ratio of the peak area of analyte to the peak area of internal standard.

The apparent permeability coefficient ($P_{app}$, cm/s), efflux ratio (ER), and recovery rate (%Solution Recovery) were calculated using the following formulas.

$$P_{app}=\frac{V_R}{Area\times Time}\times\frac{[drug]_{receiver}}{[drug]_{initial,\ donor}}=\frac{V_R}{Area\times Time}\times\frac{C_R}{C_0}$$

$$Efflux\ Ratio=\frac{P_{app}(B\text{-}A)}{P_{app}(A\text{-}B)}$$

$$\%Solution\ Recovery=\frac{C_R\times V_R+C_D\times V_D}{C_0\times V_D}\times 100$$

VR is the volume of the receiver solution (0.0750 mL for side A, and 0.250 mL for side B); Area is the surface area of the cell monolayer (0.143 $cm^2$); Time is the incubation time (5400 s); C0 is the initial peak area ratio of the test compound or the control compound at the donor; VD is the volume of the donor (0.0750 mL for side A, and 0.250 mL for side B); CD and CR are the peak area ratios of the test compound or the control compound at the donor and the receiver, respectively. The transmittance of Lucifer Yellow (%Lucifer Yellow) was calculated using the following formula:

$$\%Lucifer\ Yellow=\frac{V_{Basolateral}\times RFU_{Basolateral}}{V_{Apical}\times RFU_{Apical}+V_{Basolateral}\times RFU_{Basolateral}}\times 100$$

$RFU_{Apical}$ and $RFU_{Basolateral}$ are the relative fluorescence units of Lucifer Yellow at the apical and basolateral ends, respectively. $V_{APical}$ and $V_{Basolateral}$ are the loading volumes at the apical and basolateral ends, respectively (0.0750 and 0.250 mL, respectively).

The assay results are shown in Table 5:

Table 5. Permeability assay results of the compounds of the present disclosure

| Compound number | HMDR1 Papp($10^{-6}$ cm/s)A to B, B to A, ER |
|---|---|
| Compound 1 | 3.64, 18.3, 5.04 |
| Compound 3 | 7.52, 31.2, 4.15 |
| Compound 5 | 12.4, 42.4, 3.41 |

Assay conclusion: The compounds of the present disclosure have good permeability.

Assay Example 6: Solubility evaluation

Purpose of the assay:

The purpose of this assay was to determine the kinetic solubility of the compounds after incubation at room temperature in a 50 mM phosphate buffer solution with a pH of 2.0, 6.5, and 7.4 for 24 hours using a direct ultraviolet method.

Assay procedure:

10 μL of DMSO stock solutions of a test compound and control compound were added to a 96-well plate. 490 μL of the test medium was added to the 96-well plate, respectively. The plate was sealed, and vortexed for 2 minutes. The incubation concentration of a sample was 200 μM, wherein, DMSO accounted for 2%. The 96-well plate was placed on a shaker, and incubated at room temperature and 800 rpm for 24 hours to reach dissolution equilibrium. After the incubation, the sample was put into a centrifuge, and centrifuged at 25 °C and 4000 rpm for 10 minutes. The supernatant from the centrifuged sample was transferred to a filter plate, centrifuged at 4000 rpm for 5 minutes, and filtered to give filtrate. The concentration of the filtrate was analyzed using linear quantitative analysis with LC-UV (the sample was diluted as needed).

Data of the assay were acquired and processed using Empower software. Linear regression analysis was performed by plotting the relationship between the peak area and their concentrations in μM.

The assay results are shown in Table 6:

Table 6. Solubility assay results of the compounds of the present disclosure

| Compound number | KSY: pH(2.0/ 6.5/ 7.4)(μg/mL) |
|---|---|
| Compound 3 | 3.78/ 3.68/ 4.43 |
| Compound 5 | 4.46/ 4.57/ 4.79 |
| Compound 6 | 21.7/ 20.9/ 21.4 |

Assay conclusion: The compounds of the present disclosure have suitable solubility.

Assay Example 7: Stability evaluation in liver microsomes

Purpose of the assay:

The purpose of this study was to evaluate the phase I metabolic stability of the assay sample in liver microsomes from CD-1 mice, Sprague-Dawley (SD) rats, beagle dogs, cynomolgus monkeys, and human.

Assay materials:

The animal and human liver microsomes used in this assay system were purchased from Xenotech, Corning, or other qualified suppliers.

Assay procedure:

Preparation of buffer: 73.21 g of potassium phosphate dibasic trihydrate and 10.78 g of potassium dihydrogen phosphate were dissolved in 4000 mL of ultrapure water. The pH of the solution was adjusted to the range of 7.40 ± 0.10 using 10.0% phosphoric acid or 1.00 M potassium hydroxide. Its final concentration was 100 mM.

Preparation of working solutions: An assay sample powder was prepared into a 10.0 mM stock solution with DMSO, and then further diluted with a suitable organic solvent. The control compounds testosterone, diclofenac, and propafenone were prepared into 10.0 mM stock solutions using DMSO, and then further diluted with a suitable organic solvent.

Preparation of liver microsome solution: Microsomes of various species were diluted with 100 mM potassium phosphate buffer to a working solution of 0.56 mg/mL. The final concentration of microsomes in the reaction system was 0.500 mg/mL. Detailed preparation procedures were described in the experimental records.

Preparation of reduced nicotinamide adenine dinucleotide phosphate (NADPH) solution: An appropriate amount of reduced nicotinamide adenine dinucleotide phosphate was dissolved in magnesium chloride solution, and the mixture was shaken to mix well. The final concentrations in the reaction system were 1.00 mM NADPH and 1.00 mM magnesium chloride, respectively. Detailed preparation procedures were described in the experimental records.

Preparation of stop solution: The stop solution was prepared using acetonitrile containing internal standards (tolbutamide and labetalol). The prepared stop solution was stored at 2.0 to 8.0 °C in a refrigerator. Detailed preparation procedures were described in the experimental records.

Incubation process: Two 96-well incubation plates were prepared and named T60 incubation plate and NCF60 incubation plate, respectively. 445 μL of microsomal working solution (liver microsomal protein concentration of 0.56 mg/mL) were added to the T60 incubation plate and the NCF60 incubation plate, respectively, and then the above incubation plates were pre-incubated at 37 °C for about 10 minutes. After the pre-incubation, 5 μL of working solutions of the assay sample or the control compound were added to the T60 incubation plate and the NCF60 incubation plate, respectively, and the mixture was mixed well. 50 μL of potassium phosphate buffer was added to each well of the NCF60 incubation plate to initiate the reaction. 180 μL of stop solution (250 nM tolbutamide and 250 nM labetalol in acetonitrile) and 6 uL of NADPH working solution were added to the T0 stop plate, and 54 μL of sample was transferred from T60 incubation plate to T0 stop plate (generation of T0 sample). The reaction was initiated by adding 44 μL of NADPH working solution to each well of the T60 incubation plate. Only 54 μL of microsomal working solution, 6 uL of NADPH working solution and 180 μL of stop solution were added to the Blank plate. Therefore, in samples of the test compound or the control compound, the final reaction concentrations of the compound, testosterone, diclofenac and propafenone were 1 μM, the concentration of liver microsomes was 0.5 mg/mL, and the final concentrations of DMSO and acetonitrile in the reaction system were 0.01% (v/v) and 0.99% (v/v), respectively. After an appropriate time (e.g., 5, 15, 30, 45 and 60 minutes) of incubation, 180 μL of stop solution (250 nM tolbutamide and 250 nM labetalol in acetonitrile) was added to the sample wells of each stop plate. Then, 60 μL of sample was removed from the T60 or NCF60 incubation plate to stop the reaction. All sample plates were shaken well and then centrifuged at 3220×g for 20 minutes. Then 80 μL of supernatant

was taken out from each well, and diluted in 240 μL of pure water for liquid chromatography-tandem mass spectrometry analysis.

Data analysis:

In this study, the sample analysis of compounds was performed using liquid chromatography-tandem mass spectrometry (LC-MS/MS), without standard curves and quality control samples. Semi-quantitative determination was performed using the ratio of the peak area of analyte to the peak area of internal standard. The retention time of the analyte and internal standard, chromatogram acquisition, and chromatogram integration were processed using the software Analyst (Sciex, Framingham, Massachusetts, USA).
The CV of the peak area of internal standard in each matrix in each analytical batch should be within 20%.
The peak height of the sample with the highest concentration did not exceed $2.00 \times 10^6$ cps (using API 4000 or API 5500) or $4.00 \times 10^7$ cps (using API 6500 or API 6500 Plus). The peak height of the internal standard did not exceed $2.00 \times 10^6$ cps (using API 4000 or API 5500) or $4.00 \times 10^7$ cps (using API 6500 or API 6500 Plus).
The in vitro elimination rate constant ke for the compound and the control compound was obtained by converting the peak area ratio of the compound to internal standard into residual rate using the following formula:

$$\text{Residual rate(\%)} = \frac{\text{Peak area ratio of compound to internal standard at any time point}}{\text{Peak area ratio of compound to internal standard at 0 minutes}} \times 100$$

$$C_t = C_0 \times e^{-k_e \times t}$$

$$\text{when } C_t = \frac{1}{2} C_0$$

$$T_{1/2} = \frac{\text{Ln2}}{k_e} = \frac{0.693}{k_e}$$

The in vitro intrinsic clearance rate of liver microsomes (CLint(mic)) and intrinsic clearance rate of liver (CLint(liver)) were calculated using ke.

CLint(mic) = 0.693/T1/2/microsomal protein content (microsomal concentration during the incubation, mg/mL)

CLint(liver) = CLint(mic) × amount of microsomal protein in liver (mg/g) × ratio of liver weight to body weight

Based on the well-stirred model, the in vivo liver clearance rate (CL(liver)) was estimated:

$$\text{CL(liver)} = (\text{CLint(liver)} \times \text{fu} \times \text{Qh}) / (\text{CLint(liver)} \times \text{fu} + \text{Qh})$$

The default value for fu (the free fraction in blood) was 1.
The assay results are shown in Table 7:

Table 7. Assay results of stability in liver microsomes of the compounds of the present disclosure

| Compound number | MMS(h/ r/ m/ d/ c)$Cl_{int}$(mL/min/kg) |
|---|---|
| Compound 1 | 30/ 193/ 220/ 16/ 130 |
| Compound 3 | 19/123 /103 / <14/ 129 |
| Compound 5 | <8.6/<17/<38/<13.8/15.3 |

Assay conclusion: The compounds of the present disclosure have good stability in liver microsomes.

Assay Example 8: Evaluation of drug-drug interactions

Purpose of the assay:

The inhibitory effects of compounds on the activity of human liver microsomal cytochrome P450 isoenzymes (CYP1A2, CYP2C9, CYP2C19, CYP2D6, and CYP3A) were evaluated using a cocktail method with specific probe substrates for CYP1A2, CYP2C9, CYP2C19, CYP2D6, and CYP3A.

Assay materials:

Human liver microsomes, probe substrates, positive control inhibitor, and reduced nicotinamide adenine dinucleotide phosphate.

Assay procedure:

20 μL of a substrate solution was added to the corresponding well of a reaction plate, and 20 μL of potassium phosphate buffer was added to the blank wells.
2 μL of a test compound working solution and positive control working solution were added to the corresponding wells, and 2 μL of solvent was added to the inhibitor-free wells and blank wells.
Human liver microsomal working solution was prepared, and 158 μL of human liver microsomal working solution was added to each well of the reaction plate.
The reaction plate was preheated at 37.0 °C for 10 minutes.
20 μL of NADPH cofactor working solution was added to the reaction plate to initiate the reaction.
The mixture was mixed and incubated in a 37.0 °C water bath for 10 minutes, and 400 μL of stop solution was added to the reaction plate to terminate the reaction.
The sample plate was placed in a centrifuge, and centrifuged at 4000 rpm for 20 minutes.
200 μL of supernatant was taken out, and appropriate 100 μL of pure water was added for dilution. The plate was shaken until the mixture was mixed well.
The analysis was performed using liquid chromatography-tandem mass spectrometry (LC-MS/MS).

Data analysis:

The $IC_{50}$ values of the assay samples were calculated by performing a three-parameter or four-parameter nonlinear regression analysis using XL fit software, with the remaining percentage of activity as the ordinate and the assay sample concentration as the abscissa. When the $IC_{50}$ obtained by fitting with XL fit software was greater than the maximum administration concentration (50.0 μM) or $IC_{50}$ could not be obtained by fitting, the $IC_{50}$ value was marked as ">50.0 μM".
Three-parameter equation:

$$y = \frac{\max}{1+(\frac{x}{IC_{50}})^{-hillslope}}$$

Four-parameter equation:

$$y = \min + \frac{\max - \min}{1+(\frac{x}{IC_{50}})^{-hillslope}}$$

max: maximum enzyme activity; min: minimum enzyme activity; x: concentration of the assay sample or positive control inhibitor; y: enzyme activity at the corresponding concentration; hillslope: slope; $IC_{50}$: half-maximal inhibitory concentration; the four-parameter equation was used when the minimum enzyme activity was within ±10%, otherwise the three-parameter equation was used. The assay results are shown in Table 8:

Table 8. Drug-drug interaction assay results of the compounds of the present disclosure

| Compound number | CYP(μM)1A2/2C9/2C19/2D6/3A4 |
|---|---|
| Compound 1 | >50 / 46 / 31 / 42 / 14 |
| Compound 3 | All>50 |
| Compound 5 | All>50 |

(continued)

| Compound number | CYP(μM)1A2/2C9/2C19/2D6/3A4 |
|---|---|
| Compound 6 | All>50 |

Assay conclusion: The compounds of the present disclosure have low CYP inhibition and low risk of drug-drug interactions.

Assay Example 9: Automated patch-clamp (Qpatch) assay for the effect on hERG potassium channels

Assay method:

CHO-hERG cells were cultured in a 175 $cm^2$ culture flask. When cells grew to a density of 60 to 80%, the culture medium was removed. Cells were washed once with 7 mL of PBS (Phosphate Buffered Saline), and then 3 mL of cell dissociation reagent was added for digestion. After the digestion was completed, 7 mL of culture medium was added for neutralization, and then the mixture was centrifuged. The supernatant was aspirated, and then 5 mL of culture medium was added to resuspend the cells and ensure that the cell density was $2\sim5\times10^6$/mL.

The compound stock solution was diluted with DMSO. 10 μL of compound stock solution was added to 20 μL of DMSO solution, followed by 3-fold serial dilution to obtain 6 DMSO concentrations. 4 μL of the compound solution at each of the six DMSO concentrations was added to 396 μL of extracellular solution to achieve a 100-fold dilution to obtain six intermediate concentrations. Then, 80 μL of the compound solution at each of the six intermediate concentrations was added to 320 μL of extracellular solution to achieve a 5-fold dilution to the desired final test concentrations. The highest test concentration was 40.00 μM, and there was a total of 6 concentrations: 40.00, 13.33, 4.44, 1.48, 0.49 and 0.16 μM, respectively. The DMSO content in the final test concentration did not exceed 0.2%. This concentration of DMSO had no effect on the hERG potassium channel. All dilutions in the compound preparation were performed by a Bravo instrument.

The electrophysiological process was recorded. The single-cell high-impedance sealing and whole-cell pattern formation processes were all automatically performed by a Qpatch instrument. After obtaining the whole-cell recording mode, the cells were clamped at -80 mV. The cells were applied successively with a pre-voltage of -50 mV for 50 milliseconds and a depolarizing stimulus of +40 mV for 5 seconds, then repolarized to -50 mV for 5 seconds, and then back to -80 millivolts. This voltage stimulation was applied every 15 seconds, and recorded for 2 minutes. The extracellular solution was then given, and recorded for 5 minutes. Then a drug administration process started. The compound concentration started from the lowest test concentration, and each test concentration was administered for 2.5 minutes. At least 3 cells were tested for each concentration (n ≥ 3).

Data analysis:

Assay data were analyzed by GraphPad Prism 5.0 software. The results are shown in Table 9.

Table 9. Assay results of the $IC_{50}$ values of the compounds of the present disclosure on hERG potassium channels

| **Compound number** | **$IC_{50}$(μM)** |
|---|---|
| Compound **1** | >40 |
| Compound **3** | >40 |

Assay conclusion: The compounds of the present disclosure have no significant inhibitory effect on hERG potassium channels and have a low risk of cardiotoxicity.

## Claims

**1.** A compound of formula (I) or (V), or a pharmaceutically acceptable salt thereof,

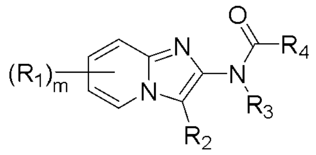

(I)

or

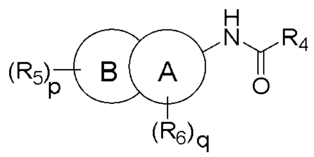

(V)

,

wherein,

ring A is 9- to 10-membered bicyclic heteroaryl;
ring B is fused with ring A, and ring B is selected from $C_{4-7}$ cycloalkyl, $C_{4-7}$ cycloalkenyl, 4- to 7-membered heterocycloalkyl and 4- to 7-membered heterocycloalkenyl;
each $R_1$ is independently selected from H, F, Cl, Br, I, OH, $NH_2$, CN, $CF_3$, $C_{2-4}$ alkyl, $C_{1-4}$ alkoxy, $C_{3-7}$ cycloalkyl and 4- to 7-membered heterocycloalkyl, wherein the $C_{2-4}$ alkyl, $C_{1-4}$ alkoxy, $C_{3-7}$ cycloalkyl and 4- to 7-membered heterocycloalkyl are each independently and optionally substituted with 1, 2 or 3 $R_a$;
$R_2$ is selected from cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl, wherein the cyclobutyl, $C_{5-8}$ bridged cycloalkyl, $C_{5-8}$ spirocycloalkyl, phenyl, 5- to 6-membered heteroaryl, 4- to 7-membered heterocycloalkyl and $C_{5-8}$ cycloalkenyl are each independently and optionally substituted with 1, 2 or 3 $R_b$;
$R_3$ is selected from H and $-C(O)C_{1-4}$ alkyl;
$R_4$ is selected from $C_{1-6}$ alkyl, $C_{1-6}$ alkoxy, $-CH_2-C_{3-7}$ cycloalkyl and $-CH_2$-4- to 7-membered heterocycloalkyl, wherein the $C_{1-6}$ alkyl, $C_{1-6}$ alkoxy, $-CH_2-C_{3-7}$ cycloalkyl and $-CH_2$-4- to 7-membered heterocycloalkyl are each independently and optionally substituted with 1, 2, 3, 4 or 5 $R_c$;
each $R_5$ is independently selected from H, F, Cl, Br, I, =O, $C_{1-4}$ alkyl and $C_{1-4}$ alkoxy, wherein the $C_{1-4}$ alkyl and $C_{1-4}$ alkoxy are each independently and optionally substituted with 1, 2 or 3 halogen;
each $R_6$ is independently selected from H, F, Cl, Br, I, CN, $C_{1-4}$ alkyl, $C_{1-4}$ alkoxy, $C_{3-7}$ cycloalkyl, 3- to 7-membered heterocycloalkyl and phenyl, wherein the $C_{1-4}$ alkyl, $C_{1-4}$ alkoxy, $C_{3-7}$ cycloalkyl, 4- to 7-membered heterocycloalkyl and phenyl are each independently and optionally substituted with 1, 2 or 3 $R_{6a}$;
each of $R_a$ and $R_c$ is independently selected from H, F, Cl, Br, I, OH, $NH_2$, CN, $CH_3$ and $CF_3$;
each $R_b$ is independently selected from H, F, Cl, Br, I, =O, OH, $NH_2$, CN, $C_{1-4}$ alkyl and $C_{1-4}$ alkoxy, wherein the $C_{1-4}$ alkyl and $C_{1-3}$ alkoxy are each independently and optionally substituted with 1, 2 or 3 F;
each $R_{6a}$ is independently selected from H, F, Cl, Br, I, OH, $NH_2$, CN, $C_{1-4}$ alkyl and $C_{1-4}$ alkoxy, wherein the $C_{1-4}$ alkyl and $C_{1-4}$ alkoxy are each independently and optionally substituted with 1, 2, 3, 4 or 5 F;
m is selected from 1, 2, 3 and 4;
p and q are each independently selected from 1, 2 and 3;
with the proviso that,
1) when $R_2$ is selected from phenyl and 5- to 6-membered heteroaryl, and the phenyl and 5- to 6-membered heteroaryl are each independently and optionally substituted with 1, 2 or 3 $R_b$, the structural fragment

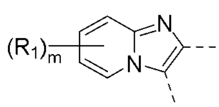

is

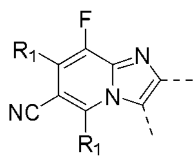

;

or,
2) when $R_2$ is cyclobutyl, and the cyclobutyl is optionally substituted with 1, 2 or 3 $R_b$, m is selected from 2, 3 and 4.

2. The compound according to claim 1, or a pharmaceutically acceptable salt thereof, wherein, each $R_a$ is independently selected from H, F and OH.

3. The compound according to claim 1, or a pharmaceutically acceptable salt thereof, wherein, each $R_b$ is independently selected from H, F, =O, $CH_3$ and $OCF_3$.

4. The compound according to claim 1, or a pharmaceutically acceptable salt thereof, wherein, each $R_c$ is independently selected from H, F and $CH_3$.

**5.** The compound according to claim 1, or a pharmaceutically acceptable salt thereof, wherein, each $R_1$ is independently selected from H, F, Cl, OH, $NH_2$, CN, $CF_3$, $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl, wherein the $CH_2CH_3$, $CH_2CH_2CH_3$, $CH(CH_3)_2$, $C(CH_3)_3$, $OCH_3$, $OCH_2CH_3$, $OCH_2CH_2CH_3$, $OCH(CH_3)_2$, cyclopropyl, cyclobutyl, cyclopentyl, oxetanyl, and azetidinyl are each independently and optionally substituted with 1, 2 or 3 $R_a$; or, each $R_1$ is independently selected from H, F, CN, $CF_3$,

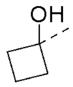, 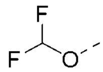 and 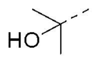.

**6.** The compound according to claim 1, or a pharmaceutically acceptable salt thereof, wherein, $R_2$ is selected from cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl, wherein the cyclobutyl, spiro[3.3]heptyl, bicyclo[1.1.1]pentyl, phenyl, pyrazolyl, thiazolyl, pyridyl, oxetanyl, azetidinyl, piperidyl, piperazinyl, cyclopentenyl and cyclohexenyl are each independently and optionally substituted with 1, 2 or 3 $R_b$; or, $R_2$ is selected from

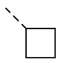, 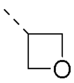, 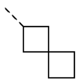, 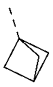, 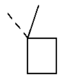, 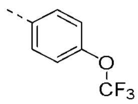, 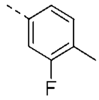, 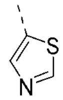, 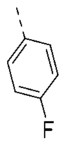, 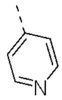, 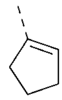,

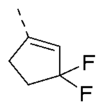 and 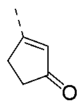; or, $R_2$

is

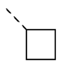, 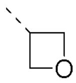, 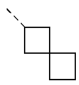, 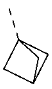, 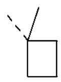 or 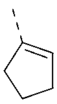.

**7.** The compound according to claim 1, or a pharmaceutically acceptable salt thereof, wherein, $R_4$ is selected from $CH_2CH_3$, $CH_2CH_2CH_3$, $CH(CH_3)_2$, $C(CH_3)_3$, $CH_2C(CH_3)_3$, $OCH_3$, $OCH_2CH_3$, $OCH_2CH_2CH_3$, $OCH(CH_3)_2$,

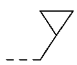, 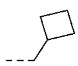, 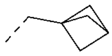, 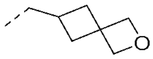, 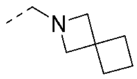, 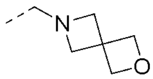, 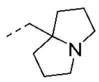

and

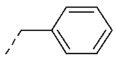,

wherein the $CH_2CH_3$, $CH_2CH_2CH_3$, $CH(CH_3)_2$, $C(CH_3)_3$, $CH_2C(CH_3)_3$, $OCH_3$, $OCH_2CH_3$, $OCH_2CH_2CH_3$, $OCH(CH_3)_2$,

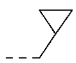, 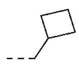, 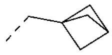, 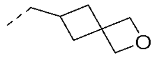, 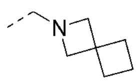, 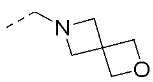,

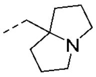 and 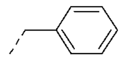

are each independently and optionally substituted with 1, 2, 3, 4 or 5 $R_e$; or, $R_4$ is selected from

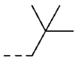, 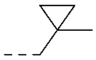, 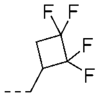, 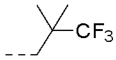, 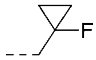, 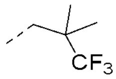, 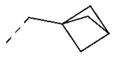 and 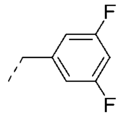.

**8.** The compound according to claim 1, or a pharmaceutically acceptable salt thereof, wherein, the structural unit

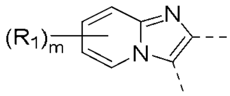

is selected from

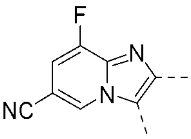, 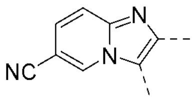, 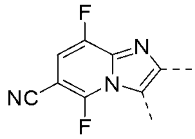,

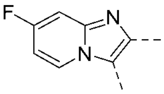, 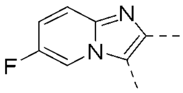, 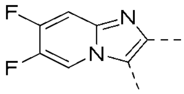, 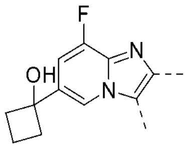, 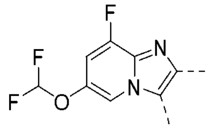,

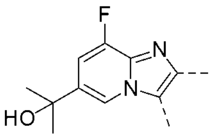 and 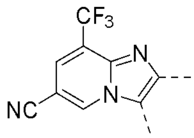.

**9.** The compound according to claim 1, or a pharmaceutically acceptable salt thereof, wherein, the structural unit

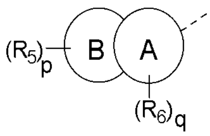

is selected from

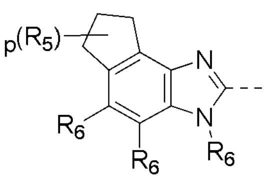, 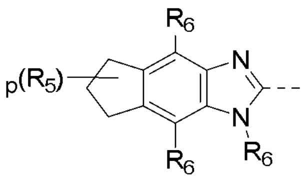,

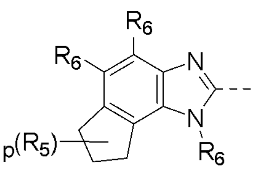, 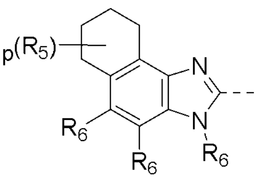, 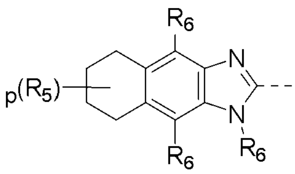, 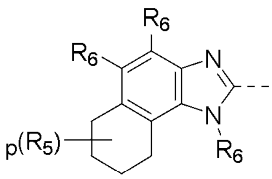,

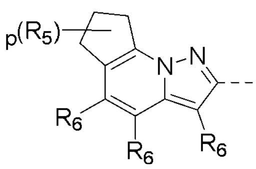 , 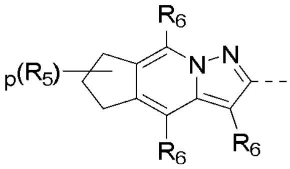 , 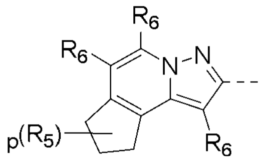 , 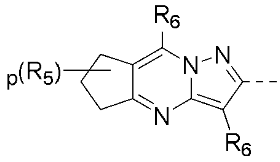 ,

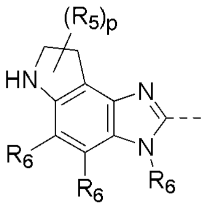 , 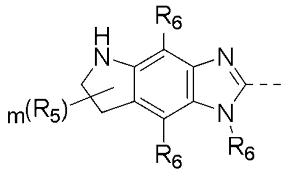 , 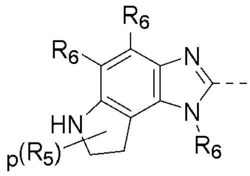 , 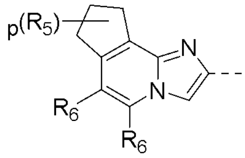 ,

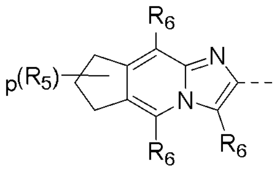 , 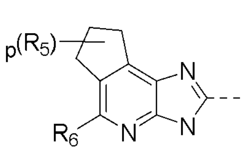 , 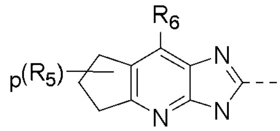 , 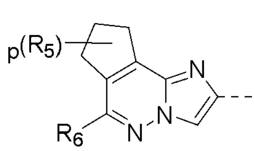 ,

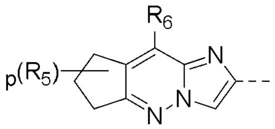 , 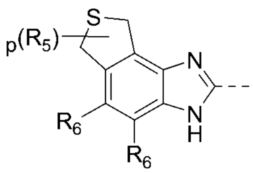 , 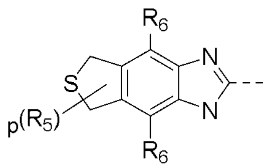 , 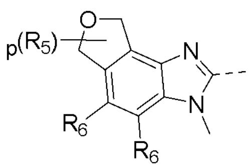 ,

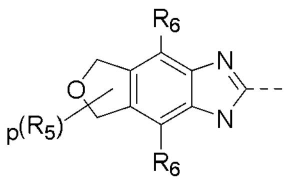 , 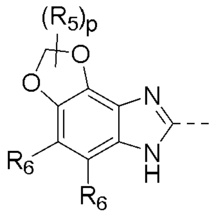 and 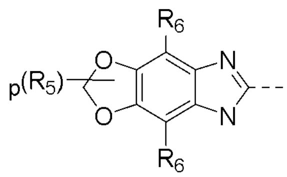 ,

wherein $R_5$, $R_6$ and p are as defined in claim 1.

**10.** The compound according to claim 9, or a pharmaceutically acceptable salt thereof, wherein the structural unit

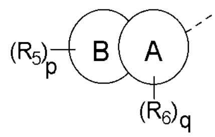

is

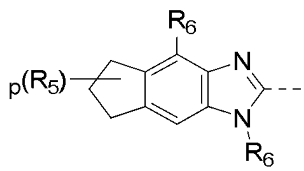 .

**11.** The compound according to claim 1, or a pharmaceutically acceptable salt thereof, wherein the compound is

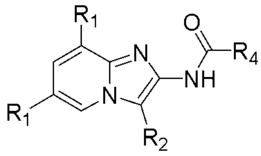

(III) ,

wherein,

each $R_1$ is independently selected from H, F, CN, $CF_3$,

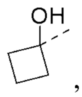, 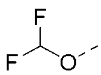 and 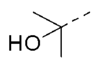;

$R_2$ is selected from $C_{5-8}$ bridged cycloalkyl, $C_{5-8}$ spirocycloalkyl, 4- to 7-membered heterocycloalkyl and $C_{5-8}$ cycloalkenyl, wherein the $C_{5-8}$ bridged cycloalkyl, $C_{5-8}$ spirocycloalkyl, 4- to 7-membered heterocycloalkyl and $C_{5-8}$ cycloalkenyl are each independently and optionally substituted with 1, 2 or 3 $R_b$;

$R_4$ is selected from

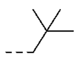, 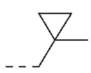, 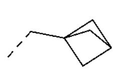, 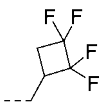, 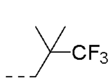 and 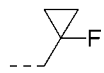.

**12.** A compound of the following, or a pharmaceutically acceptable salt thereof,

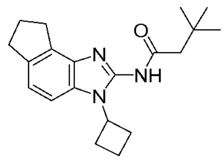 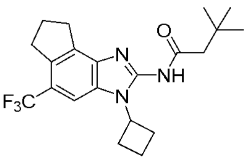 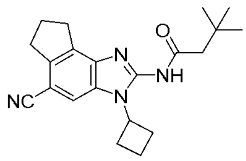 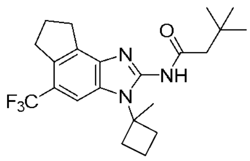 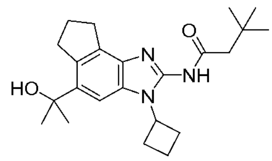

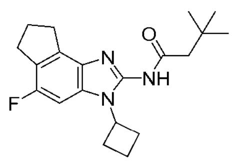 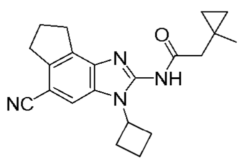 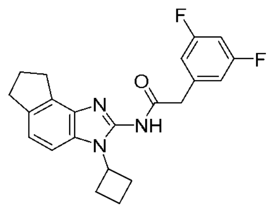 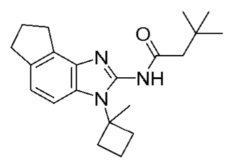 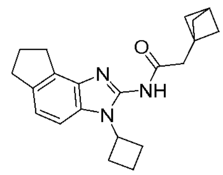

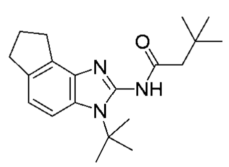 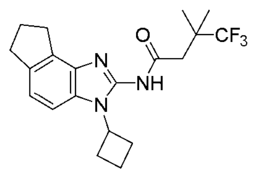 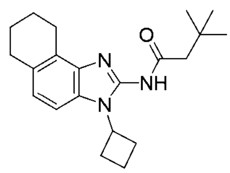 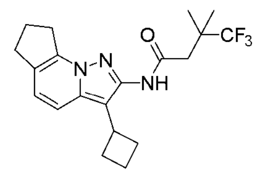 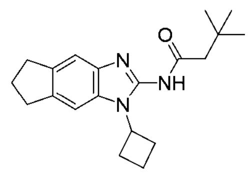

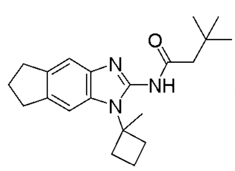 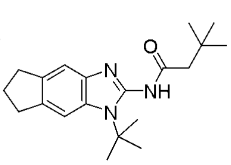 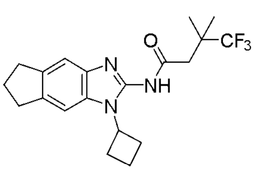 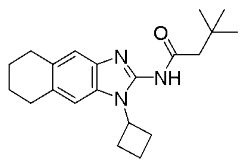 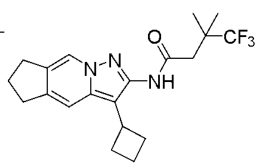

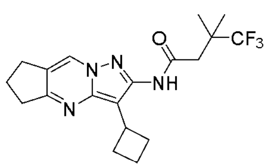
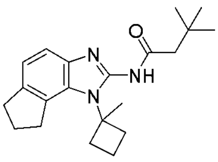
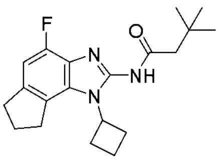
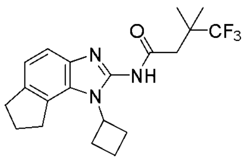
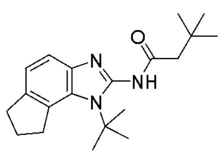

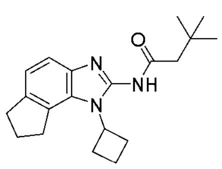
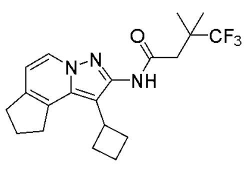
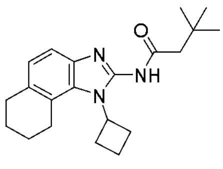
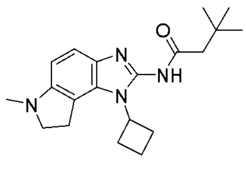
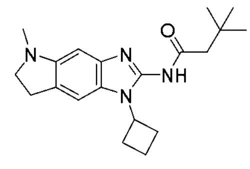

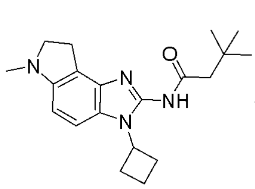
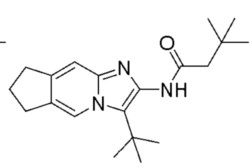
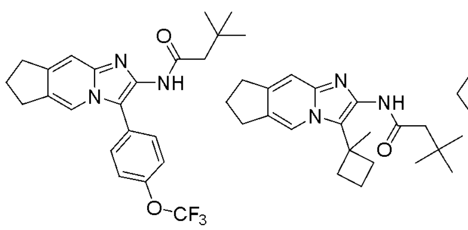
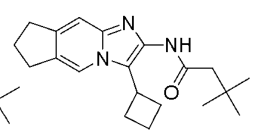

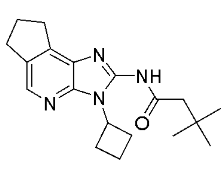
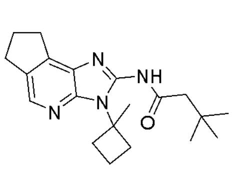
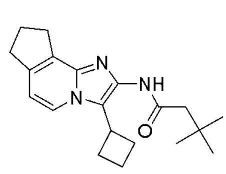
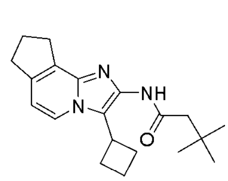
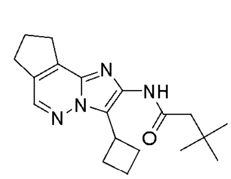

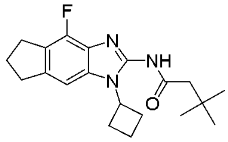
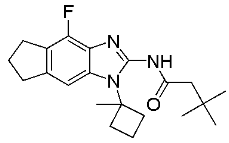
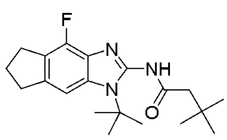
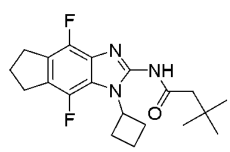
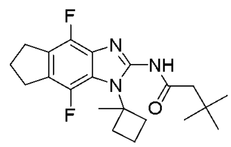

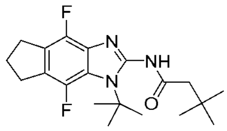
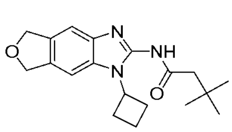
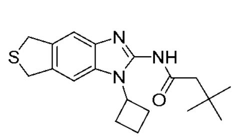
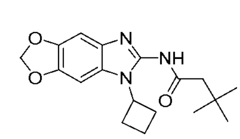
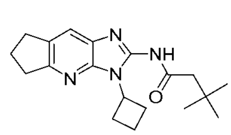

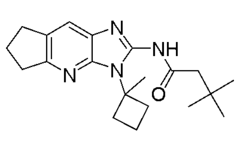
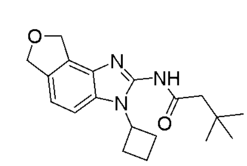
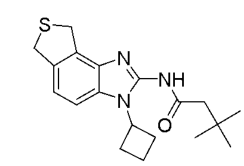
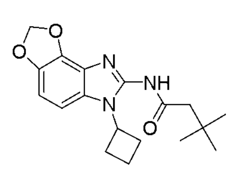
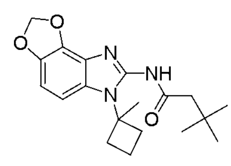

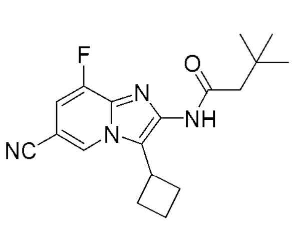
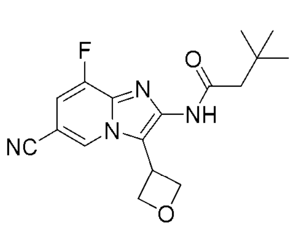
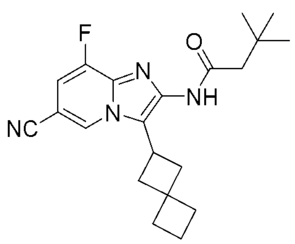
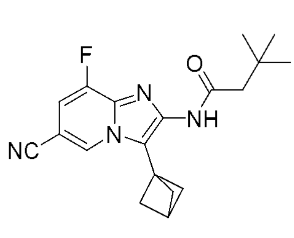

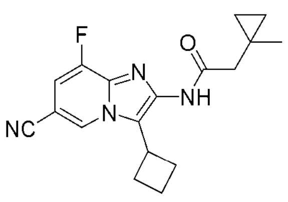
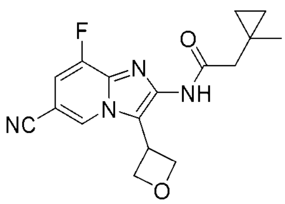
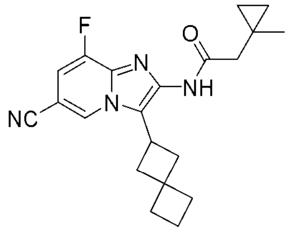
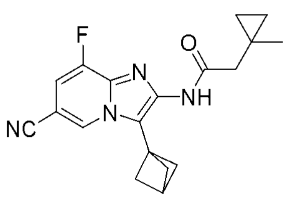

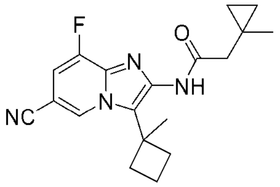
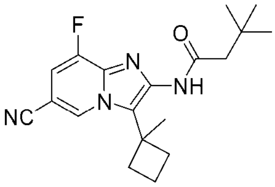
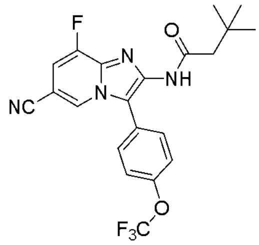
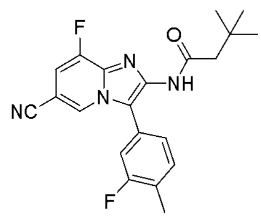

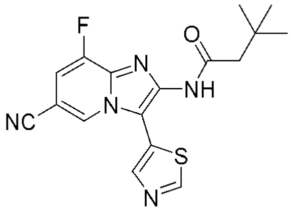
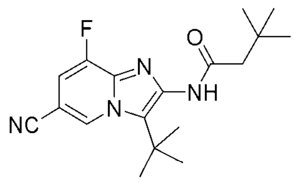
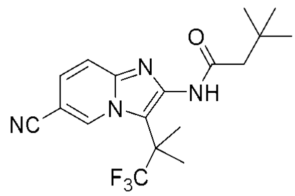
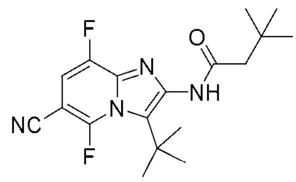

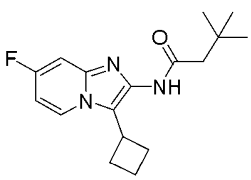
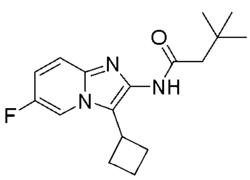
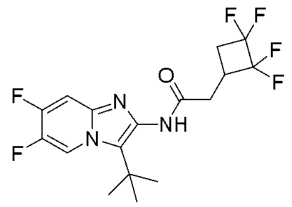
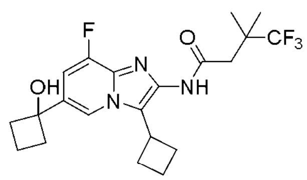

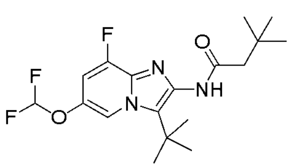
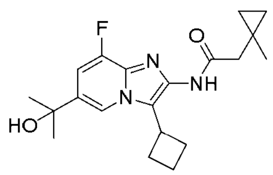
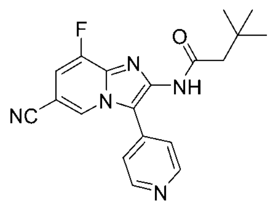
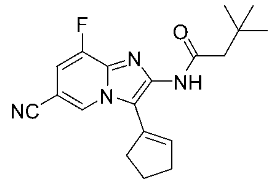

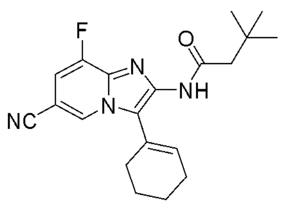
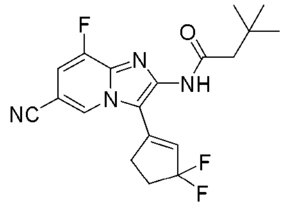
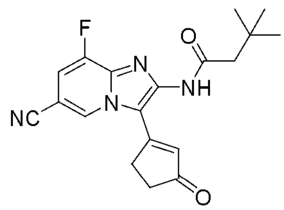
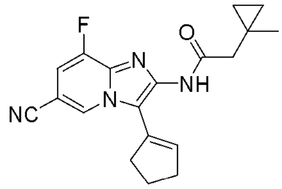

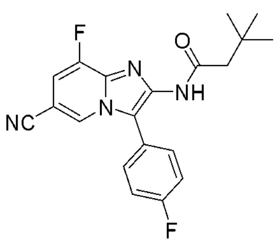
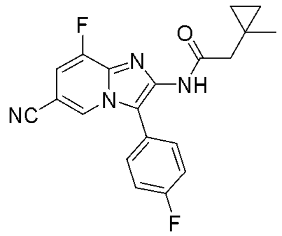
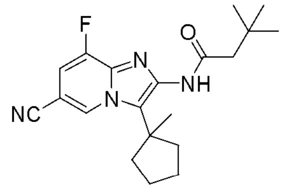
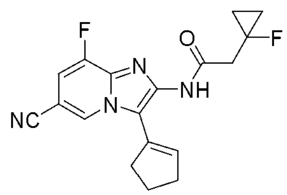

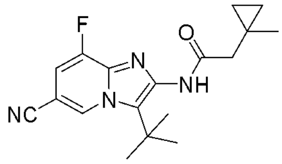
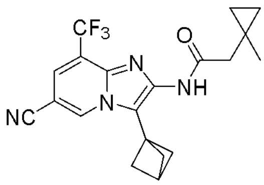
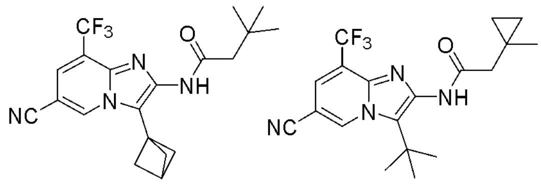

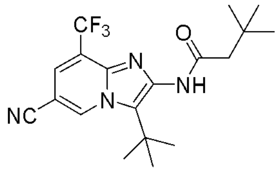
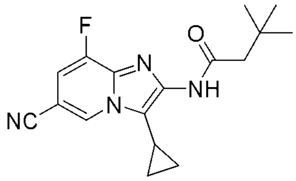.

**13.** A pharmaceutical composition, comprising a therapeutically or prophylactically effective amount of the compound according to any one of claims 1 to 12, or a stereoisomer or a pharmaceutically acceptable salt thereof; further, the pharmaceutical composition also comprises a pharmaceutically acceptable excipient.

**14.** Use of the compound according to any one of claims 1 to 12, or a pharmaceutically acceptable salt thereof, or the pharmaceutical composition according to claim 13 in the manufacture of a medicament for the treatment of a Kv7 potassium ion channel opener-related disease.

**15.** The use according to claim 14, wherein, the Kv7 potassium ion channel opener-related disease is selected from epilepsy, inflammatory pain, neuropathic pain, migraine, depression, anxiety disorder, stroke, Alzheimer's disease, neurodegenerative disease, neuronal hyperexcitability, complication caused by cocaine abuse, nicotine withdrawal syndrome, alcohol withdrawal syndrome, and tinnitus.

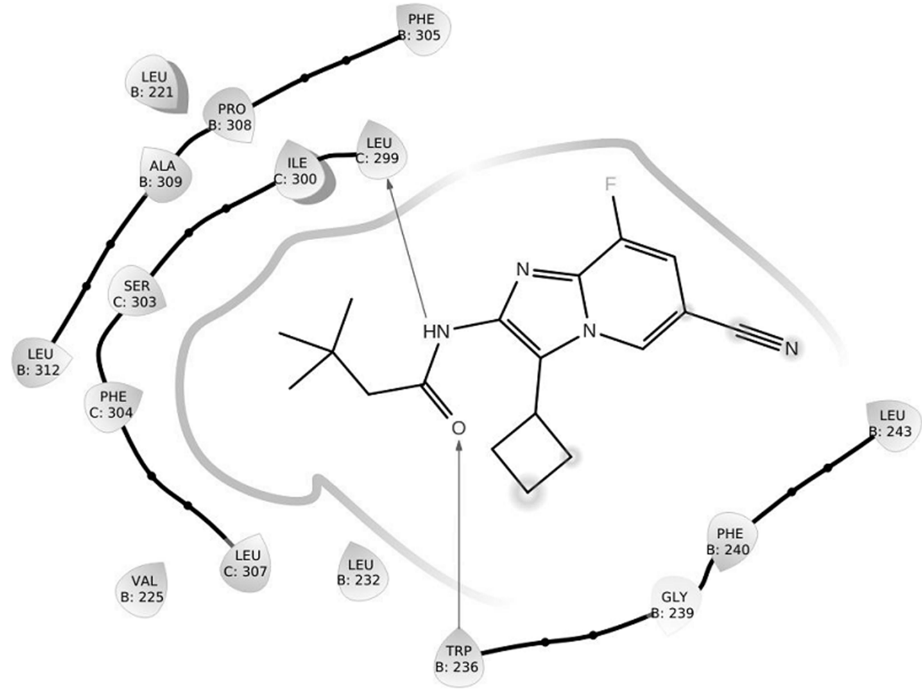
PHE B: 305
LEU B: 221
PRO B: 308
ALA B: 309
ILE C: 300
LEU C: 299
SER C: 303
LEU B: 312
PHE C: 304
LEU B: 243
PHE B: 240
GLY B: 239
VAL B: 225
LEU C: 307
LEU B: 232
TRP B: 236

Fig. 1

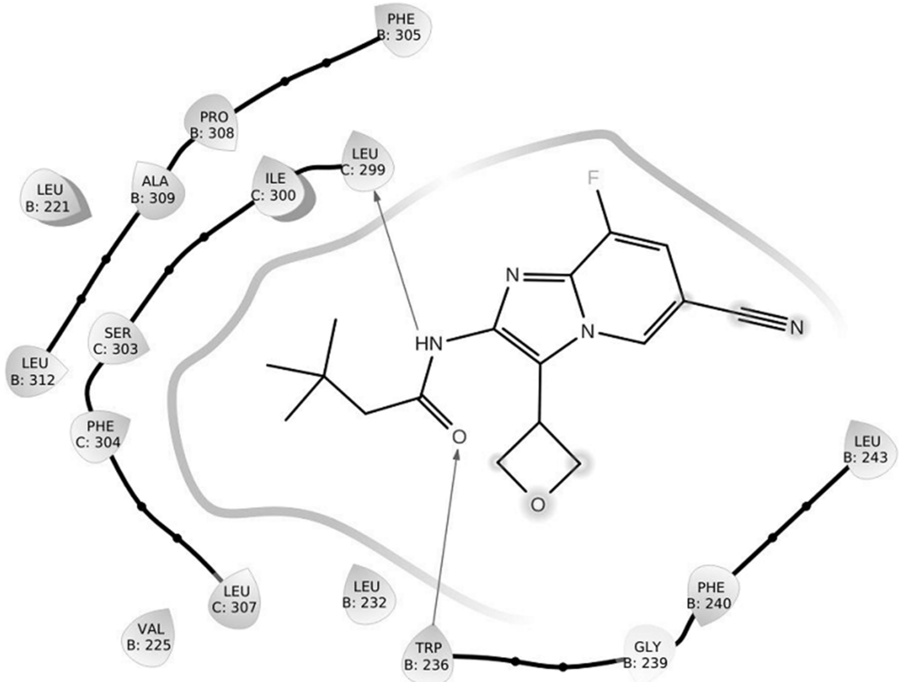
PHE B: 305
PRO B: 308
LEU C: 299
ILE C: 300
ALA B: 309
LEU B: 221
SER C: 303
LEU B: 312
PHE C: 304
LEU B: 243
PHE B: 240
LEU C: 307
LEU B: 232
VAL B: 225
TRP B: 236
GLY B: 239

Fig. 2

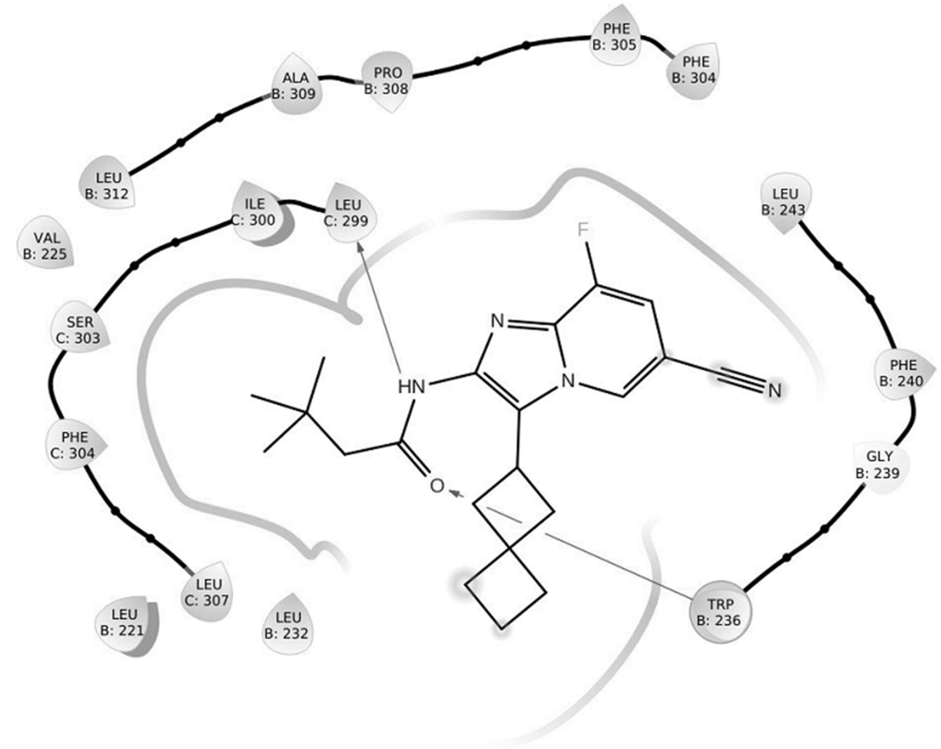
PHE B: 305
PHE B: 304
ALA B: 309
PRO B: 308
LEU B: 312
ILE C: 300
LEU C: 299
VAL B: 225
SER C: 303
PHE C: 304
LEU C: 307
LEU B: 221
LEU B: 232
LEU B: 243
PHE B: 240
GLY B: 239
TRP B: 236

Fig. 3

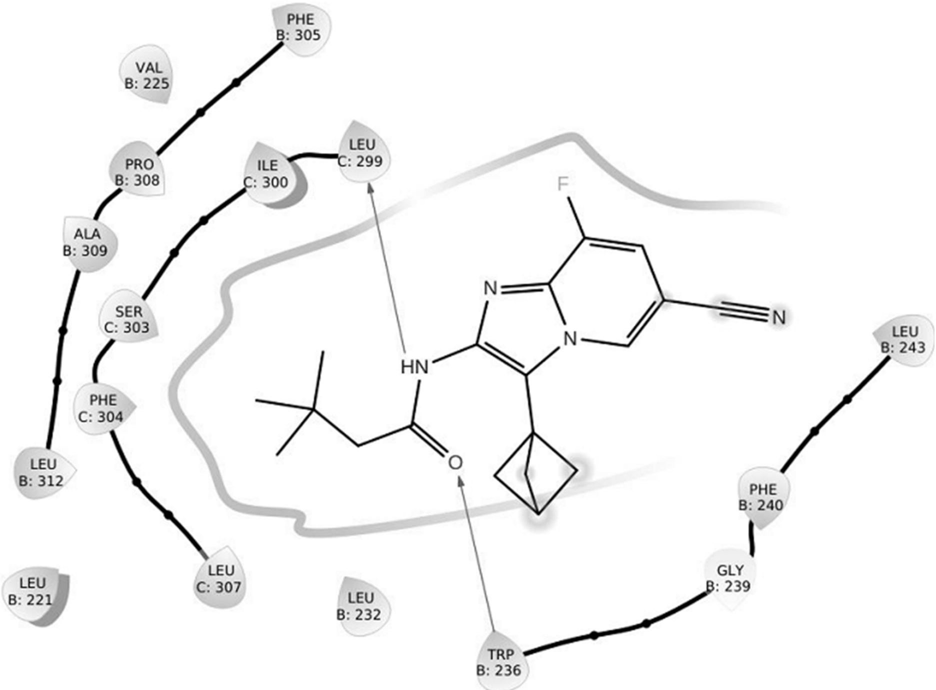
PHE B: 305
VAL B: 225
PRO B: 308
ALA B: 309
ILE C: 300
LEU C: 299
SER C: 303
PHE C: 304
LEU B: 312
LEU B: 221
LEU C: 307
LEU B: 232
TRP B: 236
GLY B: 239
PHE B: 240
LEU B: 243

Fig. 4

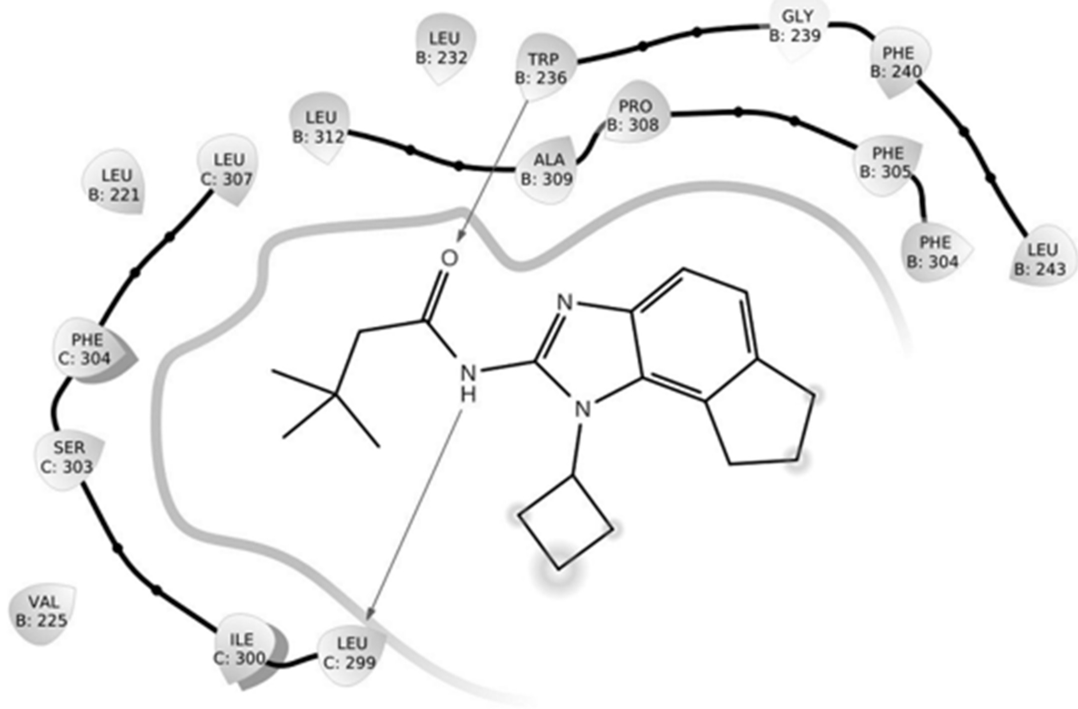
LEU B: 232
TRP B: 236
GLY B: 239
PHE B: 240
LEU B: 312
PRO B: 308
ALA B: 309
PHE B: 305
LEU B: 221
LEU C: 307
PHE B: 304
LEU B: 243
PHE C: 304
SER C: 303
VAL B: 225
ILE C: 300
LEU C: 299

Fig. 5

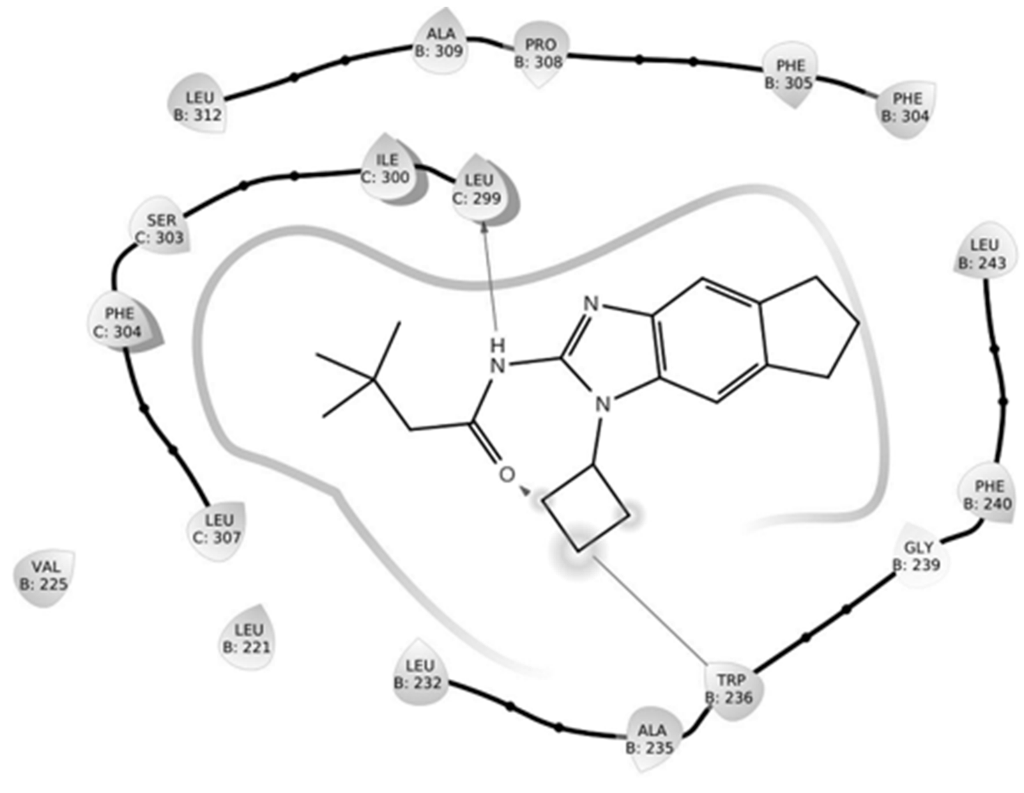
ALA B: 309
PRO B: 308
PHE B: 305
PHE B: 304
LEU B: 312
ILE C: 300
LEU C: 299
SER C: 303
LEU B: 243
PHE C: 304
PHE B: 240
LEU C: 307
GLY B: 239
VAL B: 225
LEU B: 221
LEU B: 232
TRP B: 236
ALA B: 235

Fig. 6

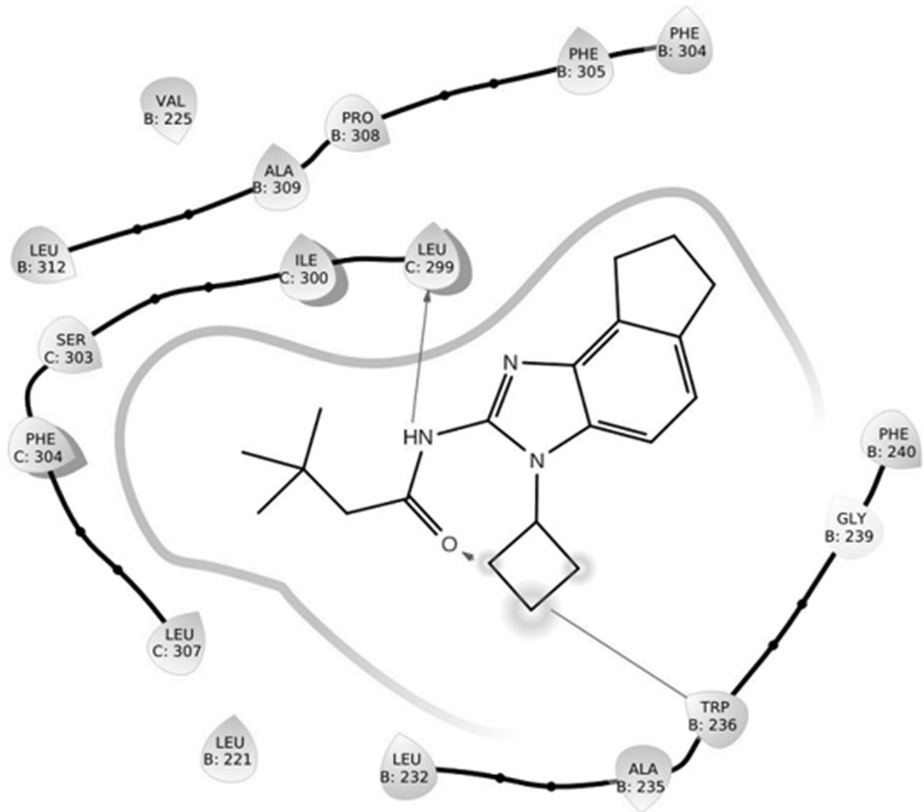
PHE
B: 304
PHE
B: 305
VAL
B: 225
PRO
B: 308
ALA
B: 309
LEU
B: 312
ILE
C: 300
LEU
C: 299
SER
C: 303
PHE
C: 304
PHE
B: 240
GLY
B: 239
LEU
C: 307
TRP
B: 236
LEU
B: 221
LEU
B: 232
ALA
B: 235

Fig. 7

**INTERNATIONAL SEARCH REPORT**

International application No.
**PCT/CN2024/121859**

**A. CLASSIFICATION OF SUBJECT MATTER**

C07D 235/02(2006.01)i; C07D 471/04(2006.01)i; A61K 31/5025(2006.01)i; A61K 31/437(2006.01)i; A61P 25/28(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, ENTXT, ENTXTC, REGISTRY(STN), CAPLUS(STN): 南京明德, 钾离子通道, 中枢神经系统, 癫痫, 疼痛, Kv7, KCNQ, potassium channel modulators, central nervous system, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 101842011 A (ICAGEN, INC.) 22 September 2010 (2010-09-22) description, page 2, paragraph 0011, pages 3-4, paragraphs 0019-0024, pages 41-42, embodiment 1, pages 43-47, paragraphs 0322-0404, page 76, embodiment 4, and page 78, paragraphs 0794-0795, and claims 1-45 | 1-8, 11-15 |
| X | US 2010240663 A1 (ICAGEN, INC.) 23 September 2010 (2010-09-23) description, page 29, right-hand column, embodiment 1, pages 30-32, paragraphs 0224-0306, and page 43, embodiment 4, and claims 1-49 | 1-8, 11-15 |
| A | CN 103508960 A (SHANGHAI SIMCERE PHARMACEUTICAL RESEARCH CO., LTD. et al.) 15 January 2014 (2014-01-15) claims 1-10 | 1-15 |
| A | WO 2023143386 A1 (SHANGHAI ZHIMENG PHARMACEUTICAL TECHNOLOGY CO., LTD.) 03 August 2023 (2023-08-03) claims 1-10 | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 December 2024** | **07 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/121859**

| Patent document cited in search report | Publication date (day/month/year) | Patent family member(s) | Publication date (day/month/year) |
|---|---|---|---|
| CN 101842011 A | 22 September 2010 | BRPI 0815503 A2 | 30 September 2014 |
| | | US 2009062290 A1 | 05 March 2009 |
| | | US 8058274 B2 | 15 November 2011 |
| | | KR 20100044852 A | 30 April 2010 |
| | | MX 2010001824 A | 21 April 2010 |
| | | CA 2696631 A1 | 26 February 2009 |
| | | AU 2008289101 A1 | 26 February 2009 |
| | | JP 2010536796 A | 02 December 2010 |
| | | JP 5463287 B2 | 09 April 2014 |
| | | ES 2400604 T3 | 11 April 2013 |
| | | EP 2178373 A1 | 28 April 2010 |
| | | EP 2178373 A4 | 19 October 2011 |
| | | EP 2178373 B1 | 23 January 2013 |
| | | WO 2009026254 A1 | 26 February 2009 |
| US 2010240663 A1 | 23 September 2010 | US 8431608 B2 | 30 April 2013 |
| | | JP 2013519728 A | 30 May 2013 |
| | | EP 2536718 A1 | 26 December 2012 |
| | | WO 2011102964 A1 | 25 August 2011 |
| | | US 2013143889 A1 | 06 June 2013 |
| | | US 9018208 B2 | 28 April 2015 |
| | | CA 2788783 A1 | 25 August 2011 |
| | | CA 2788783 C | 03 June 2014 |
| CN 103508960 A | 15 January 2014 | WO 2014000694 A1 | 03 January 2014 |
| WO 2023143386 A1 | 03 August 2023 | KR 20240138104 A | 20 September 2024 |
| | | AU 2023211370 A1 | 15 August 2024 |
| | | EP 4471013 A1 | 04 December 2024 |
| | | IL 314474 A | 01 September 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311284906
- CN 202311410637
- CN 202311849240
- CN 202410539834
- CN 202411320022


### Non-patent literature cited in the description


- *CHEMICAL ABSTRACTS*, 163755-62-2